(19) Europäisches Patentamt
European Patent Office
Office européen des brevets

(11) EP 0 792 252 B1

(12) **EUROPÄISCHE PATENTSCHRIFT**

(45) Veröffentlichungstag und Bekanntmachung des Hinweises auf die Patenterteilung:
**15.12.1999 Patentblatt 1999/50**

(21) Anmeldenummer: **95937872.0**

(22) Anmeldetag: **06.11.1995**

(51) Int Cl.[6]: **C07C 13/42**, C07C 13/28, C07C 271/20, C07C 233/62, C07C 271/28, C07C 271/24, C07C 69/753, C07C 69/007, C07C 43/162, C07D 251/34, C07D 251/40

(86) Internationale Anmeldenummer:
**PCT/EP95/04359**

(87) Internationale Veröffentlichungsnummer:
**WO 96/16008 (30.05.1996 Gazette 1996/25)**

(54) **VERNETZBARE MONOMERE UND ZUSAMMENSETZUNG SOWIE VERNETZTE POLYMERE**

MONOMERS AND COMPOSITION WHICH CAN BE CROSSLINKED AND CROSSLINKED POLYMERS

MONOMERES RETICULABLES ET COMPOSITIONS ET POLYMERES RETICULES

(84) Benannte Vertragsstaaten:
**AT BE CH DE DK ES FR GB IT LI NL SE**

(30) Priorität: **17.11.1994 CH 346694**

(43) Veröffentlichungstag der Anmeldung:
**03.09.1997 Patentblatt 1997/36**

(73) Patentinhaber: **Ciba Specialty Chemicals Holding Inc.**
**4057 Basel (CH)**

(72) Erfinder:
- **MÜHLEBACH, Andreas**
  **CH-1782 Belfaux (CH)**
- **HAFNER, Andreas**
  **CH-3177 Laupen (CH)**
- **VAN DER SCHAAF, Paul, Adriaan**
  **CH-1700 Fribourg (CH)**

(56) Entgegenhaltungen:
**EP-A- 0 287 762**
**US-A- 3 187 018**
**US-A- 4 203 930**
**US-A- 5 182 360**

- **JOURNAL OF CHEMICAL ENGINEERING DATA, Bd. 9, Nr. 2, April 1964 Seite 240 XP 000561615 T. M. MEDVED ET AL 'Bicyclic Diepoxides'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 792 252 B1

Printed by Jouve, 75001 PARIS (FR)

## Beschreibung

Die vorliegende Erfindung betrifft Verbindungen mit mindestens zwei direkt oder über eine Brückengruppe verbundenen gespannten Cycloolefinen; Zusammensetzungen mit diesen Verbindungen und einem Einkomponenten-Katalysator für die thermisch induzierte und/oder strahlungsinduzierte Metathesepolymerisation; ein Polymerisationsverfahren, vernetzte Polymere aus den genannten Verbindungen und gegebenenfalls weiteren für die Metathesepolymerisation geeigneten Olefinen und/oder Cycloolefinen; mit diesen vernetzten Polymeren beschichtete Trägermaterialien; und Polymerisationsverfahren.

In der WO 93/13171 werden luft- und wasserstabile Einkomponenten- und Zweikomponenten-Katalysatoren auf der Basis von Carbonylgruppen enthaltenden Molybdän- und Wolframverbindungen sowie Ruthenium- und Osmiumverbindungen mit mindestens einem Polyenliganden für die thermische Metathesepolymerisation und eine photoaktivierte Metathesepolymerisation von gespannten Cycloolefinen, besonders Norbornen und Norbornenderivaten, beschrieben. Andere polycyclische - vor allen Dingen nicht-kondensierte polycyclische Cycloolefine werden nicht erwähnt. Die verwendeten Einkomponenten-Katalysatoren der Rutheniumverbindungen, nämlich $[(C_6H_6)Ru(CH_3CN)_2Cl]^+PF_6^-$ und $[Ru(Cumen)Cl_2]_2$ können zwar durch UV-Bestrahlung aktiviert werden; die Lagerstabilität der Zusammensetzungen mit Norbornen sind jedoch völlig unzureichend. Diese Katalysatoren vermögen die bekannten Zweikomponenten-Katalysatoren nur unzureichend zu ersetzen.

Demonceau et al. [Demonceau, A., Noels, A.F., Saive, E., Hubert, A.J., J. Mol. Catal. 76:123-132 (1992)] beschreiben $(C_6H_5)_3]_3PRuCl_2$, (p-Cumen)$RuCl_2P(C_6H_{11})_3$ und $(C_6H_5)_3]_3PRuHCl$ als thermische Katalysatoren für die ringöffnende Metathesepolymerisation von Norbornen, einem kondensierten Polycycloolefin. Diese Katalysatoren haben sich auf Grund der zu geringen Aktivität bei der industriellen Herstellung nicht durchsetzen können. Es wird daher vorgeschlagen, die Aktivität durch den Zusatz von Diazoestern zu steigern. Es wird auch erwähnt, dass lediglich (p-Cumen)$RuCl_2P(C_6H_{11})_3$ Norbornen in relativ kurzer Zeit bei 60°C zu polymerisieren vermag. Als weiteres Monomer wird noch Cycloocten erwähnt. Andere Cycloolefine für die Metathesepolymerisation werden nicht erwähnt.

Petasis und Fu [Petasis, N. A., Fu, D., J. Am. Chem. Soc. 115:7208-7214 (1993)] beschreiben die thermische ringöffnende Metathesepolymerisation von Norbornen unter Verwendung von Biscyclopentadienyl-bis(trimethylsilyl) methyl-titan(IV) als thermisch aktiven Katalysator. Andere Cycloolefine für die Metathesepolymerisation werden nicht erwähnt.

In EP 287,762 werden vernetzte Copolymere aus einem Gemisch von 1,2-Bisnorbornenyl-ethan der Formel

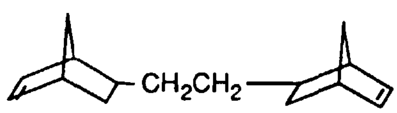

und einer Verbindung der Fromel

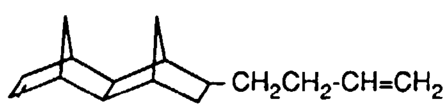

beschrieben, die unter Verwendung von Katalysatorsystemen für die thermische Metathesepolymerisation aus einem Katalysator und einem Aktivator hergestellt werden. Nachteilig bei diesen Systemen ist die Notwendigkeit, Katalysator und Aktivator zu trennen, so dass keine lagerstabilen polymerisierbaren Zusammensetzungen bereit gestellt werden können. Katalysator und Aktivator können erst direkt vor der Polymerisation vereint werden, wobei hochreaktive und unter Wärmebildung schnell gelierende Zusammensetzungen entstehen. Die Herstellung von Formkörpern ist daher auf bestimmte Verfahren wie zum Beispiel den RIM-Prozess beschränkt. Die erhaltenen vernetzten Polymerisate weisen hohe Erweichungstemperaturen auf. Beschichtete Materialien sind nicht erwähnt.

Es wurde nun gefunden, dass Zusammensetzungen aus Verbindungen mit mindestens zwei direkt oder über eine Brückengruppe verbundenen gespannten Cycloolefinen und einem Einkomponenten-Katalysator lagerstabil sind und eine hervorragende Verarbeitbarkeit aufweisen, je nach Katalysatorwahl sogar in Gegenwart von Sauerstoff und Feuchtigkeit. Diese Zusammensetzungen können ohne besondere Vorsichtsmassnahmen mittels unterschiedlichster Formgebungsverfahren zu vernetzten Metathese-Polymerisaten verarbeitet werden. Die Polymerisate weisen hohe Vernetzungsdichten und hervorragende mechanische und elektrische Eigenschaften sowie Oberflächeneigenschaften

auf, zum Beispiel niedrige ε-Werte und tan δ-Werte, und sehr niedrige Wasseraufnahme. Die verwendeten Monomere sind hervorragende Filmbildner und die Polymerfilme weisen hervorragende Eigenschaften auf. Es wurde ferner gefunden, dass man mit den Zusammensetzungen Beschichtungen in Form vernetzter Polymere erhält, die ausserordentlich hohe Haftfestigkeiten selbst auf glatten Metalloberflächen aufweisen. Die Lagerstabilität ermöglicht die Verwendung als Beschichtungen, Lacke, Photoresists, Klebstoffe und die Herstellung von Formkörpern aller Art. Femer ist die Herstellung gummiartiger oder thermoplastischer Polymere möglich, die weiter vernetzbar sind.

Ein Gegenstand der Erfindung sind Verbindungen der Formel I

$$(A)_n\text{-}B \qquad (I),$$

worin A den Rest eines gespannten Cycloolefins, n die Zahl zwei und B die direkte Bindung oder eine Brückengruppe der Formel V

$$\text{-}X_5\text{-}R_{20}\text{-}X_6\text{-} \qquad (V)$$

bedeuten, worin

| | |
|---|---|
| $X_5$ und $X_6$ | unabhängig voneinander eine direkte Bindung, -O-, $-CH_2$-O-, -C(O)O-, -O(O)C-, $-CH_2$-O(O)C-, -C(O)-$NR_{21}$-, $-R_{21}$N-(O)C-, -NH-C(O)-$NR_{21}$-, -O-C(O)-NH-, $-CH_2$-O-C(O)-NH- oder -NH-C(O)-O- bedeuten, und |
| $R_{20}$ | $C_2$-$C_{18}$-Alkylen, unsubstituiertes oder mit $C_1$-$C_4$-Alkyl oder $C_1$-$C_4$-Alkoxy substituiertes $C_5$-$C_8$-Cycloalkylen, unsubstituiertes oder mit $C_1$-$C_4$-Alkyl oder $C_1$-$C_4$-Alkoxy substituiertes $C_6$-$C_{18}$-Arylen oder $C_7$-$C_{19}$-Aralkylen, oder Polyoxaalkylen mit 2 bis 12 Oxaalkyleneinheiten und 2 bis 6 C-Atomen im Alkylen darstellt, und |
| $R_{21}$ | H oder $C_1$-$C_6$-Alkyl ist, |

mit Ausnahme von 1,2-Bisnorbomenyl-ethan,
Norbornencarbonsäure-norbornenmethylester und Verbindungen der Formel

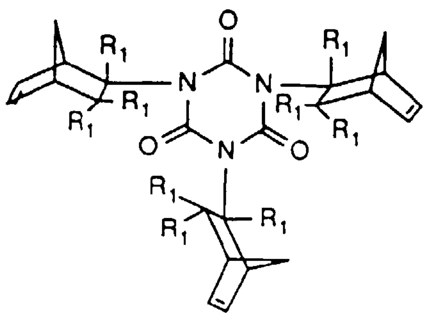

worin $R_1$ Wasserstoff oder Alkyl ist.

Bei den cyclischen Olefinen kann es sich um monocyclische oder polycyclische kondensierte und/oder überbrückte Ringsysteme, zum Beispiel mit zwei bis vier Ringen handeln, die unsubstituiert oder substituiert sind und Heteroatome wie zum Beispiel O, S, N oder Si in einem oder mehreren Ringen und/oder kondensierte alicyclische, aromatische oder heteroaromatische Ringe wie zum Beispiel o-Cyclopentylen, o-Phenylen, o-Naphthylen, o-Pyridinylen oder o-Pyrimidinylen enthalten können. Die einzelnen cyclischen Ringe können 3 bis 16, bevorzugt 3 bis 12 und besonders bevorzugt 3 bis 8 Ringglieder enthalten. Die cyclischen Olefine können weitere nichtaromatische Doppelbindungen enthalten, je nach Ringgrösse bevorzugt 2 bis 4 solcher zusätzlichen Doppelbindungen. Bei den Ringsubstituenten handelt es sich um solche, die inert sind, das heisst, die die chemische Stabilität der Einkomponenten-Katalysatoren nicht beeinträchtigen.

Ankondensierte alicyclische Ringe enthalten bevorzugt 3 bis 8, besonders bevorzugt 4 bis 7 und insbesondere bevorzugt 5 oder 6 Ring-C-Atome.

In einer bevorzugten Ausführungsform entsprechen die Reste A in Formel I Cycloolefinresten der Formel II

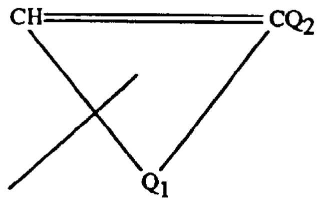

(II),

worin

| | |
|---|---|
| $Q_1$ | ein Rest mit mindestens einem Kohlenstoffatom ist, der zusammen mit der $-CH=CQ_2$-Gruppe einen mindestens 3-gliedrigen alicyclischen Ring bildet, welcher gegebenenfalls ein oder mehrere Heteroatome ausgewählt aus der Gruppe bestehend aus Silicium, Phosphor, Sauerstoff, Stickstoff und Schwefel enthält; und der unsubstituiert oder mit Halogen, =O, -CN, $-NO_2$, $R_1R_2R_3Si-(O)_u-$, -COOM, $-SO_3M$, $-PO_3M$, $-COO(M_1)_{1/2}$, $-SO_3(M_1)_{1/2}$, $-PO_3(M_1)_{1/2}$, $C_1$-$C_{20}$-Alkyl, $C_1$-$C_{20}$-Hydroxyalkyl $C_1$-$C_{20}$-Halogenalkyl, $C_1$-$C_6$-Cyanoalkyl, $C_3$-$C_8$-Cycloalkyl, $C_6$-$C_{16}$-Aryl, $C_7$-$C_{16}$-Arallcyl, $C_3$-$C_6$-Heterocycloalkyl, $C_3$-$C_{16}$-Heteroaryl, $C_4$-$C_{16}$-Heteroarakyl oder $R_4$-X- substituien ist; oder bei dem zwei benachbarte C-Atome mit -CO-O-CO- oder $-CO-NR_5-CO-$ substituiert sind; oder bei dem gegebenenfalls an benachbarten Kohlenstoffatomen des alicyclischen Rings ein aromatischer oder heteroaromatischer Ring und/oder weitere alicyclische Ringe ankondensiert sind, welche unsubstituiert oder mit Halogen, -CN, $-NO_2$, $R_6R_7R_8Si-(O)_u-$, -COOM, $-SO_3M$, $-PO_3M$, $-COO(M_1)_{1/2}$, $-SO_3(M_1)_{1/2}$, $-PO_3(M_1)_{1/2}$, $C_1$-$C_{20}$-Alkyl, $C_1$-$C_{20}$-Halogenalkyl, $C_1$-$C_{20}$-Hydroxyalkyl, $C_1$-$C_6$-Cyanoalkyl, $C_3$-$C_8$-Cycloalkyl, $C_6$-$C_{16}$-Aryl, $C_7$-$C_{16}$-Aralkyl, $C_3$-$C_6$-Heterocycloalkyl, $C_3$-$C_{16}$-Heteroaryl, $C_4$-$C_{16}$-Heteroarlkyl oder $R_{13}$-$X_1$- substituiert ist; |
| X und $X_1$ | unabhängig voneinander für -O-, -S-, -CO-, -SO-, $-SO_2-$, -O-C(O)-, -C(O)-O-, $-C(O)-NR_5-$, $-NR_{10}-C(O)-$, $-SO_2-O-$ oder $-O-SO_2-$ stehen; |
| $R_1$, $R_2$ und $R_3$ | unabhängig voneinander $C_1$-$C_{12}$-Alkyl, $C_1$-$C_{12}$-Perfluoralkyl, Phenyl oder Benzyl bedeuten; |
| $R_4$ und $R_{13}$ | unabhängig $C_1$-$C_{20}$-Alkyl, $C_1$-$C_{20}$-Halogenalkyl, $C_1$-$C_{20}$-Hydroxyalkyl, $C_3$-$C_8$-Cycloalkyl, $C_6$-$C_{16}$-Aryl, $C_7$-$C_{16}$-Aralkyl bedeuten; |
| $R_5$ und $R_{10}$ | unabhängig voneinander Wasserstoff, $C_1$-$C_{12}$-Alkyl, Phenyl oder Benzyl bedeuten, wobei die Alkylgruppen ihrerseits unsubstituiert oder mit $C_1$-$C_{12}$-Alkoxy oder $C_3$-$C_8$-Cycloalkyl substituiert sind; |
| $R_6$, $R_7$ und $R_8$ | unabhängig voneinander $C_1$-$C_{12}$-Alkyl, $C_1$-$C_{12}$-Perfluoralkyl, Phenyl oder Benzyl bedeuten; |
| M | für ein Alkalimetall und $M_1$ für ein Erdalkalimetall stehen; und |
| u | für 0 oder 1 steht; |

wobei der mit $Q_1$ gebildete alicyclische Ring gegebenenfalls weitere nicht-aromatische Doppelbindungen enthält;

| | |
|---|---|
| $Q_2$ | Wasserstoff, $C_1$-$C_{20}$-Alkyl, $C_1$-$C_{20}$-Halogenalkyl, $C_1$-$C_{12}$-Alkoxy, Halogen, -CN oder $R_{11}$-$X_2$- darstellt; |
| $R_{11}$ | $C_1$-$C_{20}$-Alkyl, $C_1$-$C_{20}$-Halogenalkyl, $C_1$-$C_{20}$-Hydroxyalkyl, $C_3$-$C_8$-Cycloalkyl, $C_6$-$C_{16}$-Aryl oder $C_7$-$C_{16}$-Aralkyl bedeutet; |
| $X_2$ | -C(O)-O- oder $-C(O)-NR_{12}-$ ist; |
| $R_{12}$ | Wasserstoff, $C_1$-$C_{12}$-Alkyl, Phenyl oder Benzyl darstellt; |

wobei die vorgenannten Cycloalkyl-, Heterocycloalkyl-, Aryl-, Heteroaryl-, Aralkyl- und Heteroaralkylgruppen unsubstituiert oder mit $C_1$-$C_{12}$-Alkyl, $C_1$-$C_{12}$-Alkoxy, $-NO_2$, -CN oder Halogen substituiert sind, und wobei die Heteroatome der vorgenannten Heterocycloalkyl-, Heteroaryl- und Heteroaralkylgruppen aus der Gruppe -O-, -S-, $-NR_9-$ und -N= ausgewählt sind; und

$R_9$ Wasserstoff, $C_1$-$C_{12}$-Alkyl, Phenyl oder Benzyl darstellt.

Die Stellung der Doppelbindung im Ring der Formel II zur freien Bindung hängt im wesentlichen von der Ringgrösse und Herstellungsmethode der Verbindungen der Formel I ab.

Ist in den Verbindungen der Formel II ein asymmetrisches Zentrum vorhanden, so hat dies zur Folge, daß die Verbindungen in optisch isomeren Formen auftreten können. Einige Verbindungen der Formel II können in tautomeren Formen (z.B. Keto-Enol-Tautomerie) vorkommen. Liegt eine aliphatische C=C-Doppelbindung vor, so kann auch geometrische Isomerie (E-Form oder Z-Form) auftreten. Ferner sind auch Exo-Endo-Konfigurationen möglich. Die

Formel II umfaßt somit alle möglichen Stereoisomere, die in Form von Enantiomeren, Tautomeren, Diastereomeren, E/Z-Isomeren oder deren Gemische vorliegen.

In den Definitionen der Substituenten können die Alkyl-, Alkenyl- und Alkinylgruppen geradkettig oder verzweigt sein. Dasselbe gilt auch für den bzw. jeden Alkylteil von Alkoxy-, Alkylthio-, Alkoxycarbonyl- und von weiteren Alkyl-enthaltenden Gruppen. Diese Alkylgruppen enthalten bevorzugt 1 bis 12, bevorzugter 1 bis 8 und besonders bevorzugt 1 bis 4 C-Atome. Diese Alkenyl- und Alkinylgruppen enthalten bevorzugt 2 bis 12, bevorzugter 2 bis 8 und besonders bevorzugt 2 bis 4 C-Atome.

Alkyl umfaßt beispielsweise Methyl, Ethyl, Isopropyl, n-Propyl, n-Butyl, iso-Butyl, sek-Butyl, tert-Butyl sowie die verschiedenen isomeren Pentyl-, Hexyl-, Heptyl-, Octyl-, Nonyl-, Decyl-, Undecyl-, Dodecyl-, Tridecyl-, Tetradecyl, Pentadecyl, Hexadecyl-, Heptadecyl, Octadecyl-, Nonadecyl- und Eicosylradikale.

Hydroxyalkyl umfaßt beispielsweise Hydroxymethyl, Hydroxyethyl, 1-Hydroxyisopropyl, 1-Hydroxy-n-propyl, 2-Hydroxy-n-butyl, 1-Hydroxy-iso-butyl, 1-Hydroxy-sek-butyl, 1-Hydroxy-tert-Butyl sowie die verschiedenen isomeren Pentyl-, Hexyl-, Heptyl-, Octyl-, Nonyl-, Decyl-, Undecyl-, Dodecyl-, Tridecyl-, Tetradecyl, Pentadecyl, Hexadecyl-, Heptadecyl, Octadecyl-, Nonadecyl- und Eicosylradikale.

Halogenalkyl umfaßt beispielsweise Fluormethyl, Difluormethyl, Trifluormethyl, Chlormethyl, Dichlormethyl, Trichlormethyl, 2,2,2-Trifluorethyl, 2-Fluorethyl, 2-Chlorethyl, 2,2,2-Trichlorethyl sowie halogenierte, besonders fluorierte oder chlorierte Alkane, wie zum Beispiel der Isopropyl-, n-Propyl-, n-Butyl-, iso-Butyl-, sek-Butyl-, tert-Butyl-, und der verschiedenen isomeren Pentyl-, Hexyl-, Heptyl-, Octyl-, Nonyl-, Decyl-, Undecyl-, Dodecyl-, Tridecyl-, Tetradecyl-, Pentadecyl-, Hexadecyl-, Heptadecyl-, Octadecyl-, Nonadecyl- und Eicosylradikale.

Alkenyl umfaßt zum Beispiel Propenyl, Isopropenyl, 2-Butenyl, 3-Butenyl, Isobutenyl, n-Penta-2,4-dienyl, 3-Methyl-but-2-enyl, n-Oct-2-enyl, n-Dodec-2-enyl, iso-Dodecenyl, n-Octadec-2-enyl und n-Octadec-4-enyl.

Beim Cycloalkyl handelt es sich bevorzugt um $C_5$-$C_8$-Cycloalkyl, besonders um $C_5$- oder $C_6$-Cycloalkyl. Einige Beispiele sind Cyclopropyl, Dimethylcyclopropyl, Cyclobutyl, Cyclopentyl, Methylcyclopentyl, Cyclohexyl, Cycloheptyl und Cyclooctyl.

Cyanoalkyl umfaßt beispielsweise Cyanomethyl (Methylnitril), Cyanoethyl (Ethylnitril), 1-Cyanoisopropyl, 1-Cyano-n-propyl, 2-Cyano-n-butyl, 1-Cyano-iso-butyl, 1-Cyano-sek-butyl, 1-Cyano-tert-butyl sowie die verschiedenen isomeren Cyanopentyl- und -hexylreste.

Aralkyl enthält bevorzugt 7 bis 12 C-Atome und besonders bevorzugt 7 bis 10 C-Atome. Es kann sich zum Beispiel um Benzyl, Phenethyl, 3-Phenylpropyl, $\alpha$-Methylbenzyl, Phenbutyl oder $\alpha,\alpha$-Dimethylbenzyl handeln.

Aryl enthält bevorzugt 6 bis 10 C-Atome. Es kann sich beispielsweise um Phenyl, Pentalin, Inden, Naphthalin, Azulin oder Anthracen handeln.

Heteroaryl enthält bevorzugt 4 oder 5 C-Atome und ein oder zwei Heteroatome aus der Gruppe O, S und N. Es kann sich beispielsweise um Pyrrol, Furan, Thiophen, Oxazol, Thiazol, Pyridin, Pyrazin, Pyrimidin, Pyridazin, Indol, Purin oder Chinolin handeln.

Heterocycloalkyl enthält bevorzugt 4 oder 5 C-Atome und ein oder zwei Heteroatome aus der Gruppe O, S und N. Es kann sich beispielsweise um Oxiran, Azirin, 1,2-Oxathiolan, Pyrazolin, Pyrrolidin, Piperidin, Piperazin, Morpholin, Tetrahydrofuran oder Tetrahydrothiophen handeln.

Alkoxy ist beispielsweise Methoxy, Ethoxy, Propyloxy, i-Propyloxy, n-Butyloxy, i-Butyloxy, sek.-Butyloxy oder t-Butyloxy.

Unter Alkalimetall ist im Rahmen der vorliegenden Erfindung Lithium, Natrium, Kalium, Rubidium und Cäsium, insbesondere Lithium, Natrium und Kalium zu verstehen.

Unter Erdalkalimetall ist im Rahmen der vorliegenden Erfindung Beryllium, Magnesium, Calcium, Strontium und Barium, insbesondere Magnesium und Calcium zu verstehen.

In den obigen Definitionen ist unter Halogen, Fluor, Chlor, Brom und Jod, vorzugsweise Fluor, Chlor und Brom zu verstehen.

In den Resten der Formel II bedeutet $Q_2$ bevorzugt Wasserstoff.

Ferner sind Verbindungen mit Resten der Formel II bevorzugt, worin der alicyclische Ring, den $Q_1$ zusammen mit der -CH=C$Q_2$- Gruppe bildet, 3 bis 16, bevorzugter 3 bis 12 und besonders bevorzugt 3 bis 8 Ringatome aufweist, und wobei es sich um ein monocyclisches, bicyclisches, tricyclisches oder tetracyclisches kondensiertes Ringsystem handeln kann.

Mit besonderem Vorteil läßt sich das erfindungsgemäße Verfahren mit denjenigen Verbindungen mit Resten der Formel II durchführen, worin

$Q_1$ ein Rest mit mindestens einem Kohlenstoffatom ist, das zusammen mit der -CH=C$Q_2$-Gruppe einen 3- bis 20-gliedrigen alicyclischen Ring bildet, welcher gegebenenfalls ein oder mehrere Heteroatome ausgewählt aus der Gruppe Silicium, Sauerstoff, Stickstoff und Schwefel enthält; und der unsubstituiert oder mit Halogen, =O, -CN, $-NO_2$, $R_1R_2R_3Si-(O)_u-$, -COOM, $-SO_3M$, $-PO_3M$, $-COO(M_1)_{1/2}$, $-SO_3(M_1)_{1/2}$, $-PO_3(M_1)_{1/2}$, $C_1$-$C_{12}$-Alkyl, $C_1$-$C_{12}$-Halogenalkyl, $C_1$-$C_{12}$-Hydroxyalkyl, $C_1$-$C_4$-

Cyanoalkyl, $C_3$-$C_6$-Cycloalkyl, $C_6$-$C_{12}$-Aryl, $C_7$-$C_{12}$-Aralkyl, $C_3$-$C_6$-Heterocycloalkyl, $C_3$-$C_{12}$-Heteroaryl, $C_4$-$C_{12}$-Heteroaralkyl oder $R_4$-X- substituiert ist; oder bei dem zwei benachbarte C-Atome in diesem Rest $Q_1$ mit -CO-O-CO- oder -CO-$NR_5$-CO- substituiert sind; oder bei dem gegebenenfalls an benachbarten Kohlenstoffatomen ein aromatischer oder heteroaromatischer Ring und/oder weitere alicyclische Ringe ankondensiert sind, welche unsubstituiert oder mit Halogen, -CN, -$NO_2$, $R_6R_7R_8$Si-, -COOM, -$SO_3$M, -$PO_3$M, -COO$(M_1)_{1/2}$, -SO$((M_1)_{1/2}$, -$PO_3(M_1)_{1/2}$, $C_1$-$C_{12}$-Alkyl, $C_1$-$C_{12}$-Halogenahyl, $C_1$-$C_{12}$-Hydroxyalkyl, $C_1$-$C_4$-Cyanoalkyl, $C_3$-$C_6$-Cycloalkyl, $C_6$-$C_{12}$-Aryl, $C_7$-$C_{12}$-Aralkyl, $C_3$-$C_6$-Heterocycloalkyl, $C_3$-$C_{12}$-Heteroaryl, $C_4$-$C_{12}$-Heteroaralkyl oder $R_{13}$-$X_1$- substituiert sind;

X und $X_1$ unabhängig voneinander für -O-, -S-, -CO-, -SO-, -$SO_2$-, -O-C(O)-, -C(O)-O-, -C(O)-$NR_5$-, -$NR_{10}$-C(O)-, -$SO_2$-O- oder -O-$SO_2$- stehen; und

$R_1$, $R_2$ und $R_3$ unabhängig voneinander $C_1$-$C_6$-Alkyl, $C_1$-$C_6$-Perfluoralkyl, Phenyl oder Benzyl bedeuten;

M für ein Alkalimetall und $M_1$ für ein Erdalkalimetall stehen;

$R_4$ und $R_{13}$ unabhängig voneinander $C_1$-$C_{12}$-Alkyl, $C_1$-$C_{12}$-Halogenalkyl, $C_1$-$C_{12}$-Hydroxyalkyl, $C_3$-$C_8$-Cycloalkyl, $C_6$-$C_{12}$-Aryl, $C_7$-$C_{12}$-Aralkyl bedeuten;

$R_5$ und $R_{10}$ unabhängig voneinander Wasserstoff, $C_1$-$C_6$-Alkyl, Phenyl oder Benzyl bedeuten,

wobei die Alkylgruppen ihrerseits unsubstituiert oder mit $C_1$-$C_6$-Alkoxy oder $C_3$-$C_6$-Cycloalkyl substituiert sind;

$R_6$, $R_7$ und $R_8$ unabhängig voneinander $C_1$-$C_6$-Alkyl, $C_1$-$C_6$-Perfluoralkyl, Phenyl oder Benzyl darstellen;

u für 0 oder 1 steht;

wobei der mit $Q_1$ gebildete alicyclische Ring gegebenenfalls weitere nichtaromatische Doppelbindungen enthält;

$Q_2$ Wasserstoff, $C_1$-$C_{12}$-Alkyl, $C_1$-$C_{12}$-Halogenalkyl, $C_1$-$C_6$-Alkoxy, Halogen, -CN oder $R_{11}$-$X_2$- bedeutet;

$R_{11}$ $C_1$-$C_{12}$-Alkyl, $C_1$-$C_{12}$-Halogenalkyl, $C_1$-$C_{12}$-Hydroxyalkyl, $C_3$-$C_6$-Cycloalkyl, $C_6$-$C_{12}$-Aryl oder $C_7$-$C_{12}$-Aralkyl darstellt;

$X_2$ -C(O)-O- oder -C(O)-$NR_{12}$- ist; und

$R_{12}$ Wasserstoff, $C_1$-$C_6$-Alkyl, Phenyl oder Benzyl bedeutet;

und wobei die Cycloalkyl-, Heterocycloalkyl-, Aryl-, Heteroaryl-, Aralkyl- und Heteroaralkylgruppen unsubstituiert oder mit $C_1$-$C_6$-Alkyl, $C_1$-$C_6$-Alkoxy, -$NO_2$, -CN oder Halogen substituiert sind, und wobei die Heteroatome der Heterocycloalkyl-, Heteroaryl- und Heteroaralkylgruppen aus der Gruppe -O-, -S-, -$NR_9$- und -N= ausgewählt sind; und $R_9$ Wasserstoff, $C_1$-$C_6$-Alkyl, Phenyl oder Benzyl bedeutet.

Aus dieser Gruppe sind diejenigen Verbindungen mit einem Rest der Formel II bevorzugt, worin

$Q_1$ ein Rest mit mindestens einem Kohlenstoffatom ist, der zusammen mit der -CH=$CQ_2$-Gruppe einen 3- bis 10-gliedrigen alicyclischen Ring bildet, der gegebenenfalls ein Heteroatom ausgewählt aus der Gruppe Silicium, Sauerstoff, Stickstoff und Schwefel enthält, und der unsubstituiert oder mit Halogen, -CN, -$NO_2$, $R_1R_2R_3$Si-, -COOM, -$SO_3$M, -$PO_3$M, -COO$(M_1)_{1/2}$, -$SO_3(M_1)_{1/2}$, -$PO_3(M_1)_{1/2}$, $C_1$-$C_6$-Alkyl, $C_1$-$C_6$-Halogenalkyl, $C_1$-$C_6$-Hydroxyalkyl, $C_1$-$C_4$-Cyanoalkyl, $C_3$-$C_6$-Cycloalkyl, Phenyl, Benzyl oder $R_4$-X- substituiert ist; oder bei dem an benachbarten Kohlenstoffatomen gegebenenfalls ein aromatischer oder heteroaromatischer Ring ankondensiert ist, welcher unsubstituiert oder durch Halogen, -CN, -$NO_2$, $R_6R_7R_8$Si-, -COOM, -$SO_3$M, -$PO_3$M, -COO$(M_1)_{1/2}$, -$SO_3(M_1)_{1/2}$, -$PO_3(M_1)_{1/2}$, $C_1$-$C_6$-Alkyl, $C_1$-$C_6$-Halogenalkyl, $C_1$-$C_6$-Hydroxyalkyl, $C_1$-$C_4$-Cyanoalkyl, $C_3$-$C_6$-Cycloalkyl, Phenyl, Benzyl oder $R_{13}$-$X_1$- substituiert ist;

$R_1$, $R_2$ und $R_3$ unabhängig voneinander $C_1$-$C_4$-Alkyl, $C_1$-$C_4$-Perfluoralkyl, Phenyl oder Benzyl bedeuten;

M für ein Alkalimetall und $M_1$ für ein Erdalkalimetall stehen;

$R_4$ und $R_{13}$ unabhängig voneinander $C_1$-$C_6$-Alkyl, $C_1$-$C_6$-Halogenalkyl, $C_1$-$C_6$-Hydroxyalkyl oder $C_3$-$C_6$-Cycloalkyl bedeuten;

X und $X_1$ unabhängig voneinander für -O-, -S-, -CO-, -SO- oder -$SO_2$- stehen;

$R_6$, $R_7$ und $R_8$ unabhängig voneinander $C_1$-$C_4$-Alkyl, $C_1$-$C_4$-Perfluoroalkyl, Phenyl oder Benzyl darstellen; und

$Q_2$ Wasserstoff bedeutet.

Besonders bevorzugt handelt es sich bei dem Cycloolefinrest der Formel II um unsubstituiertes oder substituiertes Cyclopropenyl, Cyclobutenyl, Cyclopentenyl, Cycloheptenyl, Cyclooctenyl, Cyclopentadienyl, Cyclohexadienyl, Cycloheptadienyl, Cyclooctadienyl oder Norbornenyl oder Norbornenylderivate wie zum Beispiel 7-Oxa-2,2,2-cyclohepten sowie die entsprechenden Benzoderivate. Substituenten sind bevorzugt $C_1$-$C_4$-Alkyl und $C_1$-$C_4$-Alkoxy.

Insbesondere geeignet sind als Reste der Formel II Norbomenyl und Norbornenylderivate. Von diesen Norbornenylderivaten sind diejenigen besonders bevorzugt, die entweder der Formel III

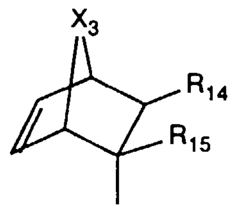

(III),

worin

$X_3$ $-CHR_{16}-$, Sauerstoff oder Schwefel;
$R_{14}$ und $R_{15}$ unabhängig voneinander Wasserstoff, -CN, Trifluormethyl, $(CH_3)_3Si-O-$, $(CH_3)_3Si-$ oder $-COOR_{17}$; und
$R_{16}$ und $R_{17}$ unabhängig voneinander Wasserstoff, $C_1$-$C_{12}$-Alkyl, Phenyl oder Benzyl bedeuten;

oder der Formel IV entsprechen

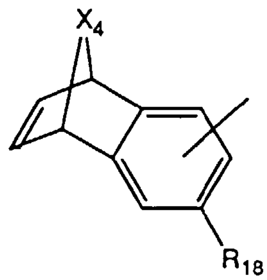

(IV),

worin

$X_4$ $-CHR_{19}-$, Sauerstoff oder Schwefel;
$R_{19}$ Wasserstoff, $C_1$-$C_{12}$-Alkyl, Phenyl oder Benzyl; und
$R_{18}$ Wasserstoff, $C_1$-$C_6$-Alkyl oder Halogen bedeuten.

Besonders bevorzugt handelt es sich bei dem Cycloolefinrest der Formel II um Norbornenyl der Formel

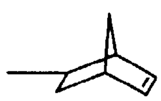

.

In Formel I steht n bevorzugt für eine ganze Zahl von 2 bis 6, besonders bevorzugt 2 bis 4 und insbesondere bevorzugt 2 oder 3.

In Formel I bedeutet B bevorzugt eine n-wertige Brückengruppe.

Als zweiwertige Brückengruppen kommen zum Beispiel solche der Formel V in Frage,

$$-X_5-R_{20}-X_6- \quad (V),$$

worin

$X_5$ und $X_6$ unabhängig voneinander eine direkte Bindung, -O-, $-CH_2-O-$, -C(O)O-, -O(O)C-, $-CH_2-O(O)C-$, $-C(O)-NR_{21}-$, $-R_{21}N-(O)C-$, $-NH-C(O)-NR_{21}-$, -O-C(O)-NH-, $-CH_2-O-C(O)-NH-$ oder -NH-C(O)-O- bedeuten, und

$R_{20}$ $C_2$-$C_{18}$-Alkylen, unsubstituiertes oder mit $C_1$-$C_4$-Alkyl oder $C_1$-$C_4$-Alkoxy substituiertes $C_5$-$C_8$-Cycloalkylen, unsubstituiertes oder mit $C_1$-$C_4$-Alkyl oder $C_1$-$C_4$-Alkoxy substituiertes $C_6$-$C_{18}$-Arylen oder $C_7$-$C_{19}$-Aralkylen, oder Polyoxaalkylen mit 2 bis 12 Oxaalkyleneinheiten und 2 bis 6 C-Atomen im Alkylen darstellt, und

$R_{21}$ H oder $C_1$-$C_6$-Alkyl ist.

$R_{20}$ enthält als Alkylen bevorzugt 2 bis 12 und besonders bevorzugt 2 bis 8 C-Atome. Das Alkylen kann linear oder verzweigt sein. Bevorzugtes Cycloalkylen ist Cyclopentylen und besonders Cyclohexylen. Einige Beispiele für Arylen sind Phenylen, Naphthylen, Biphenylen, Biphenylenether und Anthracenylen. Ein Beispiel für Aralkylen ist Benzylen. Das Polyoxaalkylen enthält bevorzugt 2 bis 6 und besonders bevorzugt 2 bis 4 Einheiten, und bevorzugt 2 oder 3 C-Atome im Alkylen.

In einer bevorzugten Ausführungsform stehen in Formel V

a) $X_5$ und $X_6$ für eine direkte Bindung und $R_{20}$ für $C_2$-$C_{18}$-Alkylen, bevorzugter $C_2$-$C_{12}$-Alkylen, oder

b) $X_5$ und $X_6$ für -O-, -$CH_2$-O-, -C(O)O-, -O(O)C-, -$CH_2$-O(O)C-, -C(O)-$NR_{21}$-, -O-C(O)-NH- oder -$CH_2$-O-C(O)-NH- und $R_{20}$ für $C_2$-$C_{12}$-Alkylen, unsubstituiertes oder mit $C_1$-$C_4$-Alkyl oder $C_1$-$C_4$-Alkoxy substituiertes Phenylen, Naphthylen oder Benzylen oder -$R_{22}$-(O-$R_{22}$-$)_x$-O$R_{22}$-, worin x eine Zahl von 2 bis 4 ist und $R_{22}$ -$C_2$-$C_4$-Alkylen bedeutet.

Einige Beispiele für Verbindungen der Formel I mit einer zweiwertigen Brückengruppe sind

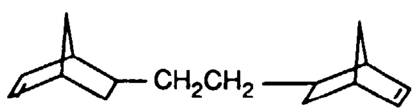

(0)

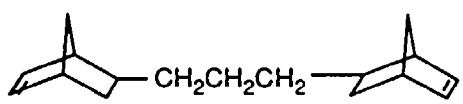

(1),

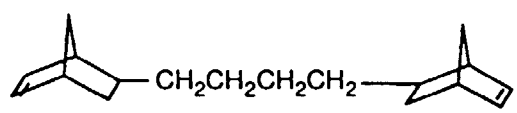

(2),

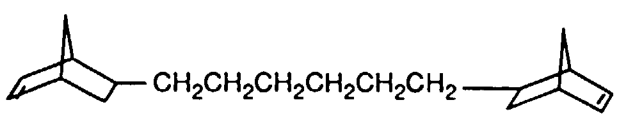

(3),

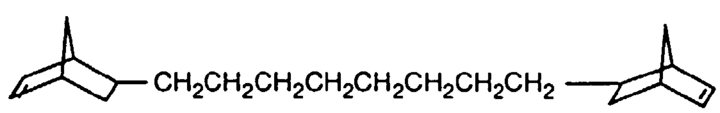

(4),

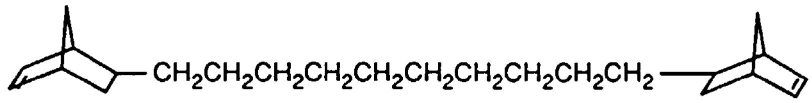

(5),

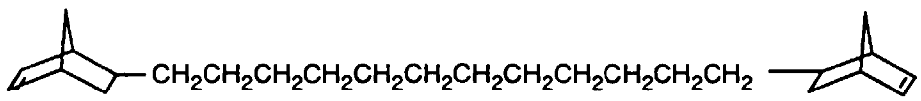

(6)

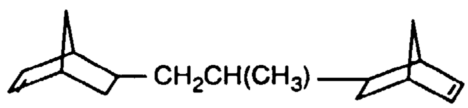

(7),

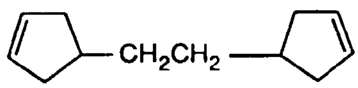

(8),

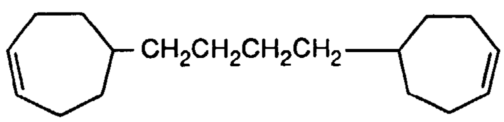

(9),

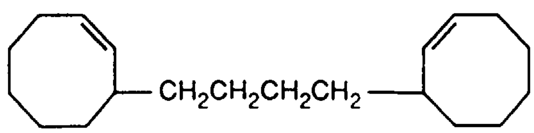

(10),

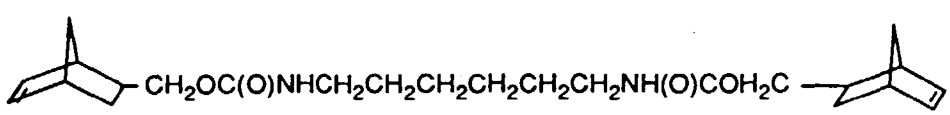

(11),

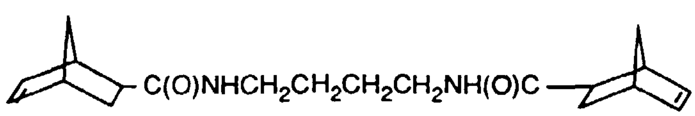

(12),

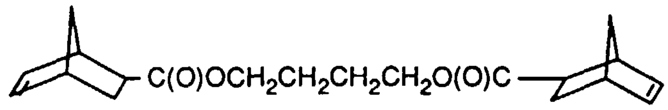

(13),

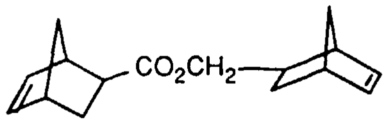

(13a),

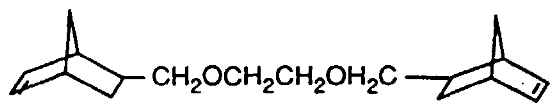

(14),

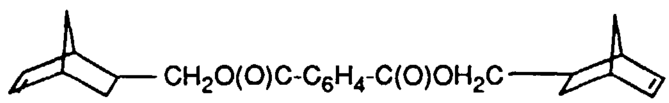

(15),

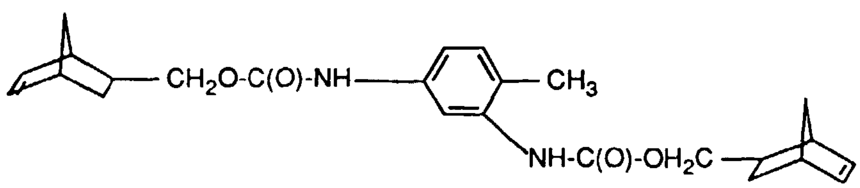

(16),

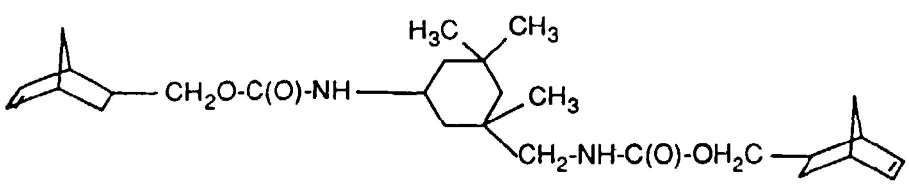

(17).

Die Verbindungen der Formel I mit einer Brückengruppe der Formel V, die eine reine Kohlenwasserstoffbrücke darstellt, sind zum Beispiel mittels Diels-Alder Reaktion eines cyclischen Diens mit einem linearen oder verzweigten aliphatischen Dien erhältlich (siehe auch EP 287,762), wobei oft Stoffgemische entstehen, die entweder direkt weiterverwendet oder zuvor mittels üblicher Methoden getrennt werden. Verbindungen der Formel I mit einer Brückengruppe der Formel V, worin $X_5$ und $X_6$ keine direkte Bindung darstellt, sind aus den entsprechenden Halogeniden oder Dihalogeniden, Alkoholen oder Diolen, Aminen oder Diaminen, Carbonsäuren oder Dicarbonsäuren oder Isocyanaten oder Diisocyanaten in an sich bekannter Weise über Veretherungs-, Veresterungs- beziehungsweise Amidierungsreaktionen erhältlich.

Als dreiwertige Brückengruppen kommen zum Beispiel solche der Formel VI in Frage,

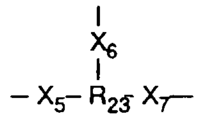 (VI),

worin

$X_5$, $X_6$ und $X_7$ -O-, -$CH_2$-O-, -C(O)O-, -O(O)C-, -$CH_2$-O(O)C-, -C(O)-$NR_{21}$-, -$R_{21}$N-(O)C-, -NH-C(O)-$NR_{21}$-, -O-C(O)-NH-, -$CH_2$-O-C(O)-NH- oder -NH-C(O)-O- bedeuten, und
$R_{23}$ einen dreiwertigen aliphatischen Kohlenwasserstoffrest mit 3 bis 20, bevorzugt 3 bis 12 C-Atomen, einen dreiwertigen unsubstituierten oder mit $C_1$-$C_4$-Alkyl oder $C_1$-$C_4$-Alkoxy substituierten cycloaliphatischen Rest mit 3 bis 8, vorzugsweise 5 oder 6 Ring-C-Atomen, oder einen dreiwertigen unsubstituierten oder mit $C_1$-$C_4$-Alkyl oder $C_1$-$C_4$-Alkoxy substituierten aromatischen Rest mit 6 bis 18, vorzugsweise 6 bis 12 C-Atomen, einen unsubstituierten oder mit $C_1$-$C_4$-Alkyl oder $C_1$-$C_4$-Alkoxy substituierten dreiwertigen araliphatischen Rest mit 7 bis 19, bevorzugt 7 bis 12 C-Atomen, oder einen unsubstituierten oder mit $C_1$-$C_4$-Alkyl oder $C_1$-$C_4$-Alkoxy substituierten dreiwertigen heteroaromatischen Rest mit 3 bis 13 C-Atomen und 1 bis drei Heteroatomen aus der Gruppe -O-, -N- und -S- darstellt, und
$R_{21}$ H oder $C_1$-$C_6$-Alkyl ist.

In einer bevorzugten Ausführungsform stellen $X_5$, $X_6$ und $X_7$ -O-, -$CH_2$-O-, -C(O)O-, -O(O)C-, -$CH_2$-O(O)C-, -C(O)-$NR_{21}$-, -O-C(O)-NH- oder -$CH_2$-O-C(O)-NH- dar.

Bevorzugte Reste $R_{23}$ leiten sich zum Beispiel von Triolen wie Glycerin, Trimethylolpropan, Butantriol, Pentantriol, Hexantriol, Trihydroxycyclohexan, Trihydroxybenzol und Cyanursäure; Triaminen wie Diethylentriamin; Tricarbonsäuren wie Cyclohexantricarbonsäure oder Trimellithsäure; und Triisocyanaten wie Benzoltriisocyanat oder Cyanurtriisocyanat ab.

Einige Beispiele für Verbindungen der Formel I mit einer dreiwertigen Brückengruppe sind

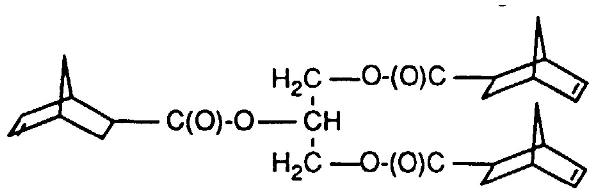 (18),

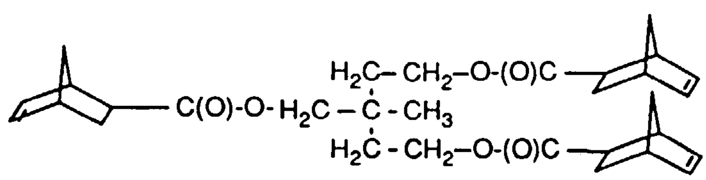 (19),

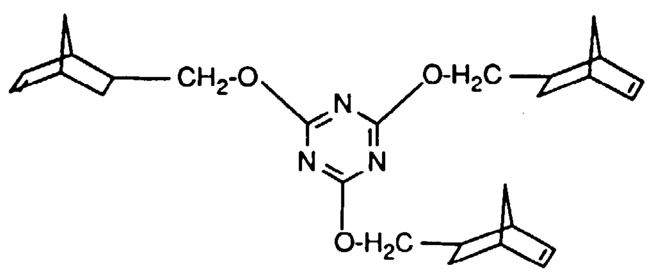 (20),

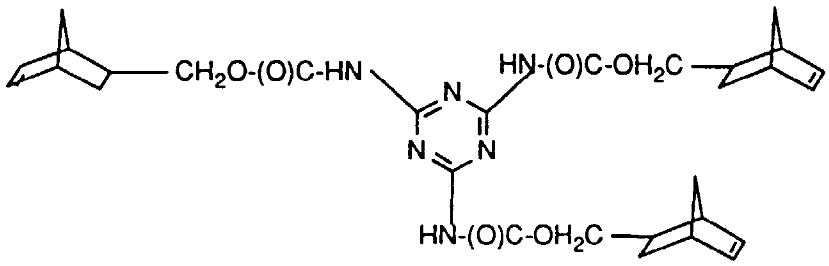

(21),

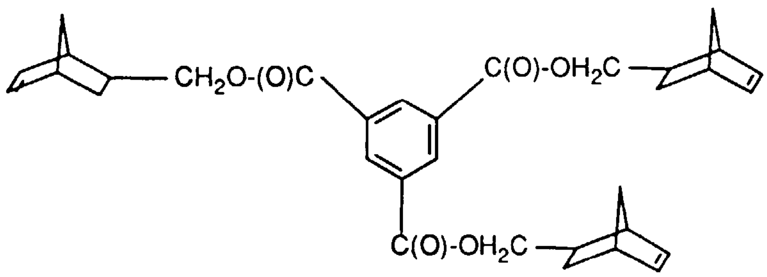

(22).

Als vierwertige Brückengruppen kommen zum Beispiel solche der Formel VII in Frage,

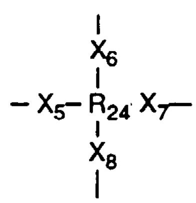

(VII),

worin

$X_5$, $X_6$, $X_7$ und $X_8$ -C(O)O-, $-CH_2$-O(O)C- oder -C(O)-$NR_{21}$- bedeuten, und

$R_{24}$ einen vierwertigen aliphatischen Kohlenwasserstoffrest mit 4 bis 20, bevorzugt 4 bis 12 C-Atomen, einen vierwertigen unsubstituierten oder mit $C_1$-$C_4$-Alkyl oder $C_1$-$C_4$-Alkoxy substituierten cycloaliphatischen Rest mit 4 bis 8, vorzugsweise 5 oder 6 Ring-C-Atomen, oder einen vierwertigen unsubstituierten oder mit $C_1$-$C_4$-Alkyl oder $C_1$-$C_4$-Alkoxy substituierten aromatischen Rest mit 6 bis 18, vorzugsweise 6 bis 12 C-Atomen, einen vierwertigen unsubstituierten oder mit $C_1$-$C_4$-Alkyl oder $C_1$-$C_4$-Alkoxy substituierten araliphatischen Rest mit 7 bis 19, bevorzugt 7 bis 12 C-Atomen, oder einen vierwertigen unsubstituierten oder mit $C_1$-$C_4$-Alkyl oder $C_1$-$C_4$-Alkoxy substituierten heteroaromatischen Rest mit 3 bis 13 C-Atomen und 1 bis drei Heteroatomen aus der Gruppe -O-, -N- und -S- darstellt, und

$R_{21}$ H oder $C_1$-$C_6$-Alkyl ist.

Einige Beispiele von tetrafunktionellen Verbindungen, von denen sich $R_{24}$ ableiten kann, sind Pentaerythrit, Pyromellithsäure und 3,4,3',4'-Biphenyltetracarbonsäure.

Als Herstellungsmethoden können die gleichen Methoden angewendet werden wie zur Herstellung der zuvorgenannten Verbindungen mit einem zwei- oder dreiwertigen Rest.

Einige Beispiele für Verbindungen der Formel I mit einer vierwertigen Brückengruppe sind

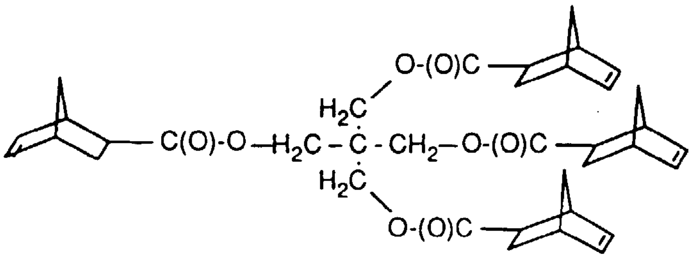

(23),

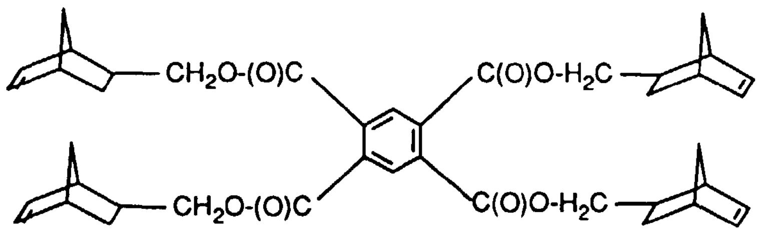

(24).

Als Beispiel für mehr als vierwertige Verbindungen, von denen sich die Brückengruppe ableiten kann, seien Polyole wie Dipentaerythrit oder Hexahydroxyhexan genannt, die mit entsprechenden Cycloolefinmonocarbonsäuren umgesetzt werden können.

In einer besonders bevorzugten Ausführungsform der Erfindung enthalten die Verbindungen der Formel I nur Kohlenstoff- und Wasserstoffatome, da Polymere daraus insofern ökologisch wertvoll sind, als sie durch einfache Pyrrolyseverfahren recyclisiert werden können.

Die Verbindungen der Formel I eignen sich als Vernetzungsmittel bei der thermisch induzierten oder strahlungsinduzierten Polymerisation von olefinisch ungesättigten Verbindungen. Die Verbindungen der Formel I eignen sich alleine oder zusammen mit anderen zur Metathesepolymerisation fähigen Monomeren hervorragend zur Herstellung von vernetzten Metathesepolymerisaten unter Verwendung von thermischen oder photochemischen Einkomponenten-Katalysatoren.

Ein weiterer Gegenstand der Erfindung ist eine Zusammensetzung aus (a) mindestens einer Verbindung der Formel I

$$(A)_n\text{-}B \qquad (I),$$

worin A den Rest eines gespannten Cycloolefins bedeutet, B für eine direkte Bindung oder eine n-wertige Brückengruppe steht, und n eine ganze Zahl von 2 bis 8 darstellt, und (b) einer katalytischen Menge mindestens eines thermo- oder strahlungsaktivierbaren Einkomponenten-Katalysators für eine Metathesepolymerisation, mit Ausnahme von Norbornencarbonsäure(norbornenmethyl)ester der Formel

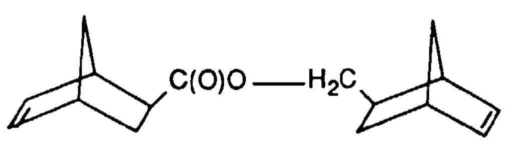

in Kombination mit einer katalytischen Menge mindestens einer thermostabilen Molybdän(VI)- oder Wolfram(VI)verbindung, die mindestens zwei Methylgruppen oder zwei monosubstituierte Methylgruppen am Metall gebunden enthält, wobei der Substituent kein Wasserstoffatom in α-Stellung enthält.

Für A, B und n gelten die zuvor angegebenen Bevorzugungen.

Thermostabilität bedeutet im Rahmen der Erfindung, dass die photokatalytisch aktiven Metallverbindungen unter Erwärmen keine aktiven Spezies für die Ringöffnungs-Metathese-Polymerisation bilden. Der Katalysator kann zum Beispiel bei Raumtemperatur bis leicht erhöhter Temperatur wie etwa + 40°C innerhalb von Wochen unter Lichtausschluß keine Ringöffnungs-Metathese-Polymerisation initiieren. Während dieser Zeit wird nur eine unbedeutende Menge an Monomer (weniger als 0,2 Gew.-%) umgesetzt. Die Thermostabilität kann zum Beispiel bestimmt werden, indem man eine Toluol-Lösung mit 20 Gew.-% Monomer und 0,33 Gew.-% Metallkatalysator bei 50°C 96 Stunden in der Dunkelheit lagert und eine eventuell gebildete Polymermenge, erkennbar am Viskositätsaufbau und durch Ausfällen in einem Fällungsmittel, beispielsweise Ethanol, Filtration und Trocknen quantitativ bestimmbar, nicht mehr als 0,5 Gew.-% und bevorzugt nicht mehr als 0,2 Gew.-% beträgt.

Vorteilhaft enthalten die erfindungsgemässen Zusammensetzungen die nachfolgenden neuen thermischen und/oder photochemischen Einkomponenten-Katalysatoren:

1. Strahlungsaktivierbare, thermostabile Ruthenium- oder Osmiumverbindungen, die mindestens einen photolabilen an das Ruthenium- oder Osmiumatom gebundenen Liganden besitzen, und deren restliche Koordinationsstellen mit nicht-photolabilen Liganden abgesättigt sind.

Als Liganden für die erfindungsgemäss zu verwendenden Ruthenium- und Osmiumverbindungen werden organische oder anorganische Verbindungen, Atome oder Ionen bezeichnet, die an ein Metallzentrum koordiniert sind.

Photolabiler Ligand bedeutet im Rahmen der vorliegenden Erfindung, daß bei Bestrahlung des Katalysators durch Licht im sichtbaren oder ultravioletten Spektralbereich der Ligand vom Katalysator dissoziiert und eine katalytisch aktive Spezies für die Metathesepolymerisation bildet. Erfindungsgemäß bevorzugt sind nicht-ionische photolabile Liganden.

Bei den photolabilen Liganden kann es sich zum Beispiel um Stickstoff ($N_2$), um unsubstituierte oder mit OH, $C_1$-$C_4$-Alkyl, $C_1$-$C_4$-Alkoxy, $C_6$-$C_{12}$-Aryl oder Halogen substituierte monocyclische, polycyclische oder kondensierte Arene mit 6 bis 24, bevorzugt 6 bis 18 und besonders bevorzugt 6 bis 12 C-Atomen oder um unsubstituierte oder mit $C_1$-$C_4$-Alkyl, $C_1$-$C_4$-Alkoxy oder Halogen substituierte monocyclische Heteroarene, kondensierte Heteroarene oder kondensierte Aren-Heteroarene mit 3 bis 22, bevorzugt 4 bis 16 und besonders 4 bis 10 C-Atomen und 1 bis 3 Heteroatomen ausgewählt aus der Gruppe O, S und N; oder um unsubstituierte oder mit $C_1$-$C_4$-Alkyl, $C_1$-$C_4$-Alkoxy oder Halogen substituierte aliphatische, cycloaliphatische, aromatische oder araliphatische Nitrile mit 1 bis 22, bevorzugt 1 bis 18, besonders bevorzugt 1 bis 12 und ganz besonders bevorzugt 1 bis 7 C-Atomen handeln. Die bevorzugten Substituenten sind Methyl, Ethyl, Methoxy, Ethoxy, Fluor, Chlor und Brom. Die Arene und Heteroarene sind bevorzugt mit ein oder zwei Resten substituiert und besonders bevorzugt unsubstituiert. Unter den Heteroarenen sind die elektronenreichen Heteroarene bevorzugt. Die Arene und Heteroarene können sowohl $\pi$- als auch $\sigma$-gebunden sein; im letzten Fall handelt es sich dann um die entsprechenden Aryl- und Heteroarylreste. Das Aryl enthält bevorzugt 6 bis 18, besonders bevorzugt 6 bis 12 C-Atome. Das Heteroaryl enthält bevorzugt 4 bis 16 C-Atome.

Einige Beispiele für Arene und Heteroarene sind Benzol, p-Cumen, Biphenyl, Naphthalin, Anthracen, Acenaphthen, Fluoren, Phenanthren, Pyren, Chrysen, Fluoranthren, Furan, Thiophen, Pyrrol, Pyridin, $\gamma$-Pyran, $\gamma$-Thiopyran, Pyrimidin, Pyrazin, Indol, Cumaron, Thionaphthen, Carbazol, Dibenzofuran, Dibenzothiophen, Pyrazol, Imidazol, Benzimidazol, Oxazol, Thiazol, Isoxazol, Isothiazol, Chinolin, Isochinolin, Acridin, Chromen, Phenazin, Phenoxazin, Phenothiazin, Triazine, Thianthren und Purin. Bevorzugte Arene und Heteroarene sind Benzol, Naphthalin, Thiophen und Benzthiophen. Ganz besonders bevorzugt ist das Aren Benzol und das Heteroaren Thiophen.

Die Nitrile können zum Beispiel mit Methoxy, Ethoxy, Fluor oder Chlor substituiert sein; bevorzugt sind die Nitrile unsubstituiert. Die Alkylnitrile sind bevorzugt linear. Einige Beispiele für Nitrile sind Acetonitril, Propionitril, Butyronitril, Pentylnitril, Hexylnitril, Cyclopentyl- und Cyclohexylnitril, Benzonitril, Methylbenzonitril, Benzylnitril und Naphthylnitril. Bei den Nitrilen handelt es sich bevorzugt um lineare $C_1$-$C_4$-Alkylnitrile oder Benzonitril. Von den Alkylnitrilen ist Acetonitril besonders bevorzugt.

In einer bevorzugten Untergruppe handelt es sich bei den photolabilen Liganden um $N_2$, unsubstituiertes oder mit ein bis drei $C_1$-$C_4$-Alkyl substituiertes Benzol, Thiophen, Benzonitril oder Acetonitril.

Nicht-photolabiler Ligand (auch als stark koordinierender Ligand bezeichnet) bedeutet im Rahmen der vorliegenden Erfindung, daß der Ligand bei Bestrahlung des Katalysators im sichtbaren oder nahen ultravioletten Spektralbereich nicht oder nur in unwesentlichem Ausmaß vom Katalysator dissoziiert.

Bei den nicht-photolabilen Liganden kann es sich zum Beispiel um die Heteroatome O, S oder N enthaltende und solvatisierende anorganische und organische Verbindungen, die häufig auch als Lösungsmittel verwendet werden, oder um unsubstituiertes oder mit $C_1$-$C_4$-Alkyl, $C_1$-$C_4$-Alkoxy, $(C_1$-$C_4$-Alkyl$)_3$Si oder $(C_1$-$C_4$-Alkyl$)_3$SiO- substituiertes Cyclopentadienyl oder Indenyl handeln. Beispiele für solche Verbindungen sind $H_2O$, $H_2S$, $NH_3$; gegebenenfalls halogenierte, besonders fluorierte oder chlorierte aliphatische oder cycloaliphatische Alkohole oder Merkaptane mit 1 bis 18, bevorzugt 1 bis 12 und besonders bevorzugt 1 bis 6 C-Atomen, aromatische Alkohole oder Thiole mit 6 bis 18, bevorzugt 6 bis 12 C-Atomen, araliphatische Alkohole oder Thiole mit 7 bis 18, bevorzugt 7 bis 12 C-Atomen; aliphatische, cycloaliphatische, araliphatische oder aromatische Ether, Thioether, Sulfoxide, Sulfone, Ketone, Aldehyde, Carbonsäureester, Lactone, gegebenenfalls N-$C_1$-$C_4$-mono- oder -dialkylierte Carbonsäureamide mit 2 bis 20, bevorzugt

2 bis 12 und besonders 2 bis 6 C-Atomen, und gegebenenfalls N-$C_1$-$C_4$-alkylierte Lactame; aliphatische, cycloaliphatische, araliphatische oder aromatische, primäre, sekundäre und tertiäre Amine mit 1 bis 20, bevorzugt 1 bis 12 und besonders bevorzugt 1 bis 6 C-Atomen; und gegebenenfalls Cyclopentadienyle wie zum Beispiel Cyclopentadienyl, Indenyl, ein- oder mehrfach methylierte oder trimethylsilylierte Cyclopentadienyle oder Indenyle.

Beispiele für solche nicht-photolabilen Liganden sind Methanol, Ethanol, n- und i-Propanol, n-, i- und t-Butanol, 1,1,1-Trifluorethanol, Bistrifluormethylmethanol, Tristrifluormethylmethanol, Pentanol, Hexanol, Methyl- oder Ethylmerkaptan, Cyclopentanol, Cyclohexanol, Cyclohexylmerkaptan, Phenol, Methylphenol, Fluorphenol, Phenylmerkaptan, Benzylmerkaptan, Benzylalkohol, Diethylether, Dimethylether, Diisopropylether, Di-n- oder Di-t-butylether, Tetrahydrofuran, Tetrahydropyran, Dioxan, Diethylthioether, Tetrahydrothiophen, Dimethylsulfoxid, Diethylsulfoxid, Tetra- und Pentamethylensulfoxid, Dimethylsulfon, Diethylsulfon, Tetra- und Pentamethylensulfon, Aceton, Methylethylketon, Diethylketon, Phenylmethylketon, Methylisobutylketon, Benzylmethylketon, Acetaldehyd, Propionaldehyd, Trifluoracetaldehyd, Benzaldehyd, Essigsäureethylester, Butyrolacton, Dimethylformamid, Dimethylacetamid, Pyrrolidon und N-Methylpyrrolidon, Indenyl, Cyclopentadienyl, Methyl- oder Dimethyl- oder Pentamethylcyclopentadienyl und Trimethylsilylcyclopentadienyl.

Die primären Amine können der Formel $R_{25}NH_2$, die sekundären Amine der Formel $R_{25}R_{26}NH$ und die tertiären Amine der Formel $R_{25}R_{26}R_{27}N$ entsprechen, worin $R_{25}$ $C_1$-$C_{18}$-Alkyl, unsubstituiertes oder mit $C_1$-$C_4$-Alkyl oder $C_1$-$C_4$-Alkoxy substituiertes $C_5$-oder $C_6$-Cycloalkyl, oder unsubstituiertes oder mit $C_1$-$C_4$-Alkyl oder $C_1$-$C_4$-Alkoxy substituiertes $C_6$-$C_{18}$-Aryl oder $C_7$-$C_{12}$-Aralkyl darstellt, $R_{26}$ unabhängig die Bedeutung von $R_{25}$ hat oder $R_{25}$ und $R_{23}$ gemeinsam Tetramethylen, Pentamethylen, 3-Oxa-1,5-pentylen oder $-CH_2-CH_2-NH-CH_2-CH_2-$ oder $-CH_2-CH_2-N(C_1-C_4\text{-Alkyl})-CH_2-CH_2-$ bedeuten, $R_{25}$ und $R_{26}$ unabhängig voneinander die zuvor angegebenen Bedeutungen haben und $R_{27}$ unabhängig die Bedeutung von $R_{25}$ hat. Das Alkyl enthält bevorzugt 1 bis 12 und besonders bevorzugt 1 bis 6 C-Atome. Das Aryl enthält bevorzugt 6 bis 12 C-Atome und das Aralkyl enthält bevorzugt 7 bis 9 C-Atome. Beispiele für Amine sind Methyl-, Dimethyl-, Trimethyl-, Ethyl-, Diethyl-, Triethyl-, Methyl-ethyl-, Dimethyl-ethyl, n-Propyl-, Di-n-propyl-, Tri-n-butyl-, Cyclohexyl-, Phenyl- und Benzylamin, sowie Pyrrolidin, N-Methylpyrrolidin, Piperidin, Piperazin, Morpholin und N-Methylmorpholin.

In einer bevorzugten Untergruppe handelt es sich bei den nicht-photolabilen Liganden um $H_2O$, $NH_3$ und unsubstituierte oder teilweise oder vollständig fluorierte $C_1$-$C_4$-Alkanole. Ganz besonders bevorzugt sind $H_2O$, $NH_3$, Cyclopentadienyl, Methanol und Ethanol.

Bei den erfindungsgemäss zu verwendenden Ruthenium- und Osmiumverbindungen kann es sich um ein- oder mehrkernige, zum Beispiel solche mit zwei oder drei Metallzentren handeln. Die Metallatome können hierbei über eine Brückengruppe oder Metall-Metall-Bindungen verbunden sein. Unter den Verbindungen mit mehr Metallzentren sind solche der Formel VIII bevorzugt

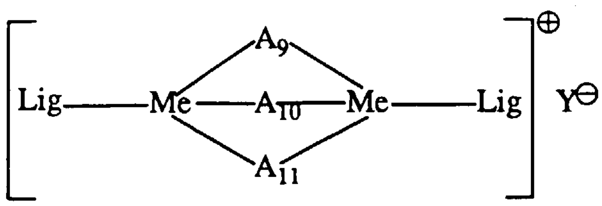

(VIII),

worin Lig für einen photolabilen Liganden und Me für Ru oder Os stehen, $A_9$, $A_{10}$ und $A_{11}$ eine bivalente Brückengruppe darstellen, und $Y^{\ominus}$ ein einwertiges nicht koordinierendes Anion bedeutet. Die Brückengruppe ist bevorzugt ionisch und besonders bevorzugt ein Halogenid, ganz besonders bevorzugt Chlorid, Bromid oder Iodid. Bei dem photolabilen Liganden handelt es sich bevorzugt um gleiche oder verschiedene Arene und $Y^{\ominus}$ kann die nachfolgend aufgeführten Anionen und ganz besonders Chlorid, Bromid oder Iodid darstellen. Ein Beispiel für solche Komplexe ist $[C_6H_6Ru(Cl)_3RuC_6H_6]Cl$.

Bevorzugte erfindungsgemäße Katalysatoren entsprechen der Formel IX

$$[(Me^{+n})(L_1^{z1})_m(L_2^{z2})_o(L_3^{z3})_p(L_4^{z4})_q(L_5^{z5})_r(L_6^{z6})_s](L_7^{z7})_t \qquad \text{(IX)}$$

worin

| | |
|---|---|
| Me | Ruthenium oder Osmium; |
| n | 0, 1, 2, 3, 4, 5, 6, 7 oder 8; |

| | |
|---|---|
| $L_1$ | einen photolabilen Liganden; |
| $L_2$, $L_3$, $L_4$, $L_5$ und $L_6$ | unabhängig voneinander einen nicht-photolabilen oder einen photolabilen Liganden; |
| m | 1, 2, 3, 4, 5, oder 6; |
| o, p, q, r, und s | unabhängig voneinander 0, 1, 2, 3, 4 oder 5; |
| $z_1$, $z_2$, $z_3$, $z_4$, $z_5$, $z_6$ und $z_7$ | unabhängig voneinander -4, -3, -2, -1, 0, +1 oder +2; und |
| $L_7$ | ein nicht koordinierendes Kation oder Anion bedeuten; |

wobei die Summe von m + o + p + q + r + s eine ganze Zahl von 2 bis 6 und t den Quotienten aus $(n + m \bullet z_1 + o \bullet z_2 + p \bullet z_3 + q \bullet z_4 + r \bullet z_5 + s \bullet z_6)/z_7$ bedeutet.

In der Formel IX steht $L_7$ bevorzugt für Halogen (zum Beispiel Cl, Br und I), das Anion einer Sauerstoffsäure, $BF_4$, $PF_6$, $SiF_6$ oder $AsF_6$.

Bei den Anionen von Sauerstoffsäuren kann es sich zum Beispiel um Sulfat, Phosphat, Perchlorat, Perbromat, Periodat, Antimonat, Arsenat, Nitrat, Carbonat, das Anion einer $C_1$-$C_8$-Carbonsäure wie zum Beispiel Formiat, Acetat, Propionat, Butyrat, Benzoat, Phenylacetat, Mono-, Di- oder Trichlor- oder -fluoracetat, Sulfonate wie zum Beispiel Methylsulfonat, Ethylsulfonat, Propylsulfonat, Butylsulfonat, Trifluormethylsulfonat (Triflat), gegebenenfalls mit $C_1$-$C_4$-Alkyl, $C_1$-$C_4$-Alkoxy oder Halogen, besonders Fluor, Chlor oder Brom substituiertes Phenylsulfonat oder Benzylsulfonat, wie zum Beispiel Tosylat, Mesylat, Brosylat, p-Methoxy- oder p-Ethoxyphenylsulfonat, Pentafluorphenylsulfonat oder 2,4,6-Triisopropylsulfonat, und Phosphonate wie zum Beispiel Methylphosphonat, Ethylphosphonat, Propylphosphonat, Butylphosphonat, Phenylphosphonat, p-Methylphenylphosphonat oder Benzylphosphonat handeln.

Vorzugsweise steht in der Formel IX Me für Ruthenium, besonders für $Ru^{2+}$.

Eine besonders hervorzuhebende Gruppe von Verbindungen der Formel IX ist jene, worin die Liganden $L_1$, $L_2$, $L_3$, $L_4$, $L_5$ und $L_6$ unabhängig voneinander unsubstituierte oder mit $C_1$-$C_4$-Akyl, $C_1$-$C_4$-Alkoxy oder Halogen substituierte aliphatische, cycloaliphatische, aromatische oder araliphatische Nitrile mit 1 bis 22 C-Atomen oder $C_6$-$C_{18}$-Aryl bedeuten; oder $L_1$, $L_2$ und $L_3$ gemeinsam unsubstituierte oder mit -OH, $C_1$-$C_4$-Alkyl, $C_1$-$C_4$-Alkoxy, $C_6$-$C_{12}$-Aryl oder Halogen substituierte monocyclische, polycyclische oder kondensierte Arene mit 6 bis 24, bevorzugt 6 bis 18 und besonders bevorzugt 6 bis 12 C-Atomen oder unsubstituierte oder mit -OH, $C_1$-$C_4$-Akyl, $C_1$-$C_4$-Alkoxy oder Halogen substituierte monocyclische Heteroarene, kondensierte Heteroarene oder kondensierte Aren-Heteroarene mit 4 bis 22 C-Atomen und 1 bis 3 Heteroatomen ausgewählt aus der Gruppe O, S und N darstellen, und $L_4$, $L_5$ und $L_6$ gemeinsam die gleiche Bedeutung haben, oder einzeln unabhängig voneinander $N_2$ oder besagtes Nitril oder besagtes $C_6$-$C_{18}$-Aryl darstellen.

Eine bevorzugte Untergruppe der obigen Verbindungen der Formel IX sind solche, worin die Liganden $L_1$, $L_2$, $L_3$, $L_4$, $L_5$ und $L_6$ unabhängig voneinander $N_2$, $C_1$-$C_{20}$-Alkylnitril, $C_6$-$C_{12}$-Arylnitril, $C_7$-$C_{12}$-Aralkylnitril, $C_6$-$C_{12}$-Aryl oder $L_1$, $L_2$ und $L_3$ je gemeinsam die Gruppen $A_1$ oder $A_2$ bedeuten

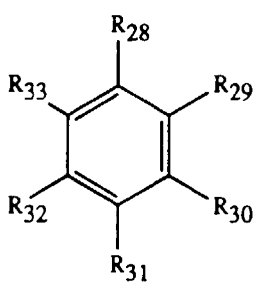

$(A_1)$,

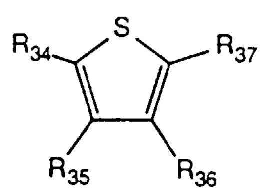

$(A_2)$;

worin

$R_{28}$, $R_{29}$, $R_{30}$, $R_{31}$, $R_{32}$, $R_{33}$, $R_{34}$, $R_{35}$, $R_{36}$ und $R_{37}$ unabhängig voneinander Wasserstoff, $C_1$-$C_{20}$-Alkyl, $C_1$-$C_{20}$-Alkoxy, Aryl oder $SiR_{38}R_{39}R_{40}$, wobei bei den Gruppen $A_1$ und $A_2$ an benachbarten Kohlenstoffatomen ein aromatischer oder heteroaromatischer Ring, dessen Heteroatome aus Sauerstoff, Schwefel und Stickstoff ausgewählt sind, ankondensiert sein kann, bedeuten; und $R_{38}$, $R_{39}$ und $R_{40}$ unabhängig voneinander $C_1$-$C_{12}$-Alkyl, $C_5$- oder $C_6$-Cycloalkyl, oder unsubstituiertes oder mit $C_1$-$C_6$-Alkyl oder $C_1$-$C_6$-Alkoxy substituiertes Phenyl oder Benzyl, vorzugsweise $C_1$-$C_8$-Alkyl, Phenyl oder Benzyl, besonders bevorzugt $C_1$-$C_4$-Alkyl, Phenyl oder Benzyl bedeuten, und

$L_4$, $L_5$ und $L_6$ ebenfalls gemeinsam diese Bedeutung haben oder je einzeln $N_2$, besagte Nitrile oder besagtes $C_6$-$C_{12}$-Aryl darstellen, oder ein Aren oder Heteroaren bedeuten.

Aus dieser hervorzuhebenden Gruppe von Verbindungen der Formel IX sind diejenigen bevorzugt, worin

$L_1$, $L_2$, $L_3$, $L_4$, $L_5$ und $L_6$ unabhängig voneinander $C_1$-$C_{12}$-Alkylnitril, $C_6$-$C_{12}$-Arylnitril oder $L_1$, $L_2$ und $L_3$ je gemeinsam die Gruppen $A_1$ oder $A_2$ bedeuten und $L_4$, $L_5$ und $L_6$ ebenfalls gemeinsam diese Bedeutung haben oder je einzeln $N_2$, besagte Nitrile oder besagtes Aren oder Heteroaren der Formeln $A_1$ und $A_2$ darstellen, worin $R_{28}$, $R_{29}$, $R_{30}$, $R_{31}$, $R_{32}$, $R_{33}$, $R_{34}$, $R_{35}$, $R_{36}$ und $R_{37}$ unabhängig voneinander für Wasserstoff, $C_1$-$C_6$-Alkyl, $C_1$-$C_6$-Alkoxy, $SiR_{38}R_{39}R_{40}$ oder Phenyl stehen, wobei bei den Gruppen $A_1$ und $A_2$ an benachbarten Kohlenstoffatomen ein Benzolring ankondensiert sein kann, und $R_{38}$, $R_{39}$ und $R_{40}$ Methyl, Ethyl oder Phenyl sind.

In einer ganz besonders bevorzugten Ausführungsform des erfindungsgemäßen Verfahrens entspricht der verwendete Katalysator der Formel IX, worin

$L_1$, $L_2$, $L_3$, $L_4$, $L_5$ und $L_6$ unabhängig voneinander Methylnitril, Ethylnitril oder Phenylnitril, oder $L_1$, $L_2$ und $L_3$ je gemeinsam die Gruppen $A_1$ oder $A_2$ bedeuten und $L_4$, $L_5$ und $L_6$ ebenfalls gemeinsam diese Bedeutung haben oder je einzeln besagte Nitrile darstellen, worin $R_{28}$, $R_{29}$, $R_{30}$, $R_{31}$, $R_{32}$, $R_{33}$, $R_{34}$, $R_{35}$, $R_{36}$ und $R_{37}$ unabhängig voneinander für Wasserstoff, Methyl, Methoxy oder Phenyl stehen, wobei bei den Gruppen $A_1$ und $A_2$ an benachbarten Kohlenstoffatomen ein Benzolring ankondensiert sein kann.

Eine andere besonders bevorzugte Untergruppe der Verbindungen der Formel IX sind solche, worin $L_1$, $L_2$ und $L_3$ gemeinsam unsubstituierte oder mit $C_1$-$C_4$-Akyl, $C_1$-$C_4$-Alkoxy, $C_6$-$C_{12}$-Aryl oder Halogen substituierte monocyclische, polycyclische oder kondensierte Arene mit 6 bis 24, bevorzugt 6 bis 18 und besonders bevorzugt 6 bis 12 C-Atomen oder unsubstituierte oder mit $C_1$-$C_4$-Akyl, $C_1$-$C_4$-Alkoxy oder Halogen substituierte monocyclische Heteroarene, kondensierte Heteroarene oder kondensierte Aren-Heteroarene mit 4 bis 22, bevorzugt 4 bis 16 und besonders 4 bis 10 C-Atomen und 1 bis 3 Heteroatomen ausgewählt aus der Gruppe O, S und N darstellen, und $L_4$, $L_5$ und $L_6$ einen nicht-photolabilen Liganden bedeuten, wobei die vorherigen Bevorzugungen auch hier gelten.

$L_1$, $L_2$ und $L_3$ stellen in dieser Untergruppe bevorzugt Benzol oder Naphthalin dar, und der nicht-photolabile Ligand bedeutet bevorzugt $H_2O$, $NH_3$, unsubstituiertes oder mit Fluor substituiertes $C_1$-$C_4$-Alkanol oder -Alkanthiol, aliphatische Ether, Thioether, Sulfoxide oder Sulfone mit 2 bis 8 C-Atomen, Dimethylformamid oder N-Methylpyrrolidon.

In einer weiteren bevorzugten Untergruppe von Verbindungen der Formel X handelt es sich um Ruthenium- und Osmiumverbindungen der Formel X

$$[L_1Me(L_8)_5]^{2\oplus}[Y_1^{x\ominus}]_{2/x} \quad (X),$$

worin $L_1$ ein photolabiler Ligand und $L_8$ ein nicht-photolabiler Ligand sind, Me Ru oder Os bedeutet, $Y_1$ ein nicht koordinierendes Anion darstellt und x für die Zahlen 1, 2 oder 3 steht. Für die photolabilen Liganden, nicht-photolabilen Liganden, Me und $Y_1$ gelten die vorher aufgeführten Bevorzugungen. Besonders bevorzugt ist $L_1$ $N_2$ oder ein Nitril wie zum Beispiel $C_1$-$C_4$-Alkylnitril (Acetonitril), Benzonitril oder Benzylnitril, $L_8$ $NH_3$ oder ein Amin mit 1 bis 12 C-Atomen, $Y_1$ ein nicht koordinierendes Anion und x die Zahl 1 oder 2.

Für die vorliegende Erfindung besonders geeignete Katalysatoren sind (tos bedeutet Tosylat und tis bedeutet 2,4,6-Triisopropylphenylsulfonat): $Ru(CH_3CN)_6(tos)_2$, $Ru(CH_3CH_2CN)_6(tos)_2$, $Ru(CH_3CN)_6(CF_3SO_3)_2$, $Ru(CH_3CH_2CN)_6(CF_3SO_3)_2$, $Ru(C_6H_6)_2(tos)_2$, $[Ru(C_6H_6)(C_6H_5OCH_3)](BF_4)_2$, $[Ru(C_6H_6)(C_6H_5$i-propyl$)](BF_4)_2$, $[Ru(C_6H_6)$(1,3,5-trimethylphenol)$](BF_4)_2$, $[Ru(C_6H_6)$(hexamethylbenzol)$](BF_4)_2$, $[Ru(C_6H_6)$(biphenyl)$](BF_4)_2$, $[Ru(C_6H_6)$(chrysen)$](BF_4)_2$, $[Ru(C_6H_6)$(naphtalin)$](BF_4)_2$, [Ru(cyclopentadienyl)(4-methylcumyl)]$PF_6$, [Ru(cyanophenyl)$_6$](tos)$_2$, [Ru(cyanophenyl)$_6$]$(CF_3SO_3)_2$, $[Ru(C_6H_6)$(tetramethylthiophen)$_3$](tos)$_2$, $[Ru(C_6H_6)(CH_3CN)_3](tos)_2$, $[Ru(C_6H_6)$(tetramethylthiophen)$_3](CF_3SO_3)_2$, $[Ru(C_6H_6)(CH_3CN)_3](CF_3SO_3)_2$, $[Ru(C_6H_6)(CH_3OH)_3](tos)_2$, $[Ru(C_6H_6)(CH_3OH)_3](tis)_2$, $[Os(NH_3)_5N_2](PF_6)_2$, $[Ru(NH_3)_5N_2](PF_6)_2$, $[Ru(NH_3)_5(CH_3CN)]BF_4$, $[Ru(C_6H_6(NH_3)_3](tis)_2$, $[Ru(C_6H_6$(Tetrahydrothiophen)$_3](CF_3SO_3)_2$, $[Ru((CH_3)_2S)_3C_6H_6](tos)_2$, [Ru(Dimethylsulfoxid)$_3C_6H_6](PF_6)_2$, [Ru(Dimethylformamid)$_3C_6H_6](PF_6)_2$, $[Ru(C_6H_6)Cl_2]_2$ und $[Os(C_6H_6)Cl_2]_2$.

Erfindungsgemäß zu verwendende Ruthenium- und Osmium-Katalysatoren sind entweder bekannt und teilweise im Handel erhältlich oder analog bekannter Verfahren herstellbar. Derartige Katalysatoren und ihre Herstellung werden beispielsweise in Gilkerson, W.R., Jackson, M.D., J. Am. Chem. Soc. 101:4096-411 (1979), Bennett, M.A., Matheson, T.W., J. Organomet. Chem. 175:87-93 (1979), Moorehouse, S., Wilkinson, G., J. Chem. Soc.; Dalton Trans., 2187-2190 (1974) und Luo, S., Rauchfuss, T.B., Wilson, S.R., J. Am. Chem. Soc. 114:8515-8520 (1992) beschrieben.

2. Thermisch oder mit Strahlung aktivierbare thermostabile Molybdän(VI)- oder Wolfram(VI)verbindungen, die mindestens zwei Methylgruppen oder zwei monosubstituierte Methylgruppen am Metall gebunden enthalten, wobei der Substituent kein Wasserstoffatom in $\alpha$-Stellung enthält.

Die übrigen Valenzen des Molybdän- und Wolframs sind bevorzugt mit thermostabilen Neutralliganden abgesättigt, die in grosser Vielzahl bekannt sind Die Zahl der Neutralliganden kann auch die stöchiometrisch mögliche Zahl überschreiten (Solvate). Thermostabilität ist vorn erläutert worden. Bei Temperaturen über 50°C, zum Beispiel 60 bis 300°C, werden diese Molybdän- und Wolframverbindungen auch thermisch aktiviert.

Bei den erfindungsgemäss zu verwendenden Molybdän- und Wolframverbindungen kann es sich um solche handeln, die ein Metallatom oder zwei über eine Einfach-, Doppel- oder Dreifachbindung verbundene Metallatome enthalten. Die am Metall gebundene Methylgruppe oder monosubstituierte Methylgruppe ist mindestens zweimal, besonders bevorzugt zwei- bis sechsmal und insbesondere bevorzugt zwei- bis viermal als Ligand gebunden. Dieser Ligand entspricht bevorzugt der Formel XI,

$$-CH_2-R \qquad (XI),$$

worin R H, $-CF_3$, $-SiR_{38}R_{39}R_{40}$, $-CR_{41}R_{42}R_{43}$, unsubstituiertes oder mit $C_1$-$C_6$-Alkyl oder $C_1$-$C_6$-Alkoxy substituiertes $C_6$-$C_{16}$-Aryl oder $C_4$-$C_{15}$-Heteroaryl mit 1 bis 3 Heteroatomen aus der Gruppe O, S und N darstellt; und

$R_{41}$, $R_{42}$ und $R_{43}$ unabhängig voneinander $C_1$-$C_{10}$- Alkyl bedeuten, das unsubstituiert oder mit $C_1$-$C_{10}$-Alkoxy substituiert ist, oder $R_{41}$ und $R_{42}$ diese Bedeutung haben und $R_{43}$ $C_6$-$C_{10}$-Aryl oder $C_4$-$C_9$-Heteroaryl ist, das unsubstituiert oder mit $C_1$-$C_6$-Alkyl oder $C_1$-$C_6$-Alkoxy substituiert ist; und
$R_{38}$, $R_{39}$ und $R_{40}$ die oben angegebenen Bedeutungen haben.

Bedeuten $R_{38}$ bis $R_{43}$ Alkyl, so kann dieses linear oder verzweigt sein und bevorzugt 1 bis 8 und besonders bevorzugt 1 bis 4 C-Atome enthalten. Bedeuten $R_{38}$ bis $R_{43}$ Aryl, so handelt es sich bevorzugt um Phenyl oder Naphthyl.

Bedeutet R in Formel XI Aryl, so handelt es sich bevorzugt um Phenyl oder Naphthyl. Bedeutet R in Formel XI Heteroaryl, so handelt es sich bevorzugt um Pyridinyl, Furanyl, Thiophenyl oder Pyrrolyl.

Bevorzugte Substituenten für $R_{38}$ bis $R_{43}$ sind im Rahmen der Definitionen Methyl, Ethyl, Methoxy und Ethoxy. Beispiele für die Reste $R_{38}$ bis $R_{43}$ sind zuvor unter den Verbindungen der Formel I angegeben worden.

In einer bevorzugten Ausführungsform bedeutet die Gruppe R in Formel XI H, $-C(CH_3)_3$, $-C(CH_3)_2C_6H_5$, unsubstituiertes oder mit Methyl, Ethyl, Methoxy oder Ethoxy substituiertes Phenyl, $-CF_3$ oder $-Si(CH_3)_3$.

Die übrigen Valenzen der Mo(VI)- und W(VI)-Atome sind gegebenenfalls mit gleichen oder verschiedenen Liganden aus der Gruppe =O, $=N-R_{44}$, sekundäre Amine mit 2 bis 18 C-Atomen, $R_{45}O$-, $R_{45}S$-, Halogen, gegebenenfalls substituiertes Cyclopentadienyl, überbrücktes Biscyclopentadienyl, tridentate monoanionische Liganden und Neutralliganden wie zum Beispiel Ethern, Nitrilen, CO und tertiären Phosphinen und Aminen abgesättigt, worin die $R_{45}$ unabhängig voneinander unsubstituiertes oder mit $C_1$-$C_6$-Alkoxy oder Halogen substituiertes lineares oder verzweigtes $C_1$-$C_{18}$-Alkyl, unsubstituiertes oder mit $C_1$-$C_6$-Alkyl, $C_1$-$C_6$-Alkoxy oder Halogen substituiertes $C_5$- oder $C_6$-Cycloalkyl, unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl oder Halogen substituiertes Phenyl, oder unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl oder Halogen substituiertes Benzyl oder Phenylethyl darstellen; und $R_{44}$ unsubstituiertes oder mit $C_1$-$C_6$-Alkoxy substituiertes lineares oder verzweigtes $C_1$-$C_{18}$-Alkyl, unsubstituiertes oder mit $C_1$-$C_6$-Alkyl, $C_1$-$C_6$-Alkoxy oder Halogen substituiertes $C_5$- oder $C_6$-Cycloalkyl, unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl, Di($C_1$-$C_6$-alkyl)amino, Di($C_1$-$C_6$-alkyl)amino-$C_1$-$C_3$-alkyl, oder Halogen substituiertes Phenyl, oder unsubstituiertes oder mit $C_1$-$C_6$-Alkyl, $C_1$-$C_6$-Alkoxy, $C_1$-$C_6$-Alkoxymethyl-, $C_1$-$C_6$-Alkoxyethyl- oder Halogen substituiertes Benzyl oder Phenylethyl bedeutet.

Sekundäre Amine sind bevorzugt solche der Formel $R_{46}R_{47}N$-, worin $R_{46}$ und $R_{47}$ unabhängig voneinander lineares oder verzweigtes $C_1$-$C_{18}$-Alkyl; $C_5$- oder $C_6$-Cycloalkyl; unsubstituiertes oder mit $C_1$-$C_6$-Alkoxy, oder Halogen substituiertes Benzyl oder Phenylethyl oder $(C_1$-$C_6$-alkyl$)_3$Si; oder $R_{46}$ und $R_{47}$ zusammen Tetramethylen, Pentamethylen oder 3-Oxapentan-1,5-diyl bedeuten. Das Alkyl enthält bevorzugt 1 bis 12 und besonders bevorzugt 1 bis 6 C-Atome. Einige Beispiele sind Dimethyl-, Diethyl-, Di-n-propyl-, Di-i-propyl-, Di-n-butyl-, Methyl-ethyl-, Dibenzyl-, Benzylmethyl-, Diphenyl-, Phenylmethylamino und Di(trimethylsilyl)amino.

Halogen als Ligand oder Substituent ist bevorzugt F oder Cl und besonders bevorzugt Cl.

Das Cyclopentadienyl kann unsubstituiert oder mit ein bis fünf $C_1$-$C_4$-Akyl, besonders Methyl, oder -Si($C_1$-$C_4$-Alkyl), besonders $-Si(CH_3)_3$ substituiert sein. Überbrückte Cyclopentadienyle sind besonders solche der Formel $R_{48}$-A-$R_{48}$, worin $R_{48}$ unsubstituiertes oder mit ein bis fünf $C_1$-$C_4$-Alkyl, besonders Methyl, oder -Si($C_1$-$C_4$-Alkyl), besonders $-Si(CH_3)_3$ substituiertes Cyclopentadienyl darstellt und A für $-CH_2$-, $-CH_2-CH_2$-, $-Si(CH_3)_2$-, $-Si(CH_3)_2$-Si

$(CH_3)_2$- oder $-Si(CH_3)_2-O-Si(CH_3)_2$- steht.

Bei Ethern als Neutralliganden kann es sich um Dialkylether mit 2 bis 8 C-Atomen oder cyclische Ether mit 5 oder 6 Ringgliedern handeln. Einige Beispiele sind Diethylether, Methylethylether, Diethylether, Di-n-propylether, Di-i-propylether, Di-n-butylether, Ethylenglykoldimethylether, Tetrahydroforan und Dioxan.

Bei Nitrilen als Neutralliganden kann es sich um aliphatische oder aromatische Nitrile mit 1 bis 12, vorzugsweise 1 bis 8 C-Atomen handeln. Einige Beispiele sind Acetonitril, Propionitril, Butylnitril, Benzonitril und Benzylnitril.

Bei tertiären Aminen und Phosphinen als Neutralliganden kann es sich um solche mit 3 bis 24, vorzugsweise 3 bis 18 C-Atomen handeln. Einige Beispiele sind Trimethylamin und -phosphin, Triethylamin und -phosphin, Tri-n-propylamin und -phosphin, Tri-n-butylamin und -phosphin, Triphenylamin und -phosphin, Tricyclohexylamin und -phosphin, Phenyldimethylamin und -phosphin, Benzyldimethylamin und -phosphin, 3,5-Dimethylphenyl-dimethylamin und -phosphin.

Bei den tridentaten monoanionischen Liganden kann es sich zum Beispiel um Hydro(tris-pyrazol-1-yl)borate oder Alkyl(trispyrazol-1-yl)borate, die unsubstituiert oder mit ein bis drei $C_1$-$C_4$-Alkyl substituiert sind [siehe Trofimenko, S., Chem. Rev., 93:943-980 (1993)], oder um $[C_5(R'_5)Co(R_{50}R_{51}P{=}O)_3]^{\ominus}$, worin R' H oder Methyl und $R_{50}$ sowie $R_{51}$ unabhängig voneinander $C_1$-$C_4$-Alkyl, $C_1$-$C_4$-Alkoxy oder Phenyl bedeuten [siehe Kläui, W., Angew. Chem. 102: 661-670 (1990)], handeln.

Bei Halogen als Substituent für die Reste $R_{44}$ und $R_{45}$ handelt es sich bevorzugt um Fluor und besonders bevorzugt um Chlor. Die Substituenten Alkyl, Alkoxy oder Alkoxy im Alkoxymethyl oder -ethyl enthalten bevorzugt 1 bis 4 und besonders 1 oder 2 C-Atome. Beispiele sind Methyl, Ethyl, n- und i-Propyl, n-, i- und t-Butyl, Methoxy, Ethoxy, n- und i-Propyloxy und n-, i- und t-Butyloxy.

$R_{44}$ und $R_{45}$ enthalten als Alkyl bevorzugt 1 bis 12, besonders bevorzugt 1 bis 8, und insbesondere bevorzugt 1 bis 4 C-Atome. Bevorzugt handelt es sich um verzweigtes Alkyl. Einige Beispiele für R44 sind Methoxy, Ethoxy, n- und i-Propyloxy, n-, i- und t-Butyloxy, Hexafluor-i-propyloxy und Hexa- sowie Perfluorbutyloxy.

Einige Beispiele für substituiertes Phenyl und Benzyl für $R_{44}$ und $R_{45}$ sind p-Methylphenyl oder Benzyl, p-Fluor- oder p-Chlorphenyl oder -benzyl, p-Ethylphenyl oder -benzyl, p-n- oder i-Propylphenyl oder -benzyl, p-i-Butylphenyl oder -benzyl, 3-Methylphenyl oder -benzyl, 3-i-Propylphenyl oder -benzyl, 2,6-Dimethylphenyl oder -benzyl, 2,6-Di-i-propylphenyl oder -benzyl, 2,6-Di-n- oder -t-butylphenyl und -benzyl. $R_{45}$ steht besonders bevorzugt für unsubstituiertes oder mit $C_1$-$C_4$-Alkyl oder $C_1$-$C_4$-Alkoxy substituiertes Phenyl.

In einer bevorzugten Ausführungsform entsprechen die Molybdän- und Wolframverbindungen besonders einer der Formeln XII bis XIIc

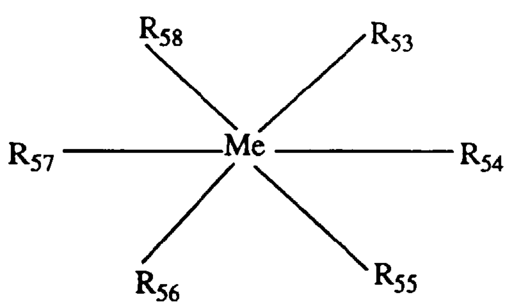

(XII),

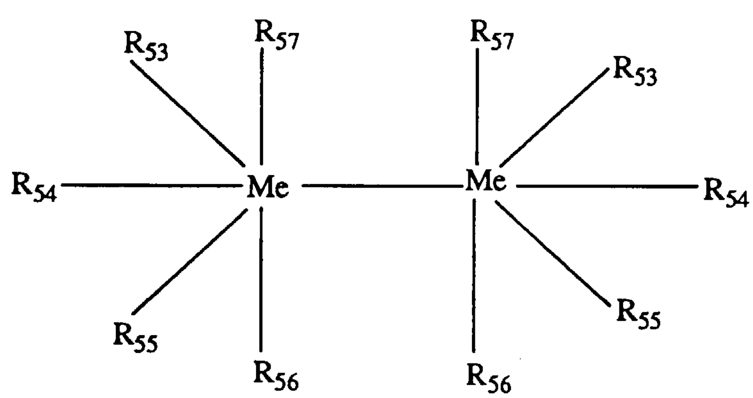

(XIIa),

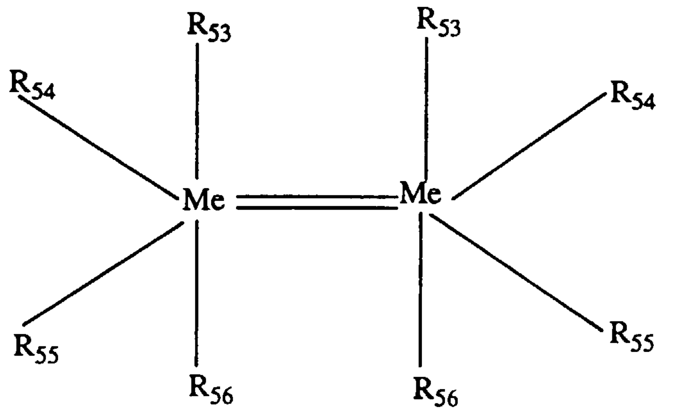

(XIIb),

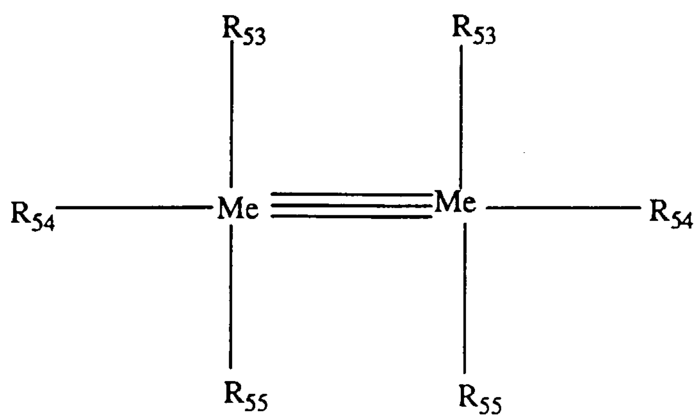

(XIIc),

worin

Me für Mo(VI) oder W(VI) steht;
mindestens zwei, bevorzugt 2 bis 4, der Reste $R_{53}$ bis $R_{58}$ einen Rest $-CH_2-R$ der Formel XI bedeuten, worin R die oben angegebenen Bedeutungen hat;
je zwei der übrigen Reste von $R_{53}$ bis $R_{58}$ =0 oder $=N-R_{44}$ bedeuten, und $R_{44}$ die oben angegebenen Bedeutungen hat; und/oder
die übrigen Reste von $R_{53}$ bis $R_{58}$ Sekundäramino mit 2 bis 18 C-Atomen, $R_{45}O-$ oder $R_{45}S-$, Halogen, Cyclopentadienyl oder überbrücktes Biscyclopentadienyl oder einen Neutralliganden bedeuten, worin $R_{45}$ die oben angegebenen Bedeutungen hat. Für die Reste R und $R_{38}$ bis $R_{45}$ gelten die zuvor angegebenen Bevorzugungen.

In einer besonders bevorzugten Ausführungsform werden in der erfindungsgemässen Zusammensetzung Molybdän- oder Wolframverbindungen der Formel XII verwendet, worin

a) $R_{53}$ bis $R_{58}$ einen Rest der Formel XI $-CH_2-R$ bedeuten, oder
b) $R_{53}$ und $R_{54}$ einen Rest der Formel XI $-CH_2-R$ darstellen, $R_{55}$ und $R_{56}$ zusammen den Rest $=N-R_{44}$ bedeuten, und $R_{57}$ und $R_{58}$ zusammen unabhängig voneinander $R_{45}-O-$ oder Halogen darstellen, oder
c) $R_{53}$ und $R_{54}$ zusammen und $R_{55}$ und $R_{56}$ zusammen den Rest $=N-R_{44}$ bedeuten, und $R_{57}$ und $R_{58}$ einen Rest der Formel XI $-CH_2-R$ darstellen,

wobei R, $R_{44}$ und $R_{45}$ die voranstehenden Bedeutungen haben. Für R, $R_{44}$ und $R_{45}$ gelten die voranstehenden Bevorzugungen.
Unter den Verbindungen der Formel XIIc sind besonders jene bevorzugt, worin $R_{53}$, $R_{54}$ und $R_{55}$ einen Rest der Formel XI darstellen, wobei es sich bei dem Rest der Formel XI besonders bevorzugt um $-CH_2-Si(C_1-C_4-Alkyl)_3$

handelt.

Ganz besonders bevorzugt werden in der erfindungsgemässen Zusammensetzung Molybdän- oder Wolframverbindungen der Formeln XIII, XIIIa oder XIIIb verwendet,

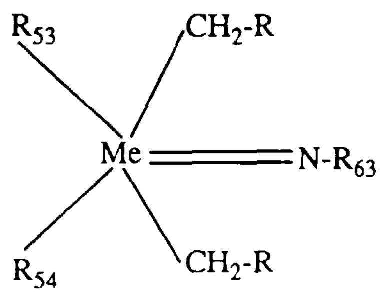

(XIII),

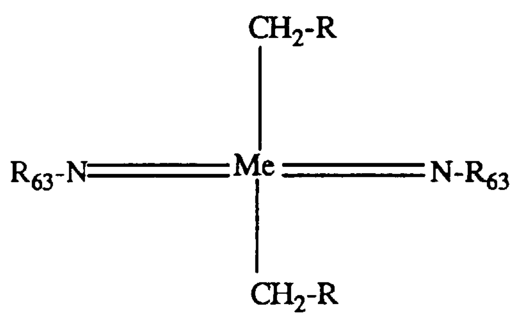

(XIIIa),

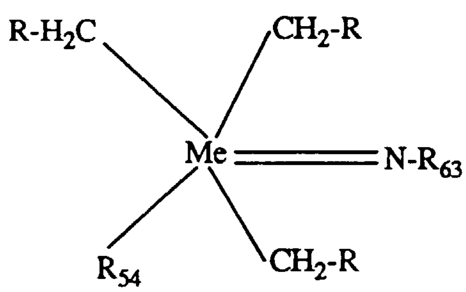

(XIIIb),

worin

Me für Mo(VI) oder W(VI) steht,
RH, $-C(CH_3)_3$, $-C(CH_3)_2-C_6H_5$, $-C_6H_5$ oder $-Si(C_1-C_4\text{-Alkyl})_3$ darstellt,
$R_{23}$ Phenyl oder mit 1 bis 3 $C_1$-$C_4$-Alkyl oder $C_1$-$C_4$-Alkoxy substituiertes Phenyl bedeutet,
$R_{53}$ unsubstituiertes oder mit Fluor substituiertes lineares oder verzweigtes $C_1$-$C_4$-Alkoxy darstellt, und
$R_{54}$ die gleiche Bedeutung wie $R_{53}$ hat oder für F, Cl oder Br steht. $R_{53}$ stellt besonders bevorzugt verzweigtes Alkoxy dar, das gegebenenfalls mit F teilweise oder vollständig substituiert ist, zum Beispiel i-Propyloxy, i- und t-Butyloxy, Hexafluorpopyloxy und Nonafluorpropyloxy. Bei $R_{54}$ handelt es sich bevorzugt um Cl.

Einige Beispiele für Molybdän- und Wolframverbindungen sind:

$W(=N-C_6H_5)(OC(CH_3)_3)(Cl)[(CH_2Si(CH_3)_3)]_2$, $[(CH_3)_3SiCH_2]_3Mo{\equiv}Mo[CH_2Si(CH_3)_3]_3$,
$W(=N-C_6H_5)(OC(CF_3)_2CH_3)_2[(CH_2Si(CH_3)_3)]_2$, $W(=NC_6H_5)[CH_2Si(CH_3)_3]_3Cl$,
$Mo(=N\text{-}2,6\text{-dimethyl}C_6H_5)_2)[(CH_2-C_6H_5)]_2$, $W[2,6-(CH_3)_2C_6H_3N]_2(CH_2-C_6H_5)_2$,

$Mo(=N\text{-}2,6\text{-diisopropyl}C_6H_3)_2C(CH_2\text{-}C_6H_5)]_2$,
$Mo(=N\text{-}2,6\text{-diisopropyl}C_6H_3)_2[(CH_2C(CH_3)_2\text{-}C_6H_5)]_2$ und
$Mo(=N\text{-}2,6\text{-dimethyl}C_6H_3)_2(CH_3)_2$(Tetrahydrofuran).

Die erfindungsgemäss zu verwendenden Molybdän- und Wolfram-Katalysatoren sind bekannt oder nach bekannten und analogen Verfahren ausgehend von den Metallhalogeniden mittels Grignardreaktionen herstellbar [siehe zum Beispiel Huq, F., Mowat, W., Shortland, A., Skapski, A.C., Wilkinson, G., J. Chem. Soc., Chem. Commun. 1079-1080 (1971) oder Schrock, R.R., Murdzeck, J.S., Bazan, G.C., Robbins, J., DiMare, M., O'Regan, M., J. Am. Chem. Soc., 112:3875-3886 (1990)].

3. Thermostabile Titan(IV), Niob(V)-, Tantal(V)-, Mo lybdän(VI)- oder Wolfram(VI)verbindungen, in der eine Silylmethylgruppe und mindestens ein Halogen am Metall gebunden sind. Diese Einkomponentenkatalysatoren sind besonders photokatalytisch aktiv.

Bei den erfindungsgemäss zu verwendenden Titan(IV)-, Niob(V)- und Tantal(V)verbindungen handelt es sich um solche, die ein Metallatom enthalten. Bei den erfindungsgemäss zu verwendenden Molybdän(VI)- und Wolfram (VI)verbindungen kann es sich um solche handeln, die ein Metallatom oder zwei über eine Einfach-, Doppel- oder Dreifachbindung verbundene Metallatome enthalten. Die übrigen Valenzen des Titans, Niobs, Tantals, Molybdäns und Wolframs sind bevorzugt mit thermostabilen Neutralliganden abgesättigt, wobei die Definition der Thermostabilität eingangs gegeben wurde. Bei dem an das Metallatom gebundenen Halogen handelt es sich bevorzugt um F, Cl, Br und I, bevorzugter um F, Cl und Br und besonders bevorzugt um F oder Cl. Der Silylmethylligand entspricht bevorzugt der Formel XIV,

$$-CH_2\text{-}SiR_{38}R_{39}R_{40} \qquad (XIV),$$

worin

$R_{38}$, $R_{39}$ und $R_{40}$ unabhängig voneinander $C_1$-$C_{18}$-Alkyl, $C_5$- oder $C_6$-Cycloalkyl oder unsubstituiertes oder mit $C_1$-$C_6$-Alkyl oder $C_1$-$C_6$-Alkoxy substituiertes Phenyl oder Benzyl bedeuten.

Bedeuten $R_{38}$ bis $R_{40}$ Alkyl, so kann dieses linear oder verzweigt sein und bevorzugt 1 bis 12, besonders bevorzugt 1 bis 8 und insbesondere 1 bis 4 C-Atome enthalten. Besonders bevorzugtes Alkyl ist Methyl und Ethyl.

Bevorzugte Substituenten für $R_{38}$ bis $R_{40}$ als Phenyl und Benzyl sind im Rahmen der Definitionen Methyl, Ethyl, Methoxy und Ethoxy.

In einer bevorzugten Ausführungsform bedeuten in der Gruppe der Formel XIV $R_{38}$ bis $R_{40}$ $C_1$-$C_4$-Alkyl, Phenyl oder Benzyl.

Einige Beispiele für die Gruppe der Formel XIV sind $-CH_2\text{-}Si(CH_3)_3$, $-CH_2\text{-}Si(C_2H_5)_3$, $-CH_2\text{-}Si(n\text{-}C_3H_7)_3$, $-CH_2\text{-}Si(n\text{-}C_4H_9)_3$, $-CH_2\text{-}Si(CH_3)_2(n\text{-}C_4H_9)$, $-CH_2\text{-}Si(CH_3)_2(t\text{-}C_4H_9)$, $-CH_2\text{-}Si(CH_3)_2(C_2H_5)$, $-CH_2\text{-}Si(CH_3)_2[C(CH_3)_2CH(CH_3)_2]$, $-CH_2\text{-}Si(CH_3)_2(n\text{-}C_{12}H_{25})$, $-CH_2\text{-}Si(CH_3)_2(n\text{-}C_{18}H_{37})$, $-CH_2\text{-}Si(C_6H_5)_3$, $-CH_2\text{-}Si(CH_2\text{-}C_6H_5)_3$, $-CH_2\text{-}Si(C_6H_5)(CH_3)_2$ und $-CH_2\text{-}Si(CH_2\text{-}C_6H_5)(CH_3)_2$. Ganz besonders bevorzugt ist $-CH_2\text{-}Si(CH_3)_3$.

Die übrigen Valenzen der Ti(IV), Nb(V)-, Ta(V)-, Mo(VI)- und W(VI)-Atome sind gegebenenfalls mit gleichen oder verschiedenen neutralen Liganden abgesättigt, beispielsweise ausgewählt aus der Gruppe bestehend aus =O, $=N\text{-}R_{44}$, sekundären Aminen mit 2 bis 18 C-Atomen, $R_{45}O$-, $R_{45}S$-, Halogen, gegebenenfalls substituiertem Cyclopentadienyl, überbrücktem Biscyclopentadienyl, tridentaten monoanionischen Liganden und Neutralliganden wie zum Beispiel Ethern, Nitrilen, CO und tertiären Phosphinen und Aminen, worin die $R_{45}$ unabhängig voneinander unsubstituiertes oder mit $C_1$-$C_6$-Alkoxy oder Halogen substituiertes lineares oder verzweigtes $C_1$-$C_{18}$-Alkyl, unsubstituiertes oder mit $C_1$-$C_6$-Alkyl, $C_1$-$C_6$-Alkoxy oder Halogen substituiertes $C_5$- oder $C_6$-Cycloalkyl, unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl oder Halogen substituiertes Phenyl, oder unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl oder Halogen substituiertes Benzyl oder Phenylethyl darstellen; und $R_{44}$ unsubstituiertes oder mit $C_1$-$C_6$-Alkoxy substituiertes lineares oder verzweigtes $C_1$-$C_{18}$-Alkyl, unsubstituiertes oder mit $C_1$-$C_6$-Alkyl, $C_1$-$C_6$-Alkoxy oder Halogen substituiertes $C_5$- oder $C_6$-Cycloalkyl, unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl, Di($C_1$-$C_6$-alkyl)amino, Di($C_1$-$C_6$-alkyl)amino-$C_1$-$C_3$-alkyl, oder Halogen substituiertes Phenyl, oder unsubstituiertes oder mit $C_1$-$C_6$-Alkyl, $C_1$-$C_6$-Alkoxy, $C_1$-$C_6$-Alkoxymethyl-, $C_1$-$C_6$-Alkoxyethyl- oder Halogen substituiertes Benzyl oder Phenylethyl bedeutet, mit der Massgabe, dass im Fall der Titanverbindungen der Ligand nicht =O oder $=N\text{-}R_{44}$ ist.

Die Bedeutungen und Bevorzugungen von $R_{44}$ und $R_{45}$, von sekundären Aminen, von Halogen als weiterem Liganden an den Metallatomen oder als Substituent, von Cyclopentadienyl, Ethern, Nitrilen, tertiären Aminen und Phosphinen als Neutralliganden und von tridentaten monoanionischen Liganden sind zuvor angegeben worden. Ebenfalls

zuvor angegeben worden sind die Bedeutungen und Bevorzugungen von Alkyl, Alkoxy oder Alkoxy als Substituenten im Alkoxymethyl oder -ethyl.

In einer bevorzugten Ausführungsform entsprechen die Metallverbindungen besonders den Formeln XV, XVa oder XVb,

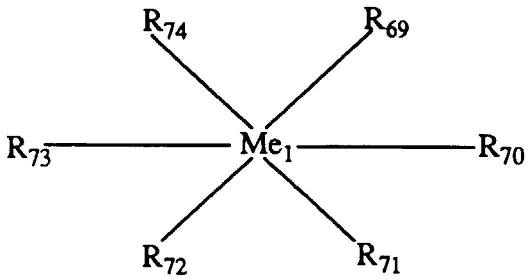

(XV),

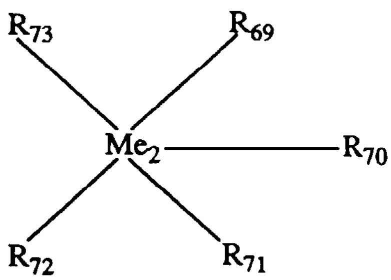

(XVa),

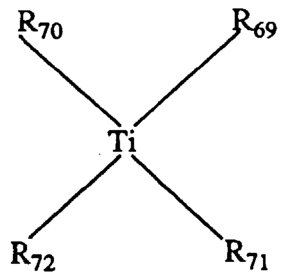

(XVb),

worin

$Me_1$ für Mo(VI) oder W(VI) steht;

$Me_2$ für Nb(V) oder Ta(V) steht;

einer der Reste $R_{69}$ bis $R_{74}$ einen Rest $-CH_2-SiR_{38}R_{39}R_{40}$ der Formel XIV bedeutet; wenigstens einer der Reste $R_{69}$ bis $R_{74}$ F, Cl oder Br darstellt;

$R_{38}$, $R_{39}$ und $R_{40}$ unabhängig voneinander $C_1$-$C_6$-Alkyl, $C_5$- oder $C_6$-Cycloalkyl, oder unsubstituiertes oder mit $C_1$-$C_6$-Alkyl oder $C_1$-$C_6$-Alkoxy substituiertes Phenyl oder Benzyl bedeuten;

in Formel XV zwei oder jeweils zwei und in Formel XVa zwei der übrigen Reste von $R_{69}$ bis $R_{74}$ je zusammen =O oder =N-$R_{44}$ bedeuten, und $R_{44}$ unsubstituiertes oder mit $C_1$-$C_6$-Alkoxy substituiertes lineares oder verzweigtes $C_1$-$C_{18}$-Alkyl, unsubstituiertes oder mit $C_1$-$C_6$-Alkyl, $C_1$-$C_6$-Alkoxy oder Halogen substituiertes $C_5$- oder $C_6$-Cycloalkyl, unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl, Di($C_1$-$C_6$-alkyl)amino, Di($C_1$-$C_6$-alkyl)amino-$C_1$-$C_3$-alkyl oder Halogen substituiertes Phenyl, oder unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl, Di($C_1$-$C_6$-alkyl)amino, Di($C_1$-$C_6$-alkyl)amino-$C_1$-$C_3$-alkyl oder Halogen substituiertes Benzyl oder Phenylethyl darstellt, und

die übrigen Reste Sekundäramino mit 2 bis 18 C-Atomen, $R_{45}$O- oder $R_{45}$S-, Halogen, unsubstituiertes oder substituiertes Cyclopentadienyl oder überbrücktes Biscyclopentadienyl oder einen Neutralliganden bedeuten, worin die $R_{45}$ unabhängig voneinander unsubstituiertes oder mit $C_1$-$C_6$-Alkoxy oder Halogen substituiertes lineares oder verzweigtes $C_1$-$C_{18}$-Alkyl, unsubstituiertes oder mit $C_1$-$C_6$-Alkyl, $C_1$-$C_6$-Alkoxy oder Halogen substituiertes $C_5$- oder $C_6$-Cycloalkyl, unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl, Di($C_1$-$C_6$-alkyl)amino, Di($C_1$-$C_6$-alkyl)amino-$C_1$-$C_3$-alkyl oder Halogen substituiertes Phenyl, oder unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl, Di($C_1$-$C_6$-alkyl)amino, Di($C_1$-$C_6$-alkyl)amino-$C_1$-$C_3$-alkyl oder Halogen substituiertes Benzyl oder Phenylethyl darstellen; oder

in den Formeln XV, XVa und XVb die übrigen Reste unabhängig voneinander Sekundäramino mit 2 bis 18 C-Atomen, $R_{45}$O- oder $R_{45}$S-, Halogen, unsubstituiertes oder substituiertes Cyclopentadienyl oder überbrücktes Biscyclopentadienyl oder einen Neutralliganden bedeuten, worin die $R_{45}$ unabhängig voneinander unsubstituiertes oder mit $C_1$-$C_6$-Alkoxy oder Halogen substituiertes lineares oder verzweigtes $C_1$-$C_{18}$-Alkyl, unsubstituiertes oder mit $C_1$-$C_6$-Alkyl, $C_1$-$C_6$-Alkoxy oder Halogen substituiertes $C_5$- oder $C_6$-Cycloalkyl, unsubstituiertes oder mit $C_1$-

$C_6$-Alkyl, $C_1$-$C_6$-Alkoxy, $C_1$-$C_6$-Alkoxymethyl, $C_1$-$C_6$-Alkoxyethyl, Di($C_1$-$C_6$-alkyl)amino, Di($C_1$-$C_6$-alkyl)amino-$C_1$-$C_3$-alkyl oder Halogen substituiertes Phenyl, oder unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl, Di($C_1$-$C_6$-alkyl)amino, Di($C_1$-$C_6$-alkyl)amino-$C_1$-$C_3$-alkyl oder Halogen substituiertes Benzyl oder Phenylethyl darstellen.

Für die Reste $R_{69}$ bis $R_{74}$ gelten die zuvor angegebenen Bevorzugungen.

In einer besonders bevorzugten Ausführungsform werden im erfindungsgemässen Verfahren Metallverbindungen der Formeln XV, XVa oder XVb verwendet, worin

$R_{69}$ einen Rest der Formel XIV $-CH_2-SiR_{38}R_{39}R_{40}$ und $R_{70}$ F, Cl oder Br darstellen; und

(a) in Formel XV $R_{71}$ und $R_{72}$ sowie $R_{73}$ und $R_{74}$ jeweils zusammen den Rest $=N-R_{44}$ bedeuten, oder $R_{71}$ und $R_{72}$ zusammen den Rest $=N-R_{44}$ bedeuten, und $R_{73}$ und $R_{74}$ unabhängig voneinander unsubstituiertes oder substituiertes Cyclopentadienyl, $R_{45}$-O- oder Halogen darstellen, oder

b) in Formel XVa $R_{71}$ und $R_{72}$ zusammen den Rest $=N-R_{44}$ bedeuten, und $R_{44}$ unsubstituiertes oder substituiertes Cyclopentadienyl, $R_{45}$-O- oder Halogen bedeuten, oder in Formel XVa $R_{71}$, $R_{72}$ und $R_{73}$ unabhängig voneinander unsubstituiertes oder substituiertes Cyclopentadienyl, $R_{45}$-O- oder Halogen bedeuten, oder

c) in Formel XVb $R_{71}$ und $R_{72}$ unabhängig voneinander unsubstituiertes oder substituiertes Cyclopentadienyl, $R_{45}$-O- oder Halogen bedeuten,

wobei $R_{38}$ bis $R_{44}$ die voranstehenden Bedeutungen haben. Für $R_{38}$, $R_{39}$, $R_{40}$, $R_{44}$ und $R_{45}$ gelten die voranstehenden Bevorzugungen.

Ganz besonders bevorzugt werden im erfindungsgemässen Verfahren Metallverbindungen der Formeln XVI, XVIa, XVIb, XVIc oder XVId verwendet,

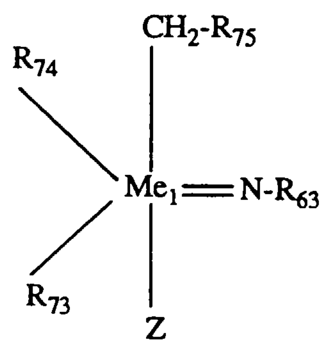

(XVI),

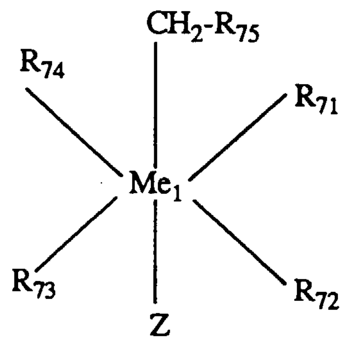

(XVIa),

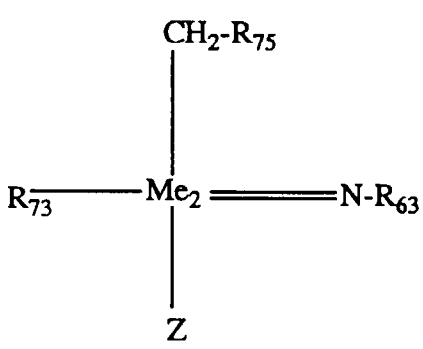

(XVIb),

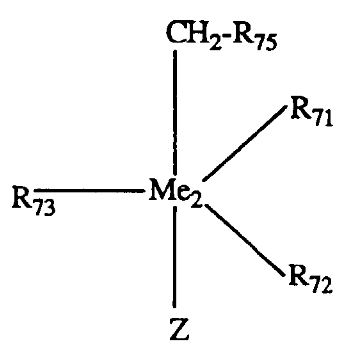

(XVIc),

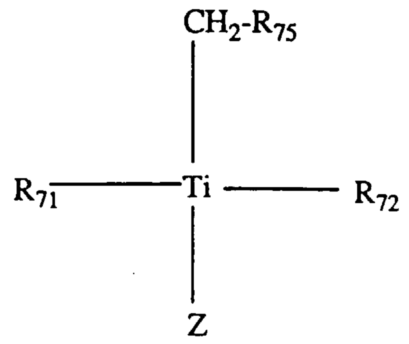

(XVId),

worin

Me$_1$ für Mo(VI) oder W(VI) steht;
Me$_2$ für Nb(V) oder Ta(V) steht;
$R_{75}$ -Si($C_1$-$C_4$-Alkyl)$_3$ darstellt;
Z für Cl oder Br steht;
$R_{63}$ Phenyl oder mit 1 bis 3 $C_1$-$C_4$-Alkyl oder $C_1$-$C_4$-Alkoxy substituiertes Phenyl bedeutet,

(a) $R_{73}$ und $R_{74}$ in Formel XVI zusammen die Gruppe =$NR_{63}$ oder einzeln unabhängig voneinander F, Cl, Br, unsubstituiertes oder mit Fluor substituiertes lineares oder verzweigtes $C_1$-$C_4$-Alkoxy, unsubstituiertes oder mit $C_1$-$C_4$-Alkyl oder $C_1$-$C_4$-Alkoxy substituiertes Phenyloxy oder unsubstituiertes oder mit $C_1$-$C_4$-Alkyl substituiertes Cyclopentadienyl darstellen;
(b) $R_{71}$, $R_{72}$, $R_{73}$ und $R_{74}$ in Formel XVIa unabhängig voneinander F, Cl, Br, unsubstituiertes oder mit Fluor substituiertes lineares oder besonders verzweigtes $C_1$-$C_4$-Alkoxy, unsubstituiertes oder mit $C_1$-$C_4$-Alkyl oder $C_1$-$C_4$-Alkoxy substituiertes Phenyloxy oder unsubstituiertes oder mit $C_1$-$C_4$-Alkyl substituiertes Cyclopentadienyl bedeuten;
(c) $R_{73}$ in Formel XVIb F, Cl, Br, unsubstituiertes oder mit Fluor substituiertes lineares oder verzweigtes $C_1$-$C_4$-Alkoxy, unsubstituiertes oder mit $C_1$-$C_4$-Alkyl oder $C_1$-$C_4$-Alkoxy substituiertes Phenyloxy oder unsubstituiertes oder mit $C_1$-$C_4$-Alkyl substituiertes Cyclopentadienyl darstellt;
(d) $R_{71}$, $R_{72}$ und $R_{73}$ in Formel XVIc unabhängig voneinander F, Cl, Br, unsubstituiertes oder mit Fluor substituiertes lineares oder besonders verzweigtes $C_1$-$C_4$-Alkoxy, unsubstituiertes oder mit $C_1$-$C_4$-Alkyl oder $C_1$-$C_4$-Alkoxy substituiertes Phenyloxy oder unsubstituiertes oder mit $C_1$-$C_4$-Alkyl substituiertes Cyclopentadienyl darstellen; und
(e) $R_{71}$ und $R_{72}$ in Formel XVId unabhängig voneinander F, Cl, Br, unsubstituiertes oder mit Fluor substituiertes lineares oder besonders verzweigtes $C_1$-$C_4$-Alkoxy, unsubstituiertes oder mit $C_1$-$C_4$-Alkyl oder $C_1$-$C_4$-Alkoxy substituiertes Phenyloxy oder unsubstituiertes oder mit $C_1$-$C_4$-Alkyl substituiertes Cyclopentadienyl darstellen. Das Alkoxy stellt besonders bevorzugt verzweigtes Alkoxy dar, das gegebenenfalls mit F teilweise oder vollständig substituiert ist, zum Beispiel i-Propyloxy, i- und t-Butyloxy, Hexafluorpopyloxy und Nonafluorpropyloxy. Bei dem Phenyloxyrest handelt es sich besonders um in den 2,6-Stellungen mit $C_1$-$C_4$-Alkyl substituiertes Phenyloxy, zum Beispiel 2,6-Dimethylphenyloxy. Beispiele für substituierte Cyclopentadienylreste sind Mono- bis Pentamethylcyclopentadienyl und Trimethylsilylcyclopentadienyl. $R_{63}$ stellt bevorzugt Phenyl oder mit $C_1$-$C_4$-Alkyl substituiertes Phenyl dar, besonders Phenyl, 3,5-Dimethyl-, 2,6-Dimethyl-, 3,5-Diethyl- und 2,6-Diethylphenyl.

Ganz besonders bevorzugte Verbindungen im erfindungsgemässen Verfahren sind solche der Formeln XVII, XVIIa, XVIIb, XVIIc und XVIId

$$(R_{63}\text{-N=})_2Me_1X_aCH_2Si(CH_3)_3 \qquad \text{(XVII)},$$

$$(R_{63}\text{-N=})R_{71}Me_1X_a(OR_{62})CH_2Si(CH_3)_3 \qquad \text{(XVIIa)},$$

$$R_{72}R_{73}Me_2X_a(OR_{62})CH_2Si(CH_3)_3 \qquad \text{(XVIIb)},$$

$$R_{63}\text{-N=}Me_2X_a(OR_{62})CH_2Si(CH_3)_3 \quad (XVIIc),$$

$$R_{71}\text{-Ti}X_a(OR_{62})CH_2Si(CH_3)_3 \quad (XVIId),$$

worin

| | |
|---|---|
| $Me_1$ | für Mo(VI) oder W(VI) steht; |
| $Me_2$ | für Nb(V) oder Ta(V) steht; |
| $X_a$ | für F oder Cl steht; |
| $R_{63}$ | Phenyl oder mit 1 oder 2 $C_1$-$C_4$-Alkylgruppen substituiertes Phenyl bedeutet; |
| $R_{62}$ | verzweigtes gegebenenfalls teilweise oder vollständig mit Fluor substituiertes $C_3$-oder $C_4$-Alkyl oder Phenyloxy oder mit 1 bis 3 Methyl- oder Ethylgruppen substituiertes Phenyloxy darstellt; |
| $R_{72}$ und $R_{73}$ | unabhängig voneinander unsubstituiertes oder mit 1 bis 5 Methylgruppen substituiertes Cyclopentadienyl, $X_a$ oder $R_{62}$O- bedeuten; und |
| $R_{71}$ | unsubstituiertes oder mit 1 bis 5 Methylgruppen substituiertes Cyclopentadienyl, $X_a$ oder $R_{72}$O- darstellt. |

Einige Beispiele für Titan(IV)-, Niob(V)-, Tantal(V)-, Molybdän(VI)- und Wolfram(VI)-verbindungen sind [Cp bedeutet Cyclopentadienyl und Me bedeutet Nb(V) oder Ta(V)]: $Ti[CH_2Si(CH_3)_3]Cl_3$, $Ti[CH_2Si(CH_3)_3]Br_3$, $Cp_2Ti[CH_2Si(CH_3)_3]Cl$, $(CH_3)_2Ti[CH_2Si(CH_3)_3]Cl$, $Cp_2Ti[CH_2Si(CH_3)_3]Br$, $Cp_2Ti[CH_2Si(CH_3)_3]I$, $CpTi[CH_2Si(CH_3)_3][CH_3]Cl$, $CpTi[CH_2Si(CH_3)_3]Br_2$, $[(CH_3)_2CHO]_2Ti[CH_2Si(CH_3)_3]Cl$, $[(CF_3)_2CHO]_2Ti[CH_2Si(CH_3)_3]Cl$, $[(CF_3)_2CHO]CpTi[CH_2Si(CH_3)_3]Cl$, $[(CH_3)_2CHO]CpTi[CH_2Si(CH_3)_3]Cl$, $(C_6H_5O)CpTi[CH_2Si(CH_3)_3]Cl$, (2,6-Dimethyl-$C_6H_5O$)$CpTi[CH_2Si(CH_3)_3]Cl$, (2,6-Dimethyl-$C_6H_5O)_2Ti[CH_2Si(CH_3)_3]Cl$, (2,6-Dimethyl-$C_6H_5O)Ti[CH_2Si(CH_3)_3]_2Br$, $[(CH_3)_3CO]CpTi[CH_2Si(CH_3)_3]Cl$, $[(CF_3)_2(CH_3)CO]CpTi[CH_2Si(CH_3)_3]Cl$, Me(=N-$C_6H_5$)$[OCH(CH_3)_2][(CH_2Si(CH_3)_3]Cl$, $Cp_2Me[(CH_2Si(CH_3)_3]Cl_2$, Me(=N-$C_6H_5$)$[OCH(CF_3)_2][(CH_2Si(CH_3)_3]Cl$, Me(=N-2,6-diisopropyl$C_6H_3$)$[(CH_2Si(CH_3)_3]Cl_2$, Me(=N-2,6-diisopropyl$C_6H_3$)$[(CH_3)_2CHO][(CH_2Si(CH_3)_3]Cl$, Me(=N-2,6-dimethyl$C_6H_3$)(2,6-Dimethyl-$C_6H_5SO$)$[CH_2Si(CH_3)_3]Cl$, Me(=N-2,6-dimethyl$C_6H_3$)$((CF_3)_2CHO)[CH_2Si(CH_3)_3]Cl$, (=N-2,6-dimethyl$C_6H_3$)$CpMe[(CH_2Si(CH_3)_3]Cl$, $(C_6H_5O)_2CpMe[(CH_2Si(CH_3)_3]Cl$, (=N-3,5-dimethyl$C_6H_3$)Me[2,6-dimethyl$C_6H_3O$)]$[(CH_2Si(CH_3)_3)]Cl$, $CpMe[OCH(CH_3)_2]_2[(CH_2Si(CH_3)_3]Br$, $CpMe[OCH(CH_3)_2]_2[(CH_2Si(CH_3)_3]Cl$, $CpMe[OCH(CF_3)_2]_2[(CH_2Si(CH_3)_3]Cl$, $Cp_2Me$(Methyl)$[(CH_2Si(CH_3)_3]Cl$, $Cp_2Me[OCH(CH_3)_2][(CH_2Si(CH_3)_3]Cl$, $[OCH(CH_3)_2]_2Me[CH_2Si(CH_3)_3]Cl_2$, Me(2,6-Dimethylphenyloxy)$(CH_3O)_2[(CH_2Si(CH_3)_3]Cl$, $Me[CH_2Si(CH_3)_3][OCH(CH_3)](CF_3O)_2Cl$, W(=N-$C_6H_5$)$[(OC(CH_3)_3][CH_2\text{-}Si(CH_3)_3]Cl_2$, (2,6-Diisopropylphenyloxy)$_2Me[CH_2Si(CH_3)_3]Cl_2$, $Cp_2Me[OC(CH_3)_3][(CH_2Si(CH_3)_3]Cl$, $CpMe[OC(CH_3)(CF_3)_2]_2[(CH_2Si(CH_3)_3]Cl$, $Mo_2[(CH_2\text{-}Si(CH_3)_3)(OCH_2C(CH_3)_3)Cl]_2$, Mo(=N-2,6-diisopropyl$C_6H_3)_2[CH_2\text{-}Si(CH_3)_3]Cl$, W(=N-$C_6H_5$)$[(OC(CH_3)_3]_2[CH_2\text{-}Si(CH_3)_3]Cl$, Mo(=N-$C_6H_5)_2[CH_2\text{-}Si(CH_3)_3]Cl$, Mo(=N-2,6-diisopropyl$C_6H_3$)$[(OCH_2C(CH_3)_3]_2[CH_2\text{-}Si(CH_3)_3]Cl$.

Die erfindungsgemäss zu verwendenden Titan-, Niob-, Tantal-, Molybdän- und Wolframverbindungen sind bekannt oder nach bekannten und analogen Verfahren ausgehend von gegebenenfalls entsprechend substituierten Metallhalogeniden mittels Grignardreaktionen herstellbar [Schrock, R.R., Murdzeck, J.S., Bazan, G.C., Robbins, J., DiMare, M., O'Regan, M., J. Am. Chem. Soc., 112:3875-3886 (1990)].

4. Weitere geeignete photoaktive Einkomponentenkatalysatoren sind Niob(V)- oder Tantal(V)verbindungen, die mindestens zwei Methylgruppen oder zwei monosubstituierte Methylgruppen am Metall gebunden enthalten, wobei der Substituent kein Wasserstoffatom in $\alpha$-Stellung enthält. Diese Verbindungen sind auch thermische Katalysatoren.

Die erfindungsgemäss zu verwendenden Niob(V)- und Tantal(V)verbindungen enthalten ein Metallatom. Die am Metall gebundene Methylgruppe oder monosubstituierte Methylgruppe ist mindestens zweimal, besonders bevorzugt zwei- bis fünfmal und insbesondere bevorzugt zwei- oder dreimal als Ligand gebunden. Dieser Ligand entspricht bevorzugt der Formel XI

$$-CH_2\text{-}R \quad (XI),$$

worin R die zuvor angegebenen Bedeutungen und Bevorzugungen hat.

Die übrigen Valenzen des Niob- und Tantalatoms sind bevorzugt mit thermostabilen Neutralliganden abgesättigt, die in grosser Vielzahl bekannt sind. Die Zahl der Neutralliganden kann auch die stöchiometrisch mögliche Zahl überschreiten (Solvate). Die Definition der Thermostabilität wurde eingangs gegeben.

Die Bedeutungen und Bevorzugungen von Neutralliganden sind zuvor gegeben worden.

In einer bevorzugten Ausführungsform entsprechen die Niob- und Tantalverbindungen besonders der Formel XVIII,

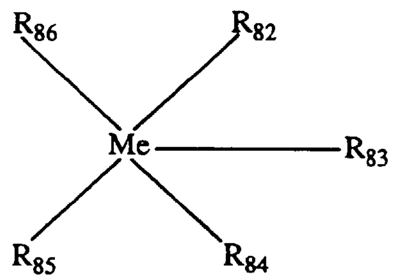

(XVIII),

worin

Me für Nb(V) oder Ta(V) steht,
mindestens zwei, bevorzugt 2 oder 3, der Reste $R_{82}$ bis $R_{86}$ einen Rest $-CH_2-R$ der Formel XI bedeuten, worin R die zuvor angegebenen Bedeutungen und Bevorzugungen hat;
zwei der übrigen Reste von $R_{82}$ bis $R_{86}$ zusammen =0 oder $=N-R_{44}$ bedeuten, und $R_{44}$ unsubstituiertes oder mit $C_1$-$C_6$-Alkoxy substituiertes lineares oder verzweigtes $C_1$-$C_{18}$-Alkyl, unsubstituiertes oder mit $C_1$-$C_6$-Alkyl, $C_1$-$C_6$-Alkoxy oder Halogen substituiertes $C_5$- oder $C_6$-Cycloalkyl, unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl, Di($C_1$-$C_6$-alkyl)amino, Di($C_1$-$C_6$-alkyl)amino-$C_1$-$C_3$-alkyl oder Halogen substituiertes Phenyl, oder unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl, Di($C_1$-$C_6$-alkyl)amino, Di($C_1$-$C_6$-alkyl)amino-$C_1$-$C_3$-alkyl oder Halogen substituiertes Benzyl oder Phenylethyl darstellt; und/oder
die übrigen Reste von $R_{82}$ bis $R_{86}$ unabhängig voneinander Sekundäramino mit 2 bis 18 C-Atomen, $R_{45}O$-, $R_{45}S$-, Halogen, Cyclopentadienyl oder überbrücktes Biscyclopentadienyl oder einen Neutralliganden bedeuten, worin die $R_{45}$ unabhängig voneinander unsubstituiertes oder mit $C_1$-$C_6$-Alkoxy oder Halogen substituiertes lineares oder verzweigtes $C_1$-$C_{18}$-Alkyl, unsubstituiertes oder mit $C_1$-$C_6$-Alkyl, $C_1$-$C_6$-Alkoxy oder Halogen substituiertes $C_5$- oder $C_6$-Cycloalkyl, unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl, Di($C_1$-$C_6$-alkyl)amino, Di($C_1$-$C_6$-alkyl)amino-$C_1$-$C_3$-alkyl oder Halogen substituiertes Phenyl, oder unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl, Di($C_1$-$C_6$-alkyl)amino, Di($C_1$-$C_6$-alkyl)amino-$C_1$-$C_3$-alkyl oder Halogen substituiertes Benzyl oder Phenylethyl darstellen.

In einer besonders bevorzugten Ausführungsform werden Niob- und Tantalverbindungen der Formel XVIII verwendet, worin

a) $R_{82}$ bis $R_{86}$ je einen Rest der Formel XI $-CH_2-R$ bedeuten, oder
b) $R_{82}$ und $R_{83}$ je einen Rest der Formel XI $-CH_2-R$ darstellen, $R_{84}$ und $R_{85}$ zusammen den Rest $=N-R_{44}$ bedeuten und $R_{86}$ unsubstituiertes oder substituiertes Cyclopentadienyl, $R_{45}$-O- oder Halogen darstellt, oder
c) $R_{82}$, $R_{83}$ und $R_{84}$ je einen Rest der Formel XI $-CH_2-R$ darstellen, und $R_{85}$ und $R_{86}$ zusammen den Rest $=N-R_{44}$ bedeuten, oder
$R_{28}$, $R_{83}$, $R_{84}$ und $R_{85}$ einen Rest der Formel XI $-CH_2-R$ darstellen und $R_{86}$ unsubstituiertes oder substituiertes Cyclopentadienyl, $R_{45}$-O- oder Halogen bedeutet,

wobei R, $R_{44}$ und $R_{45}$ die voranstehenden Bedeutungen haben. Für R, $R_{44}$ und $R_{45}$ gelten die voranstehenden Bevorzugungen.

Ganz besonders bevorzugt werden im erfindungsgemässen Verfahren Niob- und Tantalverbindungen der Formeln IXX, IXXa oder IXXb verwendet,

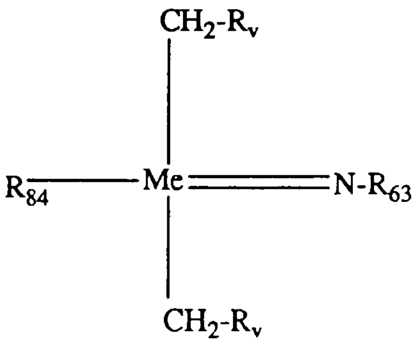 (IXX), 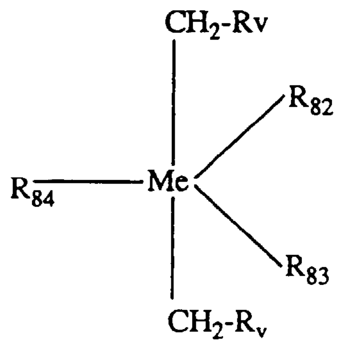 (IXXa),

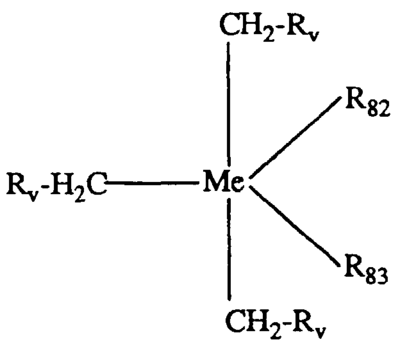 (IXXb),

worin

Me für Nb(V) oder Ta(V) steht,
$R_v$ H, $-C(CH_3)_3$, $-C(CH_3)_2-C_6H_5$, $-C_6H_5$ oder $-Si(C_1-C_4\text{-Alkyl})_3$ darstellt,
$R_{63}$ Phenyl oder mit 1 bis 3 $C_1$-$C_4$-Alkyl oder $C_1$-$C_4$-Alkoxy substituiertes Phenyl bedeutet,
$R_{84}$ in Formel IXX die Gruppe $-CH_2-R$ oder F, Cl, Br, unsubstituiertes oder mit Fluor substituiertes lineares oder besonders verzweigtes $C_1$-$C_4$-Alkoxy, unsubstituiertes oder mit $C_1$-$C_4$-Alkyl oder $C_1$-$C_4$-Alkoxy substituiertes Phenyloxy oder unsubstituiertes oder mit $C_1$-$C_4$-Alkyl substituiertes Cyclopentadienyl darstellt;
$R_{82}$, $R_{83}$ und $R_{84}$ in Formel IXXa unabhängig voneinander F, Cl, Br, unsubstituiertes oder mit Fluor substituiertes lineares oder besonders verzweigtes $C_1$-$C_4$-Alkoxy, unsubstituiertes oder mit $C_1$-$C_4$-Alkyl oder $C_1$-$C_4$-Alkoxy substituiertes Phenyloxy oder unsubstituiertes oder mit $C_1$-$C_4$-Alkyl substituiertes Cyclopentadienyl bedeuten; und
$R_{82}$ und $R_{83}$ in Formel IXXb unabhängig voneinander F, Cl, Br, unsubstituiertes oder mit Fluor substituiertes lineares oder besonders verzweigtes $C_1$-$C_4$-Alkoxy, unsubstituiertes oder mit $C_1$-$C_4$-Alkyl oder $C_1$-$C_4$-Alkoxy substituiertes Phenyloxy oder unsubstituiertes oder mit $C_1$-$C_4$-Alkyl substituiertes Cyclopentadienyl darstellen. Das Alkoxy stellt besonders bevorzugt verzweigtes Alkoxy dar, das gegebenenfalls mit F teilweise oder vollständig substituiert ist, zum Beispiel i-Propyloxy, i- und t-Butyloxy, Hexafluorpopyloxy oder Nonafluorpropyloxy.

Einige Beispiele für Niob(V)- und Tantal(V)verbindungen sind [Cp bedeutet Cyclopentadienyl und Me bedeutet Nb(V) oder Ta(V)]:
$Me[CH_2Si(CH_3)_3]_5$, $Cp_2Me[(CH_2C(CH_3)_2-C_6H_5)]_3$, Me(=N-2,6-dimethyl$C_6H_3$)$(CH_3)_3$, $Me(=N-C_6H_5)[OC(CH_3)_3][(CH_2Si(CH_3)_3)]_2$, Me(=N-2,6-diisopropyl$C_6H_3$)$[(CH_2-C_6H_5)]_3$, $Me(=N-C_6H_5)[OCCH_3(CF_3)_2][(CH_2Si(CH_3)_3)]_2$, $CpMe[OCCH_3(CF_3)_2]_2[(CH_2-C_6H_5)]_2$, Me(=N-2,6-diisopropyl$C_6H_3$)$[(CH_2C(CH_3)_2-C_6H_5)]_2Cl$, $Cp_2Me(CH_3)_2[OCH(CH_3)_2]$, Me(=N-2,6-dimethyl$C_6H_3$)$[(CH_2-C_6H_5)]_3$, $CpMe[OCH(CH_3)_2]_2[(CH_2Si(CH_3)_3)]_2$, $Cp_2Me[(CH_2-C_6H_5)]_3$, $Me[CH_2Si(CH_3)_3]_3Cl_2$, $Me[CH_2Si(CH_3)_3]_3[OCH_2C(CH_3)_3]_2$, $Cp_2Me$[3,5-dimethyl$C_6H_3O$)] $[(CH_2Si(CH_3)_3)]_2$, Me(2,6-Diisopropylphenyloxy)$_2(CH_3)_3$, $Cp_2Me(CH_3)_3$, Me(2,6-Dimethylphenyloxy)$_2(CH_3)_3$, $Me[CH_2Si(CH_3)_3]_3[OCH(CH_3)]_2$, $CpMe[OC(CH_3)_3]_2[(CH_2-C_6-H_5)]_2$ und $Cp_2Me[(CH_2Si(CH_3)_3)]_3$.
Die erfindungsgemäss zu verwendenden Niob- und Tantalverbindungen sind bekannt oder nach bekannten und analogen Verfahren ausgehend von den gegebenenfalls substituierten Metallhalogeniden über Grignardreaktionen

und/oder Substitutionsreaktionen herstellbar [Schrock, R.R., Murdzeck, J.S., Bazan, G.C., Robbins, J., DiMare, M., O'Regan, M., J. Am. Chem. Soc., 112:3875-3886 (1990)].

5. Weitere geeignete photoaktive Einkomponentenkatalysatoren sind Titan(IV)verbindungen, die mindestens zwei Methylgruppen oder zwei monosubstituierte Methylgruppen am Metall gebunden enthalten, wobei der Substituent kein Wasserstoffatom in α-Stellung enthält. Diese Verbindungen sind auch thermische Katalysatoren.

Die erfindungsgemäss zu verwendenden Titan(IV)verbindungen enthalten ein Metallatom. Die am Metall gebundene Methylgruppe oder monosubstituierte Methylgruppe ist mindestens zweimal, besonders bevorzugt zwei- bis viermal und insbesondere bevorzugt zwei- oder dreimal als Ligand gebunden. Dieser Ligand entspricht bevorzugt der Formel XI,

$$-CH_2-R \qquad (XI),$$

worin R die zuvor genannten Bedeutungen und Bevorzugungen hat.

Die übrigen Valenzen des Titanatoms sind bevorzugt mit thermostabilen Neutralliganden abgesättigt, die in grosser Vielzahl bekannt sind. Die Zahl der Neutralliganden kann auch die stöchiometrisch mögliche Zahl überschreiten (Solvate). Die Definition der Thermostabilität wurde eingangs gegeben.

Bei den Neutralliganden handelt es sich vorteilhafterweise um gleiche oder verschiedene Liganden beispielsweise aus der Gruppe bestehend aus sekundären Aminen mit 2 bis 18 C-Atomen, $R_{45}O$-, $R_{45}S$-, Halogen, Cyclopentadienyl, überbrücktem Biscyclopentadienyl, tridentaten monoanionischen Liganden und Neutralliganden wie zum Beispiel Ethern und Aminen, worin die $R_{62}$ unabhängig voneinander unsubstituiertes oder mit $C_1$-$C_6$-Alkoxy oder Halogen substituiertes lineares oder verzweigtes $C_1$-$C_{18}$-Alkyl, unsubstituiertes oder mit $C_1$-$C_6$-Alkyl, $C_1$-$C_6$-Alkoxy oder Halogen substituiertes $C_5$- oder $C_6$-Cycloalkyl, unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl oder Halogen substituiertes Phenyl, oder unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl oder Halogen substituiertes Benzyl oder Phenylethyl darstellen.

Die Bedeutungen und Bevorzugungen von $R_{45}$, von sekundären Aminen, von Halogen als weiterem Liganden an den Metallatomen oder als Substituent, von Cyclopentadienyl, Ethern, Nitrilen, tertiären Aminen und Phosphinen als Neutralliganden und von tridentaten monoanionischen Liganden sind zuvor angegeben worden. Ebenfalls zuvor angegeben worden sind die Bedeutungen und Bevorzugungen von Alkyl, Alkoxy oder Alkoxy als Substituenten im Alkoxymethyl oder -ethyl.

In einer bevorzugten Ausführungsform entsprechen die Titan(IV)verbindungen besonders der Formel XX,

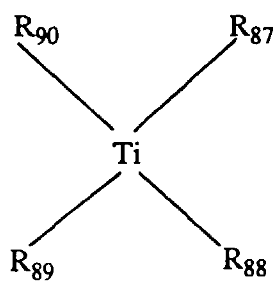

(XX),

worin

mindestens zwei, bevorzugt 2 oder 3 der Reste $R_{87}$ bis $R_{90}$ einen Rest $-CH_2-R$ der Formel XI bedeuten, worin R die zuvor genannten Bedeutungen und Bevorzugungen hat; und die übrigen Reste $R_{87}$ bis $R_{90}$ Sekundäramino mit 2 bis 18 C-Atomen, $R_{45}O$-, $R_{45}S$-, Halogen, Cyclopentadienyl oder überbrücktes Biscyclopentadienyl oder einen Neutralliganden bedeuten, worin die $R_{45}$ unabhängig voneinander unsubstituiertes oder mit $C_1$-$C_6$-Alkoxy oder Halogen substituiertes lineares oder verzweigtes $C_1$-$C_{18}$-Alkyl, unsubstituiertes oder mit $C_1$-$C_6$-Alkyl, $C_1$-$C_6$-Alkoxy oder Halogen substituiertes $C_5$- oder $C_6$-Cycloalkyl, unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl, Di($C_1$-$C_6$-alkyl)amino, Di($C_1$-$C_6$-alkyl)amino-$C_1$-$C_3$-alkyl oder Halogen substituiertes Phenyl, oder unsubstituiertes oder mit $C_1$-$C_6$-Alkyl, $C_1$-$C_6$-Alkoxy, $C_1$-$C_6$-Alkoxymethyl, $C_1$-$C_6$-Alkoxyethyl, Di($C_1$-$C_6$-alkyl)amino, Di ($C_1$-$C_6$-alkyl)amino-$C_1$-$C_3$-alkyl oder Halogen substituiertes Benzyl oder Phenylethyl darstellen.

In einer besonders bevorzugten Ausführungsform werden im erfindungsgemässen Verfahren Titan(IV)verbindungen der Formel XX verwendet, worin

a) $R_{87}$ bis $R_{90}$ einen Rest der Formel XI $-CH_2-R$ bedeuten, oder

b) $R_{87}$ und $R_{88}$ einen Rest der Formel XI $-CH_2-R$ darstellen, und $R_{89}$ und $R_{90}$ unabhängig voneinander unsubstituiertes oder substituiertes Cyclopentadienyl, $R_{45}$-O- oder Halogen bedeuten, oder

c) $R_{87}$, $R_{88}$ und $R_{89}$ einen Rest der Formel XI $-CH_2-R$ darstellen, und $R_{90}$ unsubstituiertes oder substituiertes Cyclopentadienyl, $R_{45}$-O- oder Halogen bedeutet,

wobei R und $R_{45}$ die voranstehenden Bedeutungen haben. Für R und $R_{45}$ gelten die voranstehenden Bevorzugungen.

Ganz besonders bevorzugt werden im erfindungsgemässen Verfahren Titan(IV)verbindungen der Formeln XXIa oder XXIb verwendet,

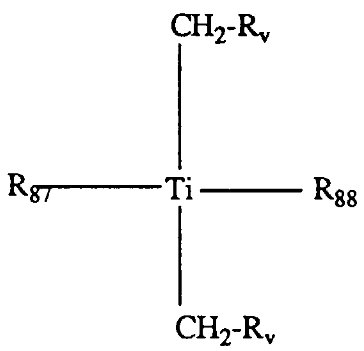
(XXIa),

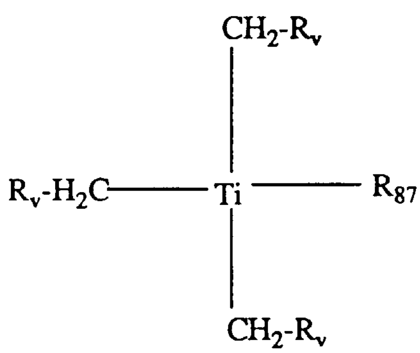
(XXIb),

worin

$R_v$ H, $-C(CH_3)_3$, $-C(CH_3)_2-C_6H_5$, $-C_6H_5$ oder $-Si(C_1-C_4\text{-Alkyl})_3$ darstellt, und

$R_{87}$ und $R_{88}$ unabhängig voneinander F, Cl, Br, unsubstituiertes oder mit Fluor substituiertes lineares oder besonders verzweigtes $C_1$-$C_4$-Alkoxy, unsubstituiertes oder mit $C_1$-$C_4$-Alkyl oder $C_1$-$C_4$-Alkoxy substituiertes Phenyloxy oder unsubstituiertes oder mit $C_1$-$C_4$-Alkyl substituiertes Cyclopentadienyl darstellen. Das Alkoxy stellt besonders bevorzugt verzweigtes Alkoxy dar, das gegebenenfalls mit F teilweise oder vollständig substituiert ist, zum Beispiel i-Propyloxy, i- und t-Butyloxy, Hexafluorpropyloxy und Nonafluorpropyloxy.

In einer bevorzugten Ausführungsform der Erfindung enthalten die Titan(IV)verbindungen ein an das Titan gebundenes Halogenatom, besonders Cl oder Br, wenn in der Gruppe $-CH_2-R$ der Rest R für $-SiR_{38}R_{39}R_{40}$ steht. Ganz besonders bevorzugt sind dann die Verbindungen der Formel XXII,

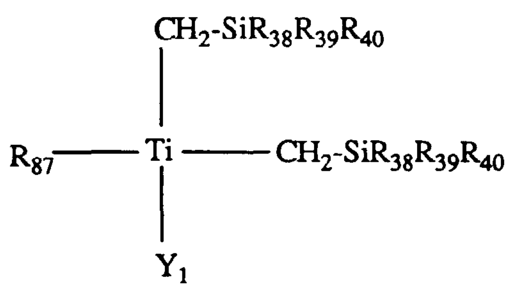
(XXII),

worin

$Y_1$ für F, Cl oder Br steht,

$R_{38}$, $R_{39}$ und $R_{40}$ unabhängig voneinander $C_1$-$C_{18}$-Alkyl, $C_5$- oder $C_6$-Cycloalkyl oder unsubstituiertes oder mit $C_1$-$C_6$-Alkyl oder $C_1$-$C_6$-Alkoxy substituiertes Phenyl oder Benzyl bedeuten; und

$R_{87}$ die Gruppe $-CH_2-SiR_{38}R_{39}R_{40}$, F, Cl, Br, unsubstituiertes oder mit Fluor substituiertes lineares oder besonders verzweigtes $C_1$-$C_4$-Alkoxy, unsubstituiertes oder mit $C_1$-$C_4$-Alkyl oder $C_1$-$C_4$-Alkoxy substituiertes Phenyloxy oder unsubstituiertes oder mit $C_1$-$C_4$-Alkyl substituiertes Cyclopentadienyl darstellt. $R_{38}$, $R_{39}$ und $R_{40}$ bedeuten bevorzugt $C_1$-$C_4$-Alkyl, Phenyl oder Benzyl, und $R_{87}$ stellt bevorzugt Cl, unsubstituiertes oder mit Fluor substituiertes

$C_3$- oder $C_4$-Alkyl oder unsubstituiertes oder mit $C_1$-$C_4$-Alkyl oder $C_1$-$C_4$-Alkoxy substituiertes Phenyl oder Benzyl dar.

Einige Beispiele für Titan(IV)verbindungen sind [Cp bedeutet Cyclopentadienyl]: $Ti[CH_2Si(CH_3)_3]_4$, $Ti[OCH(CF_3)_2]_2[(CH_2Si(CH_3)_3)]_2$, $CpTi[(CH_2C(CH_3)_2\text{-}C_6H_5)]_2Cl$, $CpTi[(CH_2\text{-}C_6H_5)]_3$, $TiCl_2[CH_2Si(CH_3)_3)]_2$, $[OCH(CF_3)_2]Ti[(CH_2\text{-}C_6H_5)]_3$, $CpBrTi[(CH_2C(CH_3)_2\text{-}C_6H_5)]_2$, CpTi[2,6-dimethyl$C_6H_3O$)][$(CH_2Si(CH_3)_3)]_2$, $Ti[OCH(CH_3)_2]_2[(CH_2\text{-}C_6H_5)]_2$, $ClTi[OCH(CH_3)_2][(CH_2Si(CH_3)_3)]_2$, $CpTi[OCH(CF_3)_2][(CH_2\text{-}C_6H_5)]_2$, CpTi(methyl)$_3$, CpTi(Methyl)$_2$[OCH$(CH_3)_2$], $Ti[CH_2Si(CH_3)_3]_2Br_2$, Ti(2,6-Dimethylphenyloxy)$_2(CH_3)_2$, $Cp_2Ti(CH_3)_2$, $Ti[CH_2Si(CH_3)_3]_3[OCH(CH_3)]$ und Ti(2,6-Diisopropylphenyloxy)$_2(CH_3)_2$.

Die erfindungsgemäss zu verwendenden Titan(IV)verbindungen sind bekannt oder nach bekannten und analogen Verfahren ausgehend von den Metallhalogeniden über Grignardreaktionen oder andere bekannte Substitutionsreaktionen herstellbar [siehe Clauss, K., Bestian, H., Justus Liebigs Ann. Chem. 654:8-19 (1962)].

6. Andere geeignete photokatalytisch aktive Verbindungen sind Ruthenium- oder Osmiumverbindungen, die mindestens eine Phosphingruppe, mindestens einen photolabilen Liganden, und gegebenenfalls Neutralliganden an das Metallatom gebunden enthalten, wobei insgesamt 2 bis 5 Liganden gebunden sind, und die Säureanionen zum Ladungsausgleich enthalten. Insgesamt bedeutet im Rahmen der Erfindung die Summe der Phosphingruppen, photolabilen Liganden und Neutralliganden. Die Neutralliganden werden auch als nicht-photolabile Liganden bezeichnet. Insgesamt sind bevorzugt 2 bis 4, und besonders bevorzugt 2 oder 3 Liganden gebunden.

Die Osmiumverbindungen sind auch thermisch wirksame Katalysatoren. Auch die Rutheniumverbindungen sind thermische Katalysatoren, wenn die Phosphingruppe keine linearen Alkyl- oder Alkoxygruppen enthält, sondern raumerfüllende Gruppen, zum Beispiel sekundäre und tertiäre Alkyl- oder Alkoxygruppen (i-Propyl, i- und t-Butyl), oder Cycloalkylgruppen, oder unsubstituierte oder mit 1 bis 3 $C_1$-$C_4$-Alkyl oder -Alkoxy substituierte Phenylgruppen oder Phenyloxygruppen.

Bei der Phosphingruppe handelt es sich bevorzugt um tertiäre Phosphine mit 3 bis 40, bevorzugter 3 bis 30 und besonders bevorzugt 3 bis 24 C-Atomen.

Die übrigen Valenzen des Rutheniums und Osmiums sind bevorzugt mit thermostabilen Neutralliganden abgesättigt, die in grosser Vielzahl bekannt sind. Die Zahl der Neutralliganden kann auch die stöchiometrisch mögliche Zahl überschreiten (Solvate).

Bei den erfindungsgemäss zu verwendenden Ruthenium- und Osmiumverbindungen kann ein Monophosphin ein bis dreimal und bevorzugt zwei oder dreimal, und ein Diphosphin einmal an das Metallatom gebunden sein. In den Ruthenium- und Osmiumkatalysatoren sind bevorzugt 1 bis 2 photolabile Liganden gebunden. Die Phosphinliganden entsprechen bevorzugt den Formeln XXIII und XXIIIa,

$$PR_{91}R_{92}R_{93} \quad \text{(XXIII)},$$

$$R_{91}R_{92}P\text{-}Z_1\text{-}PR_{91}R_{92} \quad \text{(XXIIIa)},$$

worin

$R_{91}$, $R_{92}$ und $R_{93}$ unabhängig voneinander H, $C_1$-$C_{20}$-Alkyl, unsubstituiertes oder mit $C_1$-$C_6$-Alkyl, $C_1$-$C_6$-Halogenalkyl oder $C_1$-$C_6$-Alkoxy substituiertes $C_4$-$C_{12}$-Cycloalkyl oder unsubstituiertes oder mit $C_1$-$C_6$-Alkyl, $C_1$-$C_6$-Halogenalkyl oder $C_1$-$C_6$-Alkoxy substituiertes $C_6$-$C_{16}$-Aryl oder unsubstituiertes oder mit $C_1$-$C_6$-Alkyl, $C_1$-$C_6$-Halogenalkyl oder $C_1$-$C_6$-Alkoxy substituiertes $C_7$-$C_{16}$-Aralkyl darstellt; die Reste $R_{91}$ und $R_{92}$ gemeinsam unsubstituiertes oder mit $C_1$-$C_6$-Alkyl, $C_1$-$C_6$-Halogenalkyl oder $C_1$-$C_6$-Alkoxy substituiertes Tetra- oder Pentamethylen bedeuten, oder unsubstituiertes oder mit $C_1$-$C_6$-Alkyl, $C_1$-$C_6$-Halogenalkyl oder $C_1$-$C_6$-Alkoxy substituiertes und mit 1 oder 2 1,2-Phenylen kondensiertem Tetra- oder Pentamethylen darstellen, und $R_{93}$ die zuvor angegebene Bedeutung hat; und

$Z_1$ lineares oder verzweigtes, unsubstituiertes oder mit $C_1$-$C_4$-Alkoxy substituiertes $C_2$-$C_{12}$-Alkylen, unsubstituiertes oder mit $C_1$-$C_4$-Alkyl oder $C_1$-$C_4$-Alkoxy substituiertes 1,2- oder 1,3-Cycloalkylen mit 4 bis 8 C-Atomen, oder unsubstituiertes oder mit $C_1$-$C_4$-Alkyl oder $C_1$-$C_4$-Alkoxy substituiertes 1,2 oder 1,3-Heterocycloalkylen mit 5 oder 6 Ringgliedern und einem Heteroatom aus der Gruppe O oder N bedeutet.

Bei den Resten $R_{91}$, $R_{92}$ und $R_{93}$ handelt es sich bevorzugt um gleiche Reste.

Sofern $R_{91}$, $R_{92}$ und $R_{93}$ substituiert sind, handelt es sich bei den Substituenten bevorzugt um $C_1$-$C_4$-Alkyl, $C_1$-$C_4$-Halogenalkyl oder $C_1$-$C_4$-Alkoxy. Halogen bedeutet bevorzugt Cl und besonders bevorzugt F. Beispiele für bevorzugte Substituenten sind Methyl, Methoxy, Ethyl, Ethoxy und Trifluormethyl. $R_{91}$, $R_{92}$ und $R_{93}$ sind bevorzugt mit 1 bis 3 Substituenten substituiert. Substituenten befinden sich bevorzugt in einer oder beiden Orthound/oder Metastellungen zum C-Atom der P-C-Bindung im Phosphin.

$R_{91}$, $R_{92}$ und $R_{93}$ kann als Alkyl linear oder verzweigt sein und bevorzugt 1 bis 12, bevorzugter 1 bis 8, und besonders bevorzugt 1 bis 6 C-Atome enthalten. Beispiele für Alkyl sind Methyl, Ethyl, n- und i-Propyl, n-, i- und t-Butyl, die Isomeren von Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl und Eicosyl. Bevorzugte Beispiele sind Methyl, Ethyl, n- und i-Propyl, n-, i- und t-Butyl, 1-, 2- oder 3-Pentyl und 1-, 2-, 3- oder 4-Hexyl.

Bedeuten $R_{91}$, $R_{92}$ und $R_{93}$ Cycloalkyl, so handelt es sich bevorzugt um $C_5$-$C_8$-Cycloalkyl, und besonders bevorzugt um $C_5$- oder $C_6$-Cycloalkyl. Einige Beispiele sind Cyclobutyl, Cycloheptyl, Cyclooctyl und besonders Cyclopentyl und Cyclohexyl. Beispiele für substituiertes Cycloalkyl sind Methyl-, Dimethyl-, Trimethyl-, Methoxy-, Dimethoxy-, Trimethoxy-, Trifluormethyl-, Bistrifluormethyl und Tristrifluormethylcyclopentyl und -cyclohexyl.

Bedeuten $R_{91}$, $R_{92}$ und $R_{93}$ Aryl, so handelt es sich bevorzugt um $C_6$-$C_{12}$-Aryl und besonders bevorzugt um Phenyl oder Naphthyl. Beispiele für substituiertes Aryl sind Methyl-, Dimethyl-, Trimethyl-, Methoxy-, Dimethoxy-, Trimethoxy-, Trifluormethyl-, Bistrifluormethyl und Tristrifluormethylphenyl.

Bedeuten $R_{91}$, $R_{92}$ und $R_{93}$ Aralkyl, so handelt es sich bevorzugt um $C_7$-$C_{13}$-Aralkyl, wobei die Alkylengruppe im Aralkyl bevorzugt Methylen darstellt. Besonders bevorzugt stellt das Aralkyl Benzyl dar. Beispiele für substituiertes Aralkyl sind Methyl-, Dimethyl-, Trimethyl-, Methoxy-, Dimethoxy-, Trimethoxy-, Trifluormethyl-, Bistrifluormethyl und Tristrifluormethylbenzyl.

Beispiele für an das P-Atom gebundenes, gegebenenfalls substituiertes beziehungsweise kondensiertes Tetra- oder Pentamethylen sind

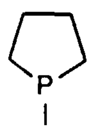 , 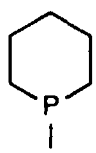 , 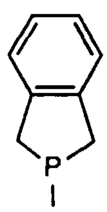 und 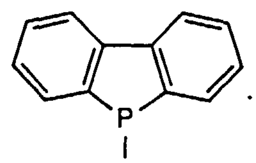 .

Andere geeignete Phosphine sind mit einer =PRa-Gruppe überbrückte Cycloaliphate mit 6 bis 8 Ringkohlenstoffatomen, zum Beispiel

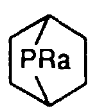 , 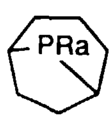 , 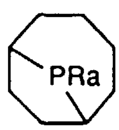 und 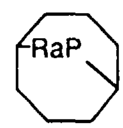 ,

worin Ra $C_1$-$C_6$-Alkyl, Cyclohexyl, Benzyl, unsubstituiertes oder mit 1 oder 2 $C_1$-$C_4$-Alkyl substituiertes Phenyl bedeutet.

Bei $Z_1$ als linearem oder verzweigtem Alkylen handelt es sich bevorzugt um 1,2-Alkylen oder 1,3-Alkylen mit vorzugsweise 2 bis 6 C-Atomen, zum Beispiel um Ethylen, 1,2-Propylen oder 1,2-Butylen.

Beispiele für $Z_1$ als Cycloalkylen sind 1,2- und 1,3-Cyclopentylen und 1,2- oder 1,3-Cyclohexylen. Beispiele für $Z_1$ als Heterocycloalkylen sind 1,2- und 1,3-Pyrrolidin, 1,2- und 1,3-Piperidin, und 1,2- und 1,3-Tetrahydrofuran.

In einer bevorzugten Ausführungsform entsprechen die Phosphinliganden der Formel XXIII, worin $R_{91}$, $R_{92}$ und $R_{93}$ unabhängig voneinander H, $C_1$-$C_6$-Alkyl, unsubstituiertes oder mit $C_1$-$C_4$-Alkyl substituiertes Cyclopentyl oder Cyclohexyl, oder unsubstituiertes oder mit $C_1$-$C_4$-Alkyl, $C_1$-$C_4$-Alkyl $C_1$-$C_4$-Alkoxy oder Trifluormethyl substituiertes Phenyl oder unsubstituiertes oder mit $C_1$-$C_4$-Alkyl, $C_1$-$C_4$-Alkyl $C_1$-$C_4$-Alkoxy oder Trifluormethyl substituiertes Benzyl darstellen. Besonders bevorzugte Beispiele für Phosphinliganden der Formel XXIII sind $(C_6H_5)_3P$, $(C_6H_5CH_2)_3P$, $(C_5H_{11})_3P$, $(CH_3)_3P$, $(C_2H_5)_3P$, $(n\text{-}C_3H_7)_3P$, $(1\text{-}C_3H_7)_3P$, $(n\text{-}C_4H_9)_3P$, $(C_6H_5)_2HP$, $(C_6H_5CH_2)_2HP$, $(C_5H_{11})_2HP$, $(C_2H_5)_2HP$, $(n\text{-}C_3H_7)_2HP$, $(i\text{-}C_3H_7)_2HP$, $(n\text{-}C_4H_9)_2HP$, $(C_6H_5)H_2P$, $(n\text{-}C_4H_9)H_2P$, $(C_6H_5CH_2)H_2P$, $(C_5H_{11})H_2P$, $(CH_3)H_2P$, $(CH_3)_2HP$, $(C_2H_5)H_2P$, $(n\text{-}C_3H_7)H_2P$, $(i\text{-}C_3H_7)H_2P$, $PH_3$$(2\text{-Methyl-}C_6H_4)_3P$, $(3\text{-}CH_3\text{-}C_6H_4)_3P$, $(4\text{-}C_2H_5\text{-}C_6H_4)_3P$, $(4\text{-}CH_3\text{-}C_6H_4)_3P$, $(2,4\text{-Di-}CH_3\text{-}C_6H_3)_3P$, $(2,6\text{-Di-}CH_3\text{-}C_6H_3)_3P$, $(2\text{-}C_2H_5\text{-}C_6H_4)_3P$, $(3\text{-}C_2H_5\text{-}C_6H_4)_3P$, $(2\text{-n-}C_3H_7\text{-}C_6H_4)_3P$, $(3\text{-n-}C_3H_7\text{-}C_6H_4)_3P$, $(4\text{-n-}C_3H_7\text{-}C_6F_4)_3P$, $(2\text{-i-}C_3H_7\text{-}C_6H_4)_3P$, $(3\text{-i-}C_3H_7\text{-}C_6H_4)_3P$, $(4\text{-i-}C_3H_7\text{-}C_6H_4)_3P$, $(2\text{-n-}C_4H_9\text{-}C_6H_4)_3P$,

$(3\text{-}n\text{-}C_4H_9\text{-}C_6H_4)_3P$, $(4\text{-}n\text{-}C_4H_9\text{-}C_6\text{-}H_4)_3P$, $(2\text{-}i\text{-}C_4H_9\text{-}C_6H_4)_3P$, $(3\text{-}i\text{-}C_4H_9\text{-}C_6H_4)_3P$, $(4\text{-}i\text{-}C_4H_9\text{-}C_6H_4)_3P$, $(2\text{-}t\text{-}C_4H_9\text{-}C_6H_4)_3P$, $(3\text{-}t\text{-}C_4H_9\text{-}C_6H_4)_3P$, $(4\text{-}t\text{-}C_4H_9\text{-}C_6H_4)_3P$, $(2\text{-}CH_3\text{-}6\text{-}t\text{-}C_4H_9\text{-}C_6H_3)_3P$, $(3\text{-}CH_3\text{-}6\text{-}t\text{-}C_4H_9\text{-}C_6H_3)_3P$, $(3\text{-}CH_3\text{-}6\text{-}t\text{-}C_4H_9\text{-}C_6H_3)_3P$, $(2,6\text{-Di-}t\text{-}C_4H_9\text{-}C_6H_3)_3P$, $(2,3\text{-Di-}t\text{-}C_4H_9\text{-}C_6H_3)_3P$ und $(2,4\text{-Di-}t\text{-}C_4H_9\text{-}C_6H_3)_3P$.

Als Liganden für die erfindungsgemäss zu verwendenden Ruthenium- und Osmiumverbindungen werden organische oder anorganische Verbindungen, Atome oder Ionen bezeichnet, die an ein Metallzentrum koordiniert sind.

Die Bedeutungen und Bevorzugungen von photolabilen Liganden und nicht-photolabilen Liganden (auch als stark koordinierende Liganden bezeichnet) sind zuvor genannt worden.

In einer bevorzugten Ausführungsform enthalten die erfindungsgemäss zu verwendenden Ru- und Os-Katalysatoren nur photolabile Liganden, Phosphingruppen und Anionen zum Ladungsausgleich. Ganz besonders bevorzugt sind die Katalysatoren, die eine Arengruppe als photolabilen Liganden, eine tertiäre Phosphingruppe und ein- oder zweiwertige Anionen zum Ladungsausgleich enthalten.

Geeignete Anionen von anorganischen oder organischen Säuren sind zum Beispiel Hydrid ($H^{\ominus}$ ), Halogenid (zum Beispiel $F^{\ominus}$, $Cl^{\ominus}$, $Br^{\ominus}$ und $I^{\ominus}$), das Anion einer Sauerstoffsäure, und $BF_4^{\ominus}$, $PF_6^{\ominus}$, $SbF_6^{\ominus}$ oder $AsF_6^{\ominus}$. Es ist zu erwähnen, dass das zuvor erwähnte Cyclopentadienyl Ligand und Anion ist.

Weitere geeignete Anionen sind $C_1$-$C_{12}$-, bevorzugt $C_1$-$C_6$- und besonders bevorzugt $C_1$-$C_4$-Alkoholate, die insbesondere verzweigt sind, zum Beispiel der Formel $R_xR_yR_zC\text{-}O^{\ominus}$ entsprechen, worin $R_x$ H oder $C_1$-$C_{10}$-Alkyl, $R_y$ $C_1$-$C_{10}$-Alkyl und $R_z$ $C_1$-$C_{10}$-Alkyl oder Phenyl darstellen, und die Summe der C-Atome von $R_x$,$R_y$ und $R_z$ 11 beträgt. Beispiele sind besonders i-Propyloxy und t-Butyloxy.

Andere geeignete Anionen sind $C_3$-$C_{18}$-, bevorzugt $C_5$-$C_{14}$- und besonders bevorzugt $C_5$-$C_{12}$-Acetylide, die der Formel $R_w\text{-}C{\equiv}C^{\ominus}$ entsprechen können, worin $R_w$ $C_1$-$C_{16}$-Alkyl, bevorzugt $\alpha$-verzweigtes $C_3$-$C_{12}$-Alkyl, zum Beispiel der Formel $R_xR_yR_zC$-, bedeutet, oder unsubstituiertes oder mit 1 bis 3 $C_1$-$C_4$-Alkyl oder $C_1$-$C_4$-Alkoxy substituiertes Phenyl oder Benzyl darstellen. Einige Beispiele sind i-Propyl-, i- und t-Butyl-, Phenyl-, Benzyl-, 2-Methyl-, 2,6-Dimethyl-, 2-1-Propyl-, 2-1-Propyl-6-methyl-, 2-t-Butyl-, 2,6-Di-t-butyl- und 2-Methyl-6-t-butylphenylacetylid.

Die Bedeutungen und Bevorzugungen von Anionen von Sauerstoffsäuren sind zuvor genannt worden.

Besonders bevorzugt sind $H^{\ominus}$, $F^{\ominus}$, $Cl^{\ominus}$, $Br^{\ominus}$, $BF_4^{\ominus}$, $PF_6^{\ominus}$ , $SbF_6^{\ominus}$, $AsF_6^{\ominus}$, $CF_3SO_3^{\ominus}$, $C_6H_5\text{-}SO_3^{\ominus}$ , 4-Methyl-$C_6H_5\text{-}SO_3^{\ominus}$, 2,6-Dimethyl-$C_6H_5\text{-}SO_3^{\ominus}$, 2,4,6-Trimethyl-$C_6H_5\text{-}SO_3^{\ominus}$ und 4-$CF_3$-$C_6H_5\text{-}SO_3^{\ominus}$ sowie Cyclopentadienyl ($Cp^{\ominus}$).

Die Anzahl der nicht-photolabilen Liganden hängt von der Anzahl der Phosphingruppen, der Grösse der nicht-photolabilen Liganden und der Anzahl der photolabilen Liganden ab.

In einer bevorzugten Ausführungsform entsprechen die Ruthenium- und Osmiumverbindungen besonders bevorzugt einer der Formeln XXIV bis XXIVf

$$R_{97}L_1Me^{2+}(Z^{n-})_{2/n} \quad \text{(XXIV)},$$

$$R_{97}L_2L_3Me^{2+}(Z^{n-})_{2/n} \quad \text{(XXIVa)},$$

$$(R_{97})_2L_2Me^{2+}(Z^{n-})_{2/n} \quad \text{(XXIVb)},$$

$$(R_{97})_3L_2Me^{2+}(Z^{n-})_{2/n} \quad \text{(XXIVc)},$$

$$R_{97}L_1L_2Me^{2+}(Z^{n-})_{2/n} \quad \text{(XXIVd)},$$

$$R_{97}L_2L_2Me^{2+}(Z^{n-})_{2/n} \quad \text{(XXIVe)},$$

$$R_{97}L_1L_3Me^{2+}(Z^{n-})_{2/n} \quad \text{(XXIVf)},$$

worin

$R_{97}$ ein tertiäres Phosphin der Formel XXIII oder XXIIIa ist;
Me für Ru oder Os steht;

n für die Zahlen 1, 2 oder 3 steht;
Z das Anion einer anorganischen oder organischen Säure ist;

(a) $L_1$ einen Aren- oder Heteroarenliganden bedeutet;
(b) $L_2$ einen von $L_1$ verschiedenen einwertigen photolabilen Liganden darstellt; und
(c) $L_3$ einen einwertigen nicht-photolabilen Liganden bedeutet.

Für $R_{97}$, $L_8$, $L_7$ und $L_{10}$ gelten die zuvor für die einzelnen Bedeutungen angegebenen Bevorzugungen.

In den Formeln XXIV bis XXIVf steht n bevorzugt für 1 oder 2 und ganz besonders für 1. Für $R_{97}$ gelten die für die Phosphinliganden der Formel XXIII angegebenen Bevorzugungen, insbesondere handelt es sich um tertiäre Phosphine.

Ganz besonders bevorzugt werden im erfindungsgemässen Verfahren Ruthenium- und Osmiumverbindungen einer der Formeln XXV bis XXVf verwendet,

$$(R_{94}R_{95}R_{96}P)L_8Me^{2+}(Z^{1-})_2 \quad \text{(XXV)},$$

$$(R_{94}R_{95}R_{96}P)_2L_9Me^{2+}(Z^{1-})_2 \quad \text{(XXVa)},$$

$$(R_{94}R_{95}R_{96}P)L_9L_{10}Me^{2+}(Z^{1-})_2 \quad \text{(XXVb)},$$

$$(R_{94}R_{95}R_{96}P)_3L_9Me^{2+}(Z^{1-})_2 \quad \text{(XXVc)},$$

$$(R_{94}R_{95}R_{96}P)L_9L_9Me^{2+}(Z^{1-})_2 \quad \text{(XXVd)},$$

$$(R_{94}R_{95}R_{96}P)L_8L_{10}Me^{2+}(Z^{1-})_2 \quad \text{(XXVe)},$$

$$(R_{94}R_{95}R_{96}P)L_8(L_9)_mMe^{2+}(Z^{1-})_2 \quad \text{(XXVf)},$$

worin

Me für Ru oder Os steht;
Z in Formeln XXV bis XXVe $H^{\ominus}$, Cyclopentadienyl, $Cl^{\ominus}$, $Br^{\ominus}$, $BF_4^{\ominus}$, $PF_6^{\ominus}$, $SbF_6^{\ominus}$, $AsF_6^{\ominus}$, $CF_3SO_3^{\ominus}$, $C_6H_5$-$SO_3^{\ominus}$, 4-Methyl-$C_6H_5$-$SO_3^{\ominus}$, 3,5-Dimethyl-$C_6H_S$-$SO_3^{\ominus}$, 2,4,6-Trimethyl-$C_6H_5$-$SO_3^{\ominus}$ und 4-$CF_3$-$C_6H_5$-$SO_3^{\ominus}$ und in Formel XXVf $H^{\ominus}$, Cyclopentadienyl, $BF_4^{\ominus}$, $PF_6^{\ominus}$, $SbF_6^{\ominus}$, $AsF_6^{\ominus}$, $CF_3SO_3^{\ominus}$, $C_6H_5$-$SO_3^{\ominus}$, 4-Methyl-$C_6H_5$-$SO_3^{\ominus}$, 2,6-Dimethyl-$C_6H_5$-$SO_3^{\ominus}$, 2,4,6-Trimethyl-$C_6H_5$-$SO_3^{\ominus}$ oder 4-$CF_3$-$C_6H_5$-$SO_3^{\ominus}$ bedeutet;
$R_{94}$, $R_{95}$ und $R_{96}$ unabhängig voneinander $C_1$-$C_6$-Alkyl, unsubstituiertes oder mit 1 bis 3 $C_1$-$C_4$-Alkyl substituiertes Cyclopentyl oder Cyclohexyl oder Cyclopentyloxy oder Cyclohexyloxy, oder unsubstituiertes oder mit 1 bis 3 $C_1$-$C_4$-Alkyl substituiertes Phenyl oder Benzyl oder Phenyloxy oder Benzyloxy darstellen;
$L_8$ unsubstituiertes oder mit 1 bis 3 $C_1$-$C_4$-Alkyl, $C_1$-$C_4$-Alkoxy, -OH, -F oder Cl substituiertes $C_6$-$C_{16}$-Aren oder $C_5$-$C_{16}$-Heteroaren darstellt;
$L_9$ $C_1$-$C_6$-Alkyl-CN, Benzonitril oder Benzylnitril bedeutet; und
$L_{10}$ $H_2O$ oder $C_1$-$C_6$-Alkanol ist.

Bevorzugte Arene und Heteroarene sind Benzol, Toluol, Xylol, Trimethylbenzol, Naphthalin, Biphenyl, Anthracen, Acenaphthen, Fluoren, Phenanthren, Pyren, Chrysen, Fluoranthren, Furan, Thiophen, Pyrrol, Pyridin, γ-Pyran, γ-Thiopyran, Pyrimidin, Pyrazin, Indol, Cumaron, Thionaphthen, Carbazol, Dibenzofuran, Dibenzothiophen, Pyrazol, Imidazol, Benzimidazol, Oxazol, Thiazol, Isoxazol, Isothiazol, Chinolin, Isochinolin, Acridin, Chromen, Phenazin, Phenoxazin, Phenothiazin, Triazine, Thianthren und Purin. Bevorzugtere Arene und Heteroarene sind Benzol, Naphthalin, Cumen, Thiophen und Benzthiophen. Ganz besonders bevorzugt ist das Aren Benzol oder ein mit $C_1$-$C_4$-Alkyl substituiertes Benzol wie zum Beispiel Toluol, Xylol, Isopropylbenzol, Teniärbutylbenzol oder Cumen und das Heteroaren ist bevorzugt Thiophen.

Wenn die Herstellung der Ruthenium- und Osmiumkatalysatoren in Lösungsmitteln vorgenommen wird, die an ein Metallatom koordinieren können, wie zum Beispiel Alkanolen, so können sich solvatierte Ru/Os-Kationkomplexe bilden, die im Rahmen der Verwendung gemäss der Erfindung mitumfasst werden.

Einige Beispiele für gemäss der Erfindung zu verwendende Ruthenium- und Osmiumverbindingen sind [Tos ist gleich Tosylat]: $(C_6H_{11})_2HPRu(p\text{-}Cumen)Cl_2$, $(C_6H_{11})_3PRu(p\text{-}Cumen)Cl_2$, $(C_6H_{11})_3PRu(p\text{-}Cumen)(Tos)_2$, $(C_6H_{11})_3PRu(p\text{-}Cumen)Br_2$, $(C_6H_{11})_3PRu(p\text{-}Cumen)ClF$, $(C_6H_{11})_3PRu(C_6H_6)(Tos)_2$, $(C_6H_{11})_3PRu(CH_3\text{-}C_6H_5)(Tos)_2$, $(C_6H_{11})_3PRu(i\text{-}C_3H_7\text{-}C_6H_5)(Tos)_2$, $(C_6H_{11})_3PRu(Chrysen)(Tos)_2$, $(C_6H_{11})_3PRu(Biphenyl)(Tos)_2$, $(C_6H_{11})_3PRu(Anthracen)(Tos)_2$, $(C_6H_{11})_3PRu(C_{10}H_8)(Tos)_2$, $(i\text{-}C_3H_7)_3PRu(p\text{-}Cumen)Cl_2$, $(CH_3)_3PRu(p\text{-}Cumen)Cl_2$, $(n\text{-}C_4H_9)_3PRu(p\text{-}Cumen)Cl_2$, $[(C_6H_{11})_3P]_2Ru(CH_3\text{-}CN)(Tos)_2$, $(C_6H_{11})_3PRu(CH_3\text{-}CN)(C_2H_5\text{-}OH)(Tos)_2$, $(C_6H_{11})_3PRu(p\text{-}Cumen)(CH_3\text{-}CN)_2(PF_6)_2$, $(C_6H_{11})_3PRu(p\text{-}Cumen)(CH_3\text{-}CN)_2(Tos)_2$, $(n\text{-}C_4H_9)_3PRu(p\text{-}Cumen)(CH_3\text{-}CN)_2(Tos)_2$, $(C_6H_{11})_3PRu(CH_3CN)Cl_2$, $(C_6H_{11})_3PRu(CH_3\text{-}CN)_2Cl_2$, $(C_6H_{11})_3PRu(p\text{-}Cumen)(C_2H_5OH)(BF_4)_2$, $(C_6H_{11})_3PRu(p\text{-}Cumen)(C_2H_5OH)_2(BF_4)_2$, $(C_6H_{11})_3PRu(p\text{-}Cumen)(C_2H_5OH)_2(PF_6)_2$, $(C_6H_{11})_3PRu\ (C_6H_6)(C_2H_5OH)_2(Tos)_2$, $(C_6H_{11})_3POs(p\text{-}Cumen)Cl_2$, $(i\text{-}C_3H_7)_3POs(p\text{-}Cumen)Cl_2$, $(CH_3)_3POS(p\text{-}Cumen)Cl_2$, $(C_6H_5)_3POs(p\text{-}Cumen)Cl_2$ und $RuCl_2(p\text{-}Cumen)[(C_6H_{11})_2PCH_2CH_2P(C_6H_{11})_2]$.

Die erfindungsgemäss zu verwendenden Ruthenium- und Osmiumverbindungen sind bekannt oder nach bekannten und analogen Verfahren ausgehend von den Metallhalogeniden (zum Beispiel $MeX_3$ oder $[MeArenX_2]_2$ und Reaktion mit Phosphinen und Ligandenbildnern herstellbar.

7. Weitere geeignete Einkomponenten-Katalysatoren sind zweiwertig-kationische Ruthenium- oder Osmiumverbindungen mit einem Metallatom, woran 1 bis 3 tertiäre Phosphinliganden mit im Fall der Rutheniumverbindungen sterisch anspruchsvollen Substituenten, gegebenenfalls nicht-photolabile Neutralliganden und Anionen zum Ladungsausgleich gebunden sind, handelt, mit der Massgabe, dass in Ruthenium(trisphenylphosphin)dihalogeniden oder -hydrid-halogeniden die Phenylgruppen mit $C_1$-$C_{18}$-Alkyl, $C_1$-$C_{18}$-Halogenalkyl oder $C_1$-$C_{18}$-Alkoxy substituiert sind.

Die Ruthenium- und Osmiumverbindungen enthalten bevorzugt 2 oder 3 tertiäre Phosphingruppen. Unter Phosphingruppen werden im Rahmen der Erfindung tertiäre Phosphine verstanden. Die Anzahl zusätzlicher nicht photolabiler Neutralliganden richtet sich zum einen nach der Anzahl der Phosphinliganden und zum anderen nach der Wertigkeit der Neutralliganden. Einbindige Neutralliganden sind bevorzugt.

In einer bevorzugten Ausführungsform enthalten die erfindungsgemäss zu verwendenden zweiwertig-kationischen Ruthenium- und Osmiumverbindungen 3 Phosphingruppen und zwei einwertige Anionen zum Ladungsausgleich; oder 3 Phosphingruppen, zwei einbindige oder einen zweibindigen nicht-photolabilen Neutralliganden und zwei einwertige Anionen zum Ladungsausgleich; oder 2 Phosphingruppen, einen monoanionischen, zusätzlich einbindigen nicht-photolabilen Neutralliganden und ein einwertiges Anion zum Ladungsausgleich.

Die Bedeutungen und Bevorzugungen von nicht-photolabilen Liganden (auch als stark koordinierende Liganden bezeichnet) sind zuvor genannt worden.

Unter sterisch anspruchsvollen Substituenten werden im Rahmen der Erfindung solche verstanden, die die Ruthenium- und Osmiumatome sterisch abschirmen. So wurde überraschend gefunden, dass lineare Alkylgruppen als Substituenten in den Phosphinliganden Rutheniumverbindungen ohne jede thermische Aktivität für die Metathesepolymerisation von gespannten Cycloolefinen ergeben. Es wurde auch beobachtet, dass bei Osmiumverbindungen überraschend lineare Alkylgruppen als Substituenten in den Phosphinliganden eine ausgezeichnete thermokatalytische Aktivität für die Metathesepolymerisation von gespannten Cycloolefinen besitzen; bevorzugt verwendet man aber auch für die Osmiumverbindungen Phosphinliganden mit sterisch anspruchsvollen Substituenten. Es wurde ferner gefunden, dass die sterische Abschirmung von Triphenylphosphinliganden bei Ruthenium-dihalogeniden und Ruthenium-hydrid-halogeniden ungenügend ist und solche Katalysatoren nur eine mässige katalytische Aktivität für die Metathesepolymerisation von gespannten Cycloolefinen besitzen. Die katalytische Aktivität kann überraschend erheblich gesteigert werden, wenn die tertiären Phosphingruppen mit Alkyl oder Alkoxygruppen substituiertes Phenyl entalten.

Die Bedeutungen und Bevorzugungen von Phosphinliganden sind zuvor genannt worden. Besonders bevorzugt handelt es sich bei $R_{91}$, $R_{92}$ und $R_{93}$ als Alkyl um $\alpha$-verzweigtes Alkyl, zum Beispiel der Formel $-CR_bR_cR_d$, worin $R_b$ H oder $C_1$-$C_{12}$-Alkyl, $R_c$ $C_1$-$C_{12}$-Alkyl, und $R_d$ $C_1$-$C_{12}$-Alkyl oder unsubstituiertes oder mit $C_1$-$C_4$-Alkyl oder $C_1$-$C_4$-Alkoxy substituiertes Phenyl darstellen, und die Summe der C-Atome im Rest $-CR_bR_cR_d$ 3 bis 18 beträgt.

Bei den verwendeten Osmiumverbindungen kann $R_{91}$, $R_{92}$ und $R_{93}$ auch lineares Alkyl mit 1 bis 18, bevorzugt 1 bis 12, bevorzugter 1 bis 8, und besonders bevorzugt 1 bis 6 C-Atomen darstellen, zum Beispiel Methyl, Ethyl, n-Propyl, n-Butyl, n-Pentyl, n-Hexyl, n-Heptyl und n-Octyl.

In einer bevorzugten Ausführungsform entsprechen die Phosphinliganden der Formel XXIII, worin $R_{91}$, $R_{92}$ und $R_{93}$ unabhängig voneinander $\alpha$-verzweigtes $C_3$-$C_8$-Alkyl, unsubstituiertes oder mit $C_1$-$C_4$-Alkyl substituiertes Cyclopentyl oder Cyclohexyl, oder unsubstituiertes oder mit $C_1$-$C_4$-Akyl, $C_1$-$C_4$-Alkyl $C_1$-$C_4$-Alkoxy oder Trifluormethyl substituiertes Phenyl darstellen. Besonders bevorzugte Beispiele für Phosphinliganden der Formel XXIII sind $(C_6H_5)_3P$, $(C_5H_9)_3P$, $(C_6H_{11})_3P$, $(i\text{-}C_3H_7)_3P$, $(i\text{-}C_4H_9)_3P$, $(t\text{-}C_4H_9)_3P$, $[C_2H_5\text{-}CH(CH_3)]_3P$, $[C_2H_5\text{-}C(CH_3)_2]_3P$, (2-Methylphenyl)$_3$P, (2,3-Dimethylphenyl)$_3$P, (2,4-Dimethylphenyl)$_3$P, (2,6-Dimethylphenyl)$_3$P, (2-Methyl-4-i-propylphenyl)$_3$P, (2-Methyl-3-i-

propylphenyl)$_3$P, (2-Methyl-5-i-propylphenyl)$_3$P, (2,4-Di-t-butylphenyl)$_3$P, (2-Methyl-6-i-propylphenyl)$_3$P, (2-Methyl-3-t-butylphenyl)$_3$P, (2,5-Di-t-butylphenyl)$_3$P, (2-Methyl-4-t-butylphenyl)$_3$P, (2-Methyl-5-i-butylphenyl)$_3$P, (2,3-Di-t-butylphenyl)$_3$P und (2,6-Di-t-butylphenyl)$_3$P.

Beispiele und Bevorzugungen für geeignete Anionen sind zuvor genannt worden.

In einer bevorzugten Ausführungsform entsprechen die Ruthenium- und Osmiumverbindungen besonders bevorzugt den Formeln XXVI, XXVIa, XXVIb, XXVIc oder XXVId

$$Me^{2\oplus}(L_{11})_2(L_{12})(Y_1^{\ominus})_2 \qquad (XXVI),$$

$$Me^{2\oplus}(L_{11})_3(Y_1^{\ominus})_2 \qquad (XXVIa),$$

$$Me^{2\oplus}(L_{11})_2L_{13}(Y_1^{\ominus}) \qquad (XXVIb),$$

$$Me^{2\oplus}(L_{11})_3L_{14}(Y_1^{\ominus})_2 \qquad (XXVIc),$$

$$Me^{2\oplus}L_1(L_2)_3(Y_1^{\ominus})_2 \qquad (XXVId),$$

worin

Me für Ru oder Os steht;
$Y_1$ das Anion einer einbasigen Säure bedeutet;
$L_{11}$ ein Phosphin der Formel XXIII oder XXma darstellt,
$L_{12}$ einen Neutralliganden bedeutet;
$L_{13}$ unsubstituiertes oder mit $C_1$-$C_4$-Alkyl substituiertes Cyclopentadienyl darstellt; und
$L_{14}$ für CO steht.

Für die einzelnen Bedeutungen von $L_{11}$, $L_{12}$, $L_{13}$ und $Y_1$ gelten die voranstehenden Bevorzugungen.

In einer besonders bevorzugten Ausführungsform stehen in Formel XXVI $L_{12}$ für ein $C_1$-$C_4$-Alkanol, in Formel XXVIb $Y_1$ für Cl oder Br, in Formel XXVIc $Y_1$ für H, und in den Formeln XXVI bis XXVIc $L_{11}$ für Tri-i-propylphosphin, Tricyclohexylphosphin, Triphenylphosphin oder in den Phenylgruppen mit 1 bis 3 $C_1$-$C_4$-Alkyl substituiertes Triphenylphosphin.

Die erfindungsgemäss zu verwendenden Ruthenium- und Osmiumverbindungen sind bekannt oder nach bekannten und analogen Verfahren ausgehend von den Metallhalogeniden (zum Beispiel $MeX_3$, $[Me(Diolefin)X_2]_2$ oder $[MeArenX_2]_2$ und Reaktion mit Phosphinen und Ligandenbildnern herstellbar.

Die erfindungsgemässe Zusammensetzung kann zusätzlich weitere offenkettige, gespannte cyclische und/oder gespannte polycyclische kondensierte und Metathesepolymerisate bildende Olefine enthalten, wobei diese Olefine bevorzugt noch weitere Doppelbindungen enthalten und zur Bildung von vernetzten Polymerisaten beitragen. Bei den cyclischen Olefinen kann es sich um monocyclische oder polycyclische kondensierte Ringsysteme, zum Beispiel mit zwei bis vier Ringen, handeln, die unsubstituiert oder substituiert sind und Heteroatome wie zum Beispiel O, S, N oder Si in einem oder mehreren Ringen und/oder kondensierte aromatische oder heteroaromatische Ringe wie zum Beispiel o-Phenylen, o-Naphthylen, o-Pyridinylen oder o-Pyrimidinylen enthalten können. Die einzelnen cyclischen Ringe können 3 bis 16, bevorzugt 3 bis 12 und besonders bevorzugt 3 bis 8 Ringglieder enthalten. Die cyclischen Olefine können weitere nichtaromatische Doppelbindungen enthalten, je nach Ringgrösse bevorzugt 2 bis 4 solcher zusätzlichen Doppelbindungen. Bei den Ringsubstituenten handelt es sich um solche, die inert sind, das heisst, die die chemische Stabilität der Einkomponenten-Katalysatoren nicht beeinträchtigen. Solche Olefine und Cycloolefine sind in grosser Zahl bekannt und in einfacher Weise durch Diels-Alder Reaktionen von Cyclodienen und Cycloolefinen oder polycyclichen oder polycyclischen kondensierten Olefinen erhältlich. Diese Cycloolefine können zum Beispiel der Formel IIa entsprechen

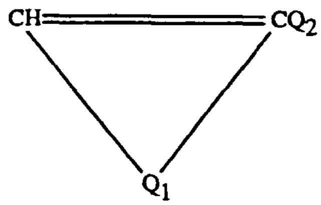

(IIa),

worin $Q_1$ und $Q_2$ die für Reste der Formel II angegebenen Bedeutungen haben, einschliesslich der Bevorzugungen.

Einige bevorzugte Beispiele für Verbindungen der Formel IIa sind Norbornen und Norbornenderivate. Spezifische Beispiele sind:

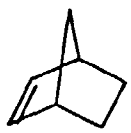

(25),

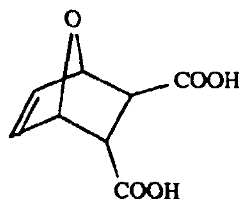

(26),

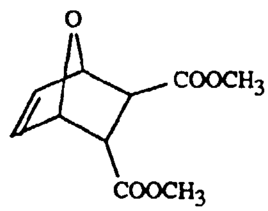

(27),

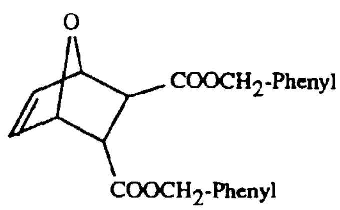

(28),

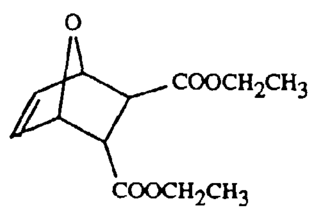

(29),

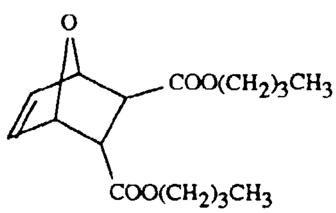

(30),

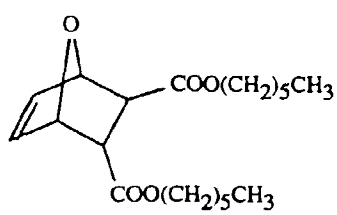

(31),

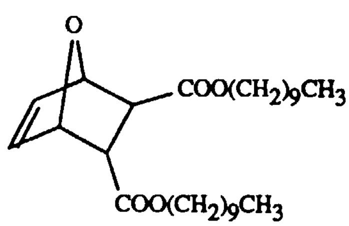

(32),

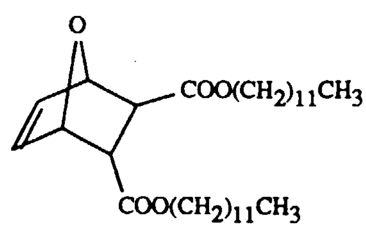

(33),

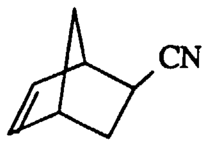 (34),

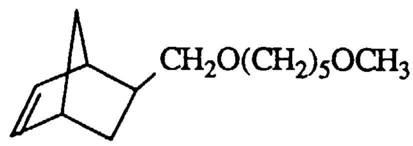 (35),

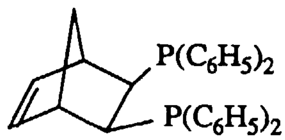 (36),

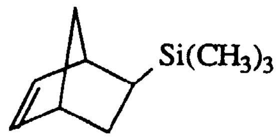 (37),

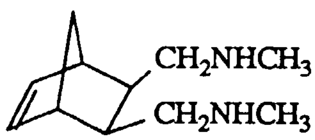 (38),

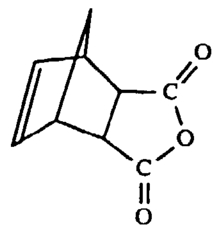 (39),

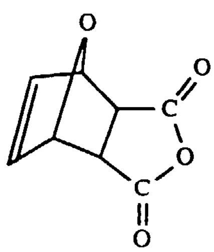 (40),

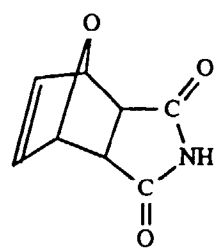 (41),

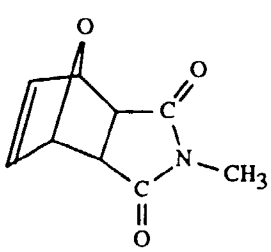 (42),

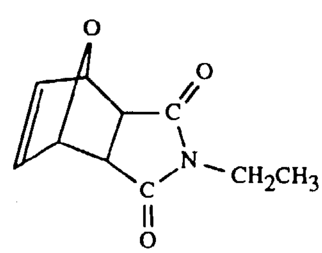 (43),

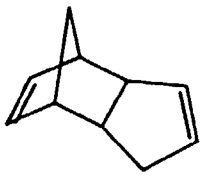 (44),

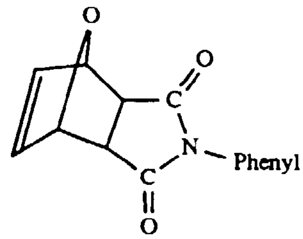 (45),

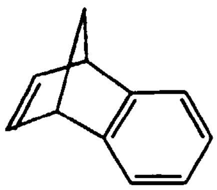 (46),

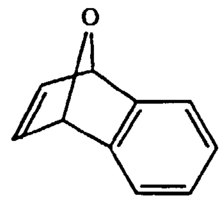 (47),

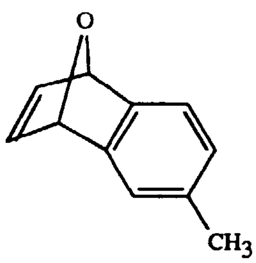 (48),

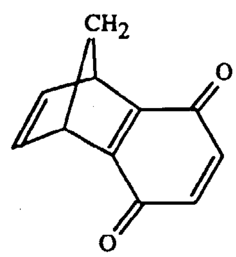 (49),

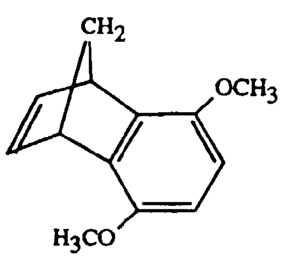 (50),

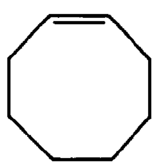 (51),

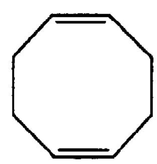 (52),

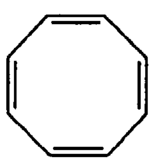 (53),

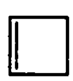 (54),

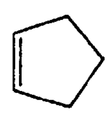 (55),

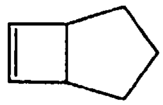 (56), 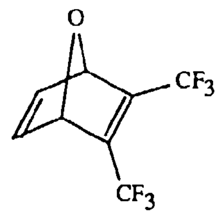 (57), 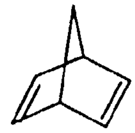 (58),

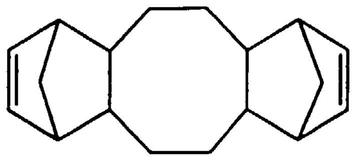 (59), 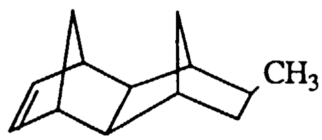 (60),

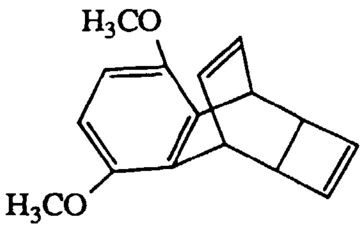 (61), 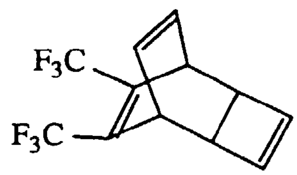 (62),

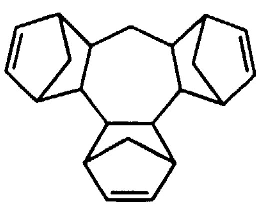 (63), 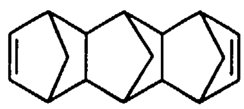 (64),

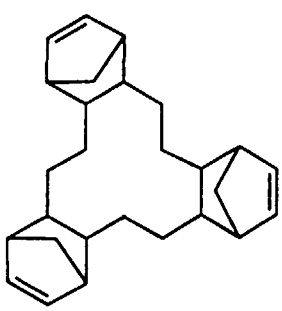 (65), 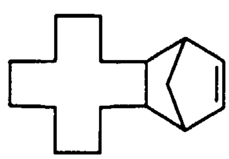 (66),

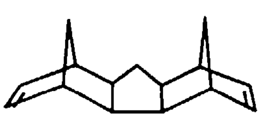 (67), 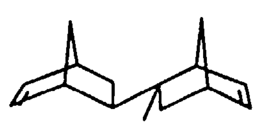 (68)

und

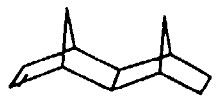

(69).

Die erfindungsgemässe Zusammensetzung kann zusätzlich weitere nichtflüchtige offenkettige Comonomere enthalten, die mit den gespannten Cycloolefinen Copolymere bilden. Bei Mitverwendung von zum Beispiel Dienen können sich vernetzte Polymerisate bilden. Einige Beispiele für solche Comonomere sind olefinisch mono- oder di-ungesättigte Verbindungen wie Olefine und Diene aus der Gruppe Penten, Hexen, Hepten, Octen, Decen, Dodecylen, Acryl- und Methacrylsäure, deren Ester und Amide, Vinylether, Styrol, Butadien, Isopren und Chlorbutadien.

Die weiteren zur Metathesepolymerisation fähigen Olefine sind in der erfindungsgemässen Zusammensetzung zum Beispiel in einer Menge von bis zu 80 Gew. %, bevorzugt 0,1 bis 80 Gew. %, bevorzugter 0,5 bis 60 Gew. % und besonders bevorzugt 5 bis 40 Gew. % enthalten, bezogen auf die Gesamtmenge an Verbindungen der Formel I und weiterer zur Metathesepolymerisation fähiger Olefine.

Katalytische Mengen bedeutet im Rahmen der vorliegenden Erfindung bevorzugt eine Menge von 0,001 bis 20 Mol-%, bevorzugter 0,01 bis 15 Mol-%, besonders bevorzugt 0,01 bis 10 Mol-%, und ganz besonders bevorzugt 0,01 bis 5 Mol-%, bezogen auf die Menge des Monomers. Auf Grund der hohen photokatalytischen Aktivität bei Phosphingruppen enthaltenden Ruthenium- und Osmiumkatalysatoren sind in diesem Fall Mengen von 0,001 bis 2 Gew.-% ganz besonders bevorzugt.

Die erfindungsgemässe Zusammensetzung kann Lösungsmittel enthalten, besonders wenn sie zur Herstellung von Beschichtungen verwendet werden.

Geeignete inerte Lösungsmittel sind zum Beispiel protisch-polare und aprotische Lösungsmittel, die allein oder in Mischungen aus mindestens zwei Lösungsmitteln verwendet werden können. Beispiele sind: Ether (Dibutylether, Tetrahydrofuran, Dioxan, Ethylenglykolmonomethyl- oder -dimethylether, Ethylenglykolmonoethyl- oder -diethylether, Diethylenglykoldiethylether, Triethylenglykoldimethylether), halogenierte Kohlenwasserstoffe (Methylenchlorid, Chloroform, 1,2-Dichlorethan, 1,1,1-Trichlorethan, 1,1,2,2-Tetrachlorethan), Carbonsäureester und Lactone (Essigsäureethylester, Propionsäuremethylester, Benzoesäureethylester, 2-Methoxyethylacetat, γ-Butyrolacton, δ-Valerolacton, Pivalolacton), Carbonsäureamide und Lactame (N,N-Dimethylformamid, N,N-Diethylformamid, N,N-Dimethylacetamid, Tetramethylharnstoff, Hexamethylphosphorsäuretriamid, γ-Butyrolactam, ε-Caprolactam, N-Methylpyrrolidon, N-Acetylpyrrolidon, N-Methylcaprolactam), Sulfoxide (Dimethylsulfoxid), Sulfone (Dimethylsulfon, Diethylsulfon, Trimethylensulfon, Tetramethylensulfon), tertiäre Amine (N-Methylpiperidin, N-Methylmorpholin), aliphatische und aromatische Kohlenwasserstoffe wie zum Beispiel Petrolether, Pentan, Hexan, Cyclohexan, Methylcyclohexan, Benzol oder substituierte Benzole (Chlorbenzol, o-Dichlorbenzol, 1,2,4-Trichlorbenzol, Nitrobenzol, Toluol, Xylol) und Nitrile (Acetonitril, Propionitril, Benzonitril, Phenylacetonitril). Bevorzugte Lösungsmittel sind aprotische polare und unpolare Lösungsmittel.

Die Wahl der Lösungsmittel richtet sich hauptsächlich nach den Eigenschaften der Einkomponenten-Katalysatoren, die durch die verwendeten Lösungsmittel nicht desaktiviert werden dürfen. Ruthenium- und Osmiumkatalysatoren können zusammen mit polaren protischen Lösungsmitteln wie zum Beispiel Wasser oder Alkanolen verwendet werden. Diese Katalysatoren sind auch luft-, sauerstoff- und feuchtigkeitsunempfindlich, und entsprechende vernetzbare Zusammensetzungen können ohne besondere Schutzvorkehrungen verarbeitet werden. Bei den anderen Einkomponenten-Katalysatoren empfiehlt sich der Ausschluss von Sauerstoff und Feuchtigkeit. Die Zusammensetzungen sind lagerstabil, wobei wegen der Lichtempfindlichkeit eine Lagerung im Dunkeln zu empfehlen ist.

Die erfindungsgemässe Zusammensetzung kann Formulierungshilfstoffe enthalten. Bekannte Hilfsstoffe sind Antistatika, Antioxidantien, Lichtschutzmittel, Weichmacher, Farbstoffe, Pigmente, Füllstoffe, Verstärkerfüllstoffe, Gleitmittel, Haftvermittler, viskositätserhöhende Mittel und Entformungshilfsmittel. Die Füllstoffe können in überraschend hohen Anteilen zugegen sein, ohne die Polymerisation nachteilig zu beeinflussen, zum Beispiel in Mengen von bis zu 70 Gew.-%, bevorzugt 1 bis 70 Gew.%, bevorzugter 5 bis 60 Gew.-%, besonders bevorzugt 10 bis 50 Gew.-% und insbesondere bevorzugt 10 bis 40 Gew.-%, bezogen auf die Zusammensetzung. Füllstoffe und Verstärkerfüllstoffe zur Verbesserung der optischen, physikalischen, mechanischen und elektrischen Eigenschaften sind in grosser Vielzahl bekannt geworden. Einige Beispiele sind Glas und Quarz in Form von Pulvern, Kugeln und Fasern, Metall- und Halbmetalloxide, Carbonate wie $MgCO_3$, $CaCO_3$, Dolomit, Metallsulfate wie Gips und Schwerspat, natürliche und synthetische Silikate wie Talk, Zeolithe, Wollastonit, Feldspate, Tonerden wie Chinaclay, Gesteinsmehle, Whisker, Carbonfasern, Kunststofffasern oder -pulver und Russ. Viskositätserhöhende Mittel sind insbesondere Metathesepolymerisate, die olefinisch ungesättigte Gruppen aufweisen und bei der Polymerisation in das Polymer eingebaut werden können. Solche Metathesepolymerisate sind bekannt und zum Beispiel unter dem Handelsnamen Vestenamere® käuflich. Andere viskositätserhöhende Mittel sind Polybutadien, Polyisopren oder Polychlorbutadien, sowie Copolymere von Butadien, Isopren und Chloropren mit Olefinen. Die viskositätserhöhenden Mittel können in einer Menge von 0,1 bis 50, bevorzugt 1 bis 30, und besonders bevorzugt 1 bis 20 Gew.-% enthalten sein bezogen auf die Zusammensetzung. Bei der Verwendung von Füllstoffen ist es zweckmässig, die optische Transparenz für die Polymerisation zu erhalten oder die Polymerisation in dünnen Schichten durchzuführen.

Ein weiterer Gegenstand der Erfindung ist ein Verfahren zur Herstellung vernetzter Polymerisate durch Metathesepolymerisation, das dadurch gekennzeichnet ist, dass man eine Zusammensetzung aus

(a) mindestens einer Verbindung der Formel I

$$(A)_n\text{-}B \qquad (I),$$

worin A den Rest eines gespannten Cycloolefins bedeutet, B für eine direkte Bindung oder eine n-wertige Brükkengruppe steht, und n eine ganze Zahl von 2 bis 8 darstellt, und

(b) einer katalytischen Menge mindestens eines thermo- oder strahlungsaktivierbaren Einkomponenten-Katalysators für eine Metathesepolymerisation,

mit Ausnahme von Norbornencarbonsäure(norbomenmethyl)ester der Formel

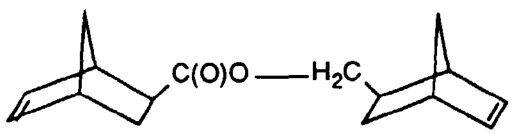

in Kombination mit einer katalytischen Menge mindestens einer thermostabilen Molybdän(VI)- oder Wolfram(VI) verbindung, die mindestens zwei Methylgruppen oder zwei monosubstituierte Methylgruppen am Metall gebunden enthält, wobei der Substituent kein Wasserstoffatom in α-Stellung enthält,

(c) durch Erwärmen polymerisiert,

(d) durch Bestrahlung polymerisiert,

(e) durch Erwärmung und Bestrahlung polymerisiert,

(f) durch kurzzeitige Erwärmung den Einkomponenten-Katalysator aktiviert und die Polymerisation durch Bestrahlung beendet, oder

(g) durch kurzzeitige Bestrahlung den Einkomponenten-Katalysator aktiviert und die Polymerisation durch Erwärmen beendet.

Erwärmen kann eine Temperatur von 50 bis 300°C, bevorzugt 60 bis 250°C, besonders bevorzugt 60 bis 200°C, und insbesondere bevorzugt 60 bis 150°C bedeuten. Die Polymerisationszeiten hängen im wesentlichen von der Katalysatoraktivität ab und die Zeiten können von Minuten bis zu meheren Stunden reichen.

Es ist beim erfindungsgemässen Verfahren nicht notwendig, die Bestrahlung der Reaktionsmischung über die gesamte Reaktionsdauer aufrechtzuerhalten. Ist die Polymerisation einmal photochemisch initiiert, erfolgt der weitere Reaktionsverlauf selbst im Dunkeln selbstständig. Vorteilhafterweise wird die Bestrahlung mit Licht der Wellenlänge im Bereich von 50 nm bis 1000 nm, bevorzugt im Bereich von 200 nm bis 500 nm und ganz besonders bevorzugt im UV-Bereich durchgeführt. Die Bestrahlungsdauer ist von der Art der Lichtquelle abhängig. Als Bestrahlungsquellen eignen sich zum Beispiel die Sonne, Laser-, Röntgen- und besonders UV-Strahlungsquellen. Vorzugsweise werden erfindungsgemäß UV-Laser oder UV-Lampen eingesetzt. Die Bestrahlung des Katalysators kann sowohl vor, während, als auch nach der Monomerenzugabe erfolgen.

Geeignete Bestrahlungszeiten sind von einer Sekunden bis zu mehreren Stunden, insbesondere Minuten bis zu Stunden. Die Reihenfolge der Zugabe von Monomeren und Katalysator ist unkritisch. Das Monomer kann sowohl vorgelegt als auch nach Einbringen des Katalysators zugegeben werden. Ebenso kann der Katalysator vorbestrahlt und anschliessend das Monomer zugegeben werden. Ferner kann auch die Katalysator und Monomer enthaltende Lösung bestrahlt werden.

Das erfindungsgemäße Verfahren wird bei Bestrahlung bevorzugt bei Raumtemperatur bis leicht erhöhter Temperatur durchgeführt. Eine Temperaturerhöhung dient der Erhöhung der Reaktionsgeschwindigkeit. Die verwendeten Katalysatoren initiieren nur in Ausnahmefällen bei den gewählten Temperaturen für sich eine thermische Metathesepolymerisation unter Vernetzung. Bei den zur Reaktionsbeschleunigung gewählten Temperaturen findet daher überwiegend eine Photopolymerisation statt. Es ist aber zu erwähnen, dass die Katalysatoren durch ausreichende Bestrahlung in thermoaktive Katalysatoren umgewandelt werden können.

Insbesondere wird das erfindungsgemäße Verfahren unter Bestrahlung bevorzugt bei Temperaturen von -20 bis +110 °C, besonders bevorzugt 20 bis 80 °C durchgeführt.

Die Bestrahlungsdauer hängt im wesentlichen von der gewünschten Reaktionsführung ab. Eine kurzzeitige Bestrahlung wird zum Beispiel dann gewählt, wenn man die Polymerisation nur durch Bestrahlung initiieren und durch Erhitzen beenden will. Kurzzeitig kann eine Bestrahlungszeit bis zu 60 Sekunden, bevorzugt 5 bis 60 Sekunden und besonders bevorzugt 10 bis 40 Sekunden bedeuten. Eine längere Bestrahlungszeit wird zum Beispiel dann gewählt, wenn man die Polymerisation überwiegend unter Bestrahlung durchführen und die endgültige Polymerisation nur durch Nachtempern beenden will.

Ein ganz besonderer und überraschender Vorteil des erfindungsgemässen Verfahrens besteht darin, dass die verwendeten Einkomponenten-Katalysatoren nach der Bestrahlung als thermische Katalysatoren wirken. Daraus ergibt sich die Möglichkeit, die Polymerisation nach einer kurzen Bestrahlungszeit durch Wärmezufuhr fortzusetzen und zu beenden, was in verschiedenen Bereichen der Herstellung von Formkörpern oder Beschichtungen wirtschaftliche und technische Vorteile bietet.

Ein weiterer Gegenstand der vorliegenden Erfindung sind vernetzte Metathesepolymerisate aus mindestens einer Verbindung der Formel I

$$(A)_n\text{-}B \qquad (I),$$

worin A den Rest eines gespannten Cycloolefins bedeutet, B für eine direkte Bindung oder eine n-wertige Brückengruppe steht, und n eine ganze Zahl von 2 bis 8 darstellt, und gegebenenfalls weiteren zur Metathesepolymerisation befähigten Monomeren, mit Ausnahme von Norbornencarbonsäure-norbornenmethylester.

Mit dem erfindungsgemässen Verfahren können Werkstoffe zur spanabhebenden Herstellung von Formkörpern oder direkt Formkörper aller Art, sowie Beschichtungen und Reliefabbildungen hergestellt werden.

Je nach verwendetem Monomer können die erfindungsgemäßen Polymere sehr verschiedene Eigenschaften aufweisen. Einige zeichnen sich durch sehr hohe Sauerstoffpermeabilität, tiefe Dielektrizitätskonstanten, gute Wärmestabilität und geringe Wasserabsorption aus. Andere haben hervorragende optische Eigenschaften wie zum Beispiel hohe Transparenz und niedrige Brechungsindices. Ferner ist insbesondere der geringe Schrumpf hervorzuheben. Daher können sie in sehr unterschiedlichen technischen Gebieten Verwendung finden.

Die erfindungsgemäßen Zusammensetzungen zeichnen sich als Schichten auf den Oberflächen von Trägermaterialien durch eine hohe Haftfestigkeit aus. Ferner zeichnen sich die beschichteten Materialien durch eine sehr hohe Oberflächenglätte und -glanz aus. Unter den guten mechanischen Eigenschaften ist insbesondere der geringe Schrumpf und die hohe Schlagzähigkeit hervorzuheben, aber auch die thermische Beständigkeit. Ferner ist die leichte Entformbarkeit bei der Verarbeitung in Formen und die hohe Lösungsmittelbeständigkeit zu erwähnen.

Diese Polymeren eignen sich zur Herstellung von medizinischen Geräten, Implantaten oder Kontaktlinsen; zur Herstellung von elektronischen Bauteilen; als Bindemittel für Lacke; als photohärtbare Zusammensetzungen für den Modellbau oder als Klebstoffe zum Verkleben von Substraten mit niedrigen Oberflächenenergien (zum Beispiel Teflon, Polyethylen und Polypropylen), sowie als photopolymerisierbare Zusammensetzung in der Stereolithographie. Die erfindungsgemäßen Zusammensetzungen können auch zur Herstellung von Lacken durch Photopolymerisation verwendet werden, wobei einerseits klare (transparente) und sogar pigmentierte Zusammensetzungen verwendet werden können. Es können sowohl Weiss- als auch Buntpigmente verwendet werden.

Die erfindungsgemäßen Zusammensetzungen eignen sich besonders zur Herstellung von Schutzschichten und Reliefabbildungen. Ein weiterer Gegenstand der Erfindung ist eine Variante des erfindungsgemäßen Verfahrens zur Herstellung von beschichteten Materialien oder Reliefabbildungen auf Trägermaterialien, bei dem man eine erfindungsgemässe Zusammensetzung und gegebenenfalls Lösungsmittel als Schicht auf einem Träger aufbringt, zum Beispiel durch Tauch-, Streich-, Giess-, Walz-, Rakel- oder Schleudergießverfahren, gegebenenfalls das Lösungsmittel entfernt, und die Schicht zur Polymerisation bestrahlt oder erwärmt, oder die Schicht durch eine Photomaske bestrahlt und anschließend die nichtbestrahlten Anteile mit einem Lösungsmittel entfernt. Danach kann sich noch eine Temperung anschliessen. Mit diesem Verfahren können Oberflächen von Substraten modifiziert oder geschützt werden, oder es können zum Beispiel gedruckte Schaltungen, Druckplatten oder Druckwalzen hergestellt werden. Bei der Herstellung von gedruckten Schaltungen können die erfindungsgemäßen Zusammensetzungen auch als Lötstopplacke eingesetzt werden. Weitere Anwendungsmöglichkeiten sind die Herstellung von Siebdruckmasken, die Verwendung als strahlungshärtbare Druckfarben für den Offset-, Sieb- und Flexodruck.

Gegenstand der vorliegenden Erfindung ist ferner ein beschichtetes Trägermaterial, das dadurch gekennzeichnet ist, daß auf einem Substrat eine Schicht aus einer erfindungsgemässen Zusammensetzung aufgebracht ist.

Gegenstand der vorliegenden Erfindung ist ebenfalls ein beschichtetes Substrat mit einer gehärteten Schicht aus einer erfindungsgemässen Zusammensetzung. Die ausserordentlich hohe Haftfestigkeit der Schichten sogar auf Metalloberflächen ist besonders hervorzuheben, selbst wenn es sich um reine Kohlenwasserstoffpolymerisate handelt.

Geeignete Trägermaterialien sind beispielsweise solche aus Glas, Mineralien, Keramiken, Kunststoffen, Holz, Halbmetallen, Metallen, Metalloxiden und Metalinitriden. Die Schichtdicken richten sich im wesentlichen nach der gewünschten Verwendung und können z.B. 0,1 bis 1000 µm, bevorzugt 0,5 bis 500 µm, besonders bevorzugt 1 bis 100 µm betragen. Die beschichteten Materialien zeichnen sich durch eine hohe Haftfestigkeit und gute thermische und mechanische Eigenschaften aus.

Die Herstellung der erfindungsgemässen beschichteten Materialen kann nach bekannten Methoden wie zum Beispiel Streichen, Rakeln, Giessverfahren wie Vorhanggiessen oder Schleudergiessen erfolgen.

Die erfindungsgemässen Zusammensetzungen eignen sich auch zur Herstellung von gummiartigen oder thermoplastischen Polymerisaten, die noch weiter vernetzt werden können, wenn sie reaktive Gruppen wie zum Beispiel (Meth)Acrylat- oder Epoxidgruppen enthalten.

Die erfindungsgemässen Zusammensetzungen können auch als thermisch oder mittels Strahlung härtbare Klebstoffe zur festen Verbindung von unterschiedlichsten Materialien verwendet werden, wobei hervorragende Schälfestigkeiten erzielt werden können.

Die erfindungsgemässen Polymerisate zeichnen sich neben den hohen Haftfestigkeiten, der hervorragenden Verarbeitbarkeit, den guten Oberflächeneigenschaften (Glätte, Glanz), der hohen Vernetzungsdichte und der Beständigkeit gegen Lösungsmittel und andere Flüssigkeiten besonders noch durch sehr gute physikalisch-mechanische Eigenschaften wie zum Beispiel hohe Temperaturbeständigkeit, Bruch- und Biegefestigkeit und Schlagzähigkeit und hervorragende elektrische Eigenschaften wie zum Beispiel niedrige Oberflächenspannungen und -ladungen (sehr niedrige ε- und tan δ- Werte) aus. Ferner sind die hohe Sauerstoffpermeabilität und die geringe Wasseraufnahme zu erwähnen. Nur aus Kohlenstoff und Wasserstoff aufgebaute Polymere sind ökologisch besonders wertvoll, da sie zum Beispiel durch Pyrrolyse vollständig recyclisiert werden können.

Die nachfolgenden Beispiele erläutern die Erfindung näher.

A) Herstellung von Biscycloolefinen

Beispiel A1: Herstellung von Verbindung Nr. 0 (siehe EP 287,762)

70 g (0,86 mol) 1,5-Hexadien und 56 g (0,42 mol) Dicyclopentadien werden in einem Autoklaven gemischt und 8 h auf 190°C erhitzt Nach dem Abkühlen wird die Mischung im Vakuum destilliert. Bei 80 bis 110°C und 0,28 bis 0,30 mbar erhält man 32,6 g (36%) Produkt als farblose Flüssigkeit; $n_D^{20}$ = 1,525.

| | | | |
|---|---|---|---|
| Elementaranalyse | berechnet | C 89,65; | H 10,35; |
| | gefunden | C 89,72; | H 10,13. |

Beispiel A2: Herstellung von Verbindung Nr. 2

35 g (0,32 mol) 1,7-Octadien und 28 g (0,21 mol) Dicyclopentadien werden in einem Autoklaven gemischt und 8 h auf 190°C erhitzt Nach dem Abkühlen wird die Mischung im Vakuum destilliert. Bei 100°C und 0,21 mbar erhält man 6,7 g (13%) Produkt als farblose Flüssigkeit; $n_D^{20}$ = 1,516.

| | | | |
|---|---|---|---|
| Elementaranalyse | berechnet | C 89,19; | H 10,81; |
| | gefunden | C 89,50; | H 10,60. |

Beispiel A3: Herstellung von Verbindung Nr. 3

34,6 g (0,25 mol) 1,5-Decadien und 33,1 g (0,25 mol) Dicyclopentadien werden in einem Autoklaven gemischt und 8 h auf 190°C erhitzt Nach dem Abkühlen wird die Mischung im Vakuum destilliert. Bei 80 bis 100°C und 0,24 mbar erhält man 11,4 g (17%) Produkt als farblose Flüssigkeit; $n_D^{20}$ = 1,504, die bei Raumtemperatur fest wird.

| | | | |
|---|---|---|---|
| Elementaranalyse | berechnet | C 88,82; | H 11,18; |
| | gefunden | C 88,62; | H 11,18. |

Beispiel A4: Herstellung von Verbindung Nr. 20

99,7 g (0,40 mol) Cyanursäuretriallylester und 79,3 g (0,6 mol) Dicyclopentadien werden in einem Autoklaven gemischt und 8 h auf 190°C erhitzt. Nach dem Abkühlen erhält man 160 g (89,4%) eines bräunlichen harzartigen Feststoffs mit einem Schmelzpunkt von von 50°C, der in Toluol und Chloroform löslich ist.

| | | | | |
|---|---|---|---|---|
| Elementaranalyse | berechnet | C 72,46; | H 7,43; | N 9,39. |
| | gefunden | C 72,21; | H 7,52; | N 9,32. |

A') Herstellung von Comonomeren

Beispiel A'1: Herstellung von Verbindung Nr. 59

100 g (0,92 mol) 1,5-Cyclooctadien und 200 g (1,51 mol) Dicyclopentadien werden zusammen mit 0,4 g Hydrchinonmonomethylether in einem Autoklaven gemischt und 3 h auf 190°C erhitzt. Nach dem Abkühlen wird die Mischung im Vakuum destilliert. Bei 110°C und 4,6 mbar erhält man 73,5 g (33%) Produkt als farblose Flüssigkeit; $n_D^{20}$ = 1,534. MS: $M^+$ = 240.

| | | | |
|---|---|---|---|
| Elementaranalyse | berechnet | C 89,92; | H 10,08; |
| | gefunden | C 90,11; | H 9,04. |

Beispiel A'2: Herstellung von Verbindung Nr. 65

64,9 g (0,40 mol) 1,5,9-Cyclododecatrien und 79,3 g (0,60 mol) Dicyclopentadien werden in einem Autoklaven gemischt und 8 h auf 190°C erhitzt. Nach dem Abkühlen wird die Mischung im Vakuum destilliert. Bei 50 bis 60°C und 0,04 mbar erhält man 22,6 g (16%) Produkt als farblose Flüssigkeit; $n_D^{20}$ = 1,541. MS: $M^+$ = 360.

| | | | |
|---|---|---|---|
| Elementaranalyse | berechnet | C 89,94; | H 10,06; |
| | gefunden | C 89,96; | H 9,90. |

Beispiel A'3: Herstellung von Verbindung Nr. 63

92,1 g (1,00 mol) Cycloheptatrien und 198,3 g (1,50 mol) Dicyclopentadien werden in einem Autoklaven gemischt und 8 h auf 190°C erhitzt. Nach dem Abkühlen wird die Mischung im Vakuum destilliert. Bei 75°C und 0,12 mbar erhält man 80,6 g (28%) Produkt als farblose Flüssigkeit; $n_D^{20}$ = 1,542. MS: $M^+$ = 290.

| | | | |
|---|---|---|---|
| Elementaranalyse | berechnet | C 90,98; | H 9,02; |
| | gefunden | C 90,71; | H 9,31. |

Beispiel A'4: Herstellung von Verbindung Nr. 64

100 g (1,09 mol) Norbornadien und 50 g (0,38 mol) Dicyclopentadien werden zusammen mit 0,2 g Hydrochinonmonomethylether in einem Autoklaven gemischt und 3 h auf 190°C erhitzt. Nach dem Abkühlen wird die Mischung im Vakuum destilliert. Bei 35°C und 0,25 mbar erhält man 25,0 g (29%) Produkt als farblose Flüssigkeit; $n_D^{20}$ = 1,532. MS: $M^+$ = 224.

| | | | |
|---|---|---|---|
| Elementaranalyse | berechnet | C 91,01; | H 8,99; |
| | gefunden | C 90,94; | H 9,01. |

Beispiel A'5: Herstellung von Verbindung Nr. 66

80,2 g (0,50 mol) Cyclododecen und 33,05 g (0,25 mol) Dicyclopentadien werden in einem Autoklaven gemischt und 8 h auf 190°C erhitzt. Nach dem Abkühlen wird die Mischung im Vakuum destilliert. Bei 61°C und 0,12 mbar erhält man 8,10 g (7%) Produkt als farblose Flüssigkeit; $n_D^{20}$ = 1,528. MS: $M^+$ = 232.

| Elementaranalyse | berechnet | C 87,86; | H 12,14; |
|---|---|---|---|
| | gefunden | C 87,98; | H 11,72. |

Beispiel A'6: Herstellung von Verbindung Nr. 68

104,25 g (1,5 mol) Isopren (98 %), 208,7 g (1,5 mol) Dicyclopentadien (95 %) und 1,0 g tert-Butyl-brenzkatechin werden gemischt und im Autoklaven unter Rühren 8 h auf 200°C unter $N_2$ erhitzt. Nach dem Abkühlen destilliert man die halbfeste Masse im Hochvakuum. Siedepunkt: 60°C (0,07 mbar). Ausbeute: 202,4 g (67,4 %). $n_D^{20}$ = 1,53

| Elementaranalyse | berechnet | C 89,94; | H 10,06 |
|---|---|---|---|
| | gefunden | C 89,85; | H 10,08 |

Beispiel A'7: Herstellung von Verbindung Nr. 69

139,2 g (1,0 mol) Dicyclopentadien (95 %), 194,2 g (2,0 mol) 2-Norbornen (97 %) und 3,0 g tert-Butyl-brenzkatechin werden gemischt und im Autoklaven unter Rühren 2 h auf 230°C unter $N_2$ erhitzt. Nach dem Abkühlen destilliert man die halbfeste Masse im Hochvakuum. Siedepunkt: 65°C (0,065 mbar). Ausbeute: 69,0 g (21,5 %). $n_D^{20}$ = 1,54

| Elementaranalyse | berechnet | C 89,93; | H 10,07 |
|---|---|---|---|
| | gefunden | C 90,07; | H 9,88 |

B) Herstellung von vernetzten Polymerisaten.

Als Katalysatoren werden verwendet:

A) $W(=NC_6H_5)[OC(CH_3)_3][CH_2Si((CH_3)_3]_2Cl$
B) $W(=NC_6H_5)[OCCH_3(CF_3)_2][CH_2Si((CH_3)_3]_2$
C) $RuCl_2$(p-Cumen)$P(C_6H_{11})_3$

Beispiel B1:

Die Verbindung gemäss Beispiel A1 wird mit 0,7 Gew.-% des Katalysators A vermischt und die Mischung in eine Glasform gegossen. Dann wird 30 Minuten bei Raumtemperatur in einem UV-Ofen bestrahlt (4 Röhren mit 100 W Leistung), und dann 1 h bei 80°C thermisch polymerisiert. Man erhält eine formstabile Platte, $T_g$ 75°C (bestimmt mittels Differential Scanning Calometrie). Das Polymer quillt in Toluol, ohne sich zu lösen. Der niedrige Quellungsgrad von 44% weist auf eine hohe Vernetzungsdichte hin.

Beispiel B2:

Die Mischung gemäss Beispiel B1 wird nur mit einer 200 W Quecksilbermitteldruckdampflampe bestrahlt. Man erhält eine formstabile Platte, $T_g$ 60°C. Der Quellungsgrad in Toluol beträgt 54%.

Beispiel B3:

Es wird wie in Beispiel B1 verfahren, aber mit 1 Gew.-% Katalysator und einer zusätzlichen thermischen Nachhärtung bei 100°C während 30 Minuten. Man erhält eine formstabile Platte mit einer Dichte von 1,06 g/cm$^3$, einer $T_g$ von 125°C und einem Elastizitätsmodul von 2210 N/mm$^2$. Die Shore D Härte beträgt 85 und der Quellungsgrad in Toluol ist 54%.

Beispiel B4:

Die Verbindung gemäss Beispiel A1 wird mit 1 Gew.-% Katalysator B vermischt und in eine Glasform gegossen. Man bestrahlt 2 h bei Raumtemperatur in einem UV-Ofen gemäss Beispiel B1, und polymerisiert dann 30 Minuten bei 80°C, 30 Minuten bei 100°C und 30 Minuten bei 120°C. Man erhält eine formstabile Platte mit einer Dichte von 1,06 g/cm$^3$, einer $T_g$ von 125°C und einem Elastizitätsmodul von 2390 N/mm$^2$. Die maximale Spannung beträgt 40,4

N/mm$^2$, die maximale Dehnung ist 2,0% und die Schlagzähigkeit (nach Charpy) beträgt 8,9 kJ/m$^2$. Der Quellungsgrad in Toluol beträgt 2000%.

Beispiele B5 bis B13:

Die gemäss Beispiel hergestellte Monomer wird mit 0,5 Gew.-% Katalysator C vermischt und in eine Glasform gegossen. Man härtet thermisch 1 h bei 60°C, 1 h bei 80°C, 1 h bei 100°C und 2 h bei 120°C. Die Nachhärtung erfolgt 2 h bei 150°C. Die folgende Tabelle zeigt die Resultate.

| Beispiel | Monomer | $T_g$ | Quellung | $T_g$* | Quellung* |
|---|---|---|---|---|---|
| B5 | A1 | 104 | 34 | 117 | 16 |
| B6 | A2 | 100 | 28 | 120 | 9 |
| B7 | A3 | 1 | 180 | 8 | 172 |
| B8 | A'1 | 122 | 85 | 143 | 76 |
| B9 | A'3 | - | 45 | 15 | 44 |
| B10 | A'4 | 111 | 55 | 118 | 52 |
| B11 | A'5 | 117 | 88 | 124 | 87 |
| B12 | A'6 | 22 | 53 | 29 | 53 |
| B13 | A'7 | 135 | 81 | 156 | 90 |

$T_g$: °C; Quellung: in Toluol;
*: nach Nachhärtung

## Patentansprüche

**1.** Verbindungen der Formel I

$$(A)_n\text{-}B \qquad (I),$$

worin A den Rest eines gespannten Cycloolefins, n die Zahl zwei und B die direkte Bindung oder eine Brückengruppe der Formel V

$$\text{-}X_5\text{-}R_{20}\text{-}X_6\text{-} \qquad (V)$$

bedeuten, worin

$X_5$ und $X_6$ unabhängig voneinander eine direkte Bindung, -O-, $-CH_2-O-$, -C(O)O-, -O(O)C-, $-CH_2-O(O)C-$, -C(O)-$NR_{21}$-, -$R_{21}$N-(O)C-, -NH-C(O)-$NR_{21}$-, -O-C(O)-NH-, $-CH_2$-O-C(O)-NH- oder -NH-C(O)-O- bedeuten, und

$R_{20}$ $C_2$-$C_{18}$-Alkylen, unsubstituiertes oder mit $C_1$-$C_4$-Alkyl oder $C_1$-$C_4$-Alkoxy substituiertes $C_5$-$C_8$-Cycloalkylen, unsubstituiertes oder mit $C_1$-$C_4$-Alkyl oder $C_1$-$C_4$-Alkoxy substituiertes $C_6$-$C_{18}$-Arylen oder $C_7$-$C_{19}$-Aralkylen, oder Polyoxaalkylen mit 2 bis 12 Oxaalkyleneinheiten und 2 bis 6 C-Atomen im Alkylen darstellt, und

$R_{21}$ H oder $C_1$-$C_6$-Alkyl ist,

mit Ausnahme von 1,2-Bisnorbornenyl-ethan,
Norbornencarbonsäure-norbornenmethylester und Verbindungen der Formel

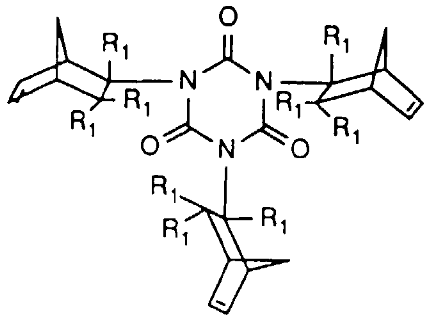

worin $R_1$ Wasserstoff oder Alkyl ist.

**2.** Verbindungen gemäss Anspruch 1, dadurch gekennzeichnet, dass in Formel V

a) $X_5$ und $X_6$ für eine direkte Bindung und $R_{20}$ für $C_2$-$C_{18}$-Alkylen oder
b) $X_5$ und $X_6$ für -O-, $-CH_2-O-$, -C(O)O-, -O(O)C-, $-CH_2-O(O)C-$, $-C(O)-NR_{21}-$, -O-C(O)-NH- oder $-CH_2-O-C(O)-NH-$ und $R_{20}$ für $C_2$-$C_{12}$-Alkylen, unsubstituiertes oder mit $C_1$-$C_4$-Alkyl oder $C_1$-$C_4$-Alkoxy substituiertes Phenylen, Naphthylen oder Benzylen, oder $-R_{22}-(O-R_{22}-)_x-OR_{22}-$ stehen, worin x eine Zahl von 2 bis 4 ist und $R_{22}$ $-C_2$-$C_4$-Alkylen bedeutet.

**3.** Verbindungen gemäss Anspruch 1, dadurch gekennzeichnet, dass sie aus der folgenden Gruppe von Verbindungen ausgewählt sind

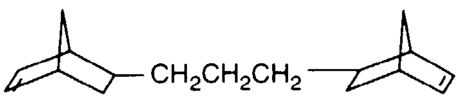

(1),

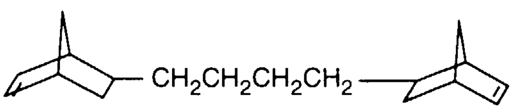

(2),

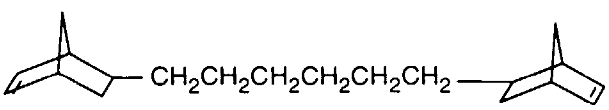

(3),

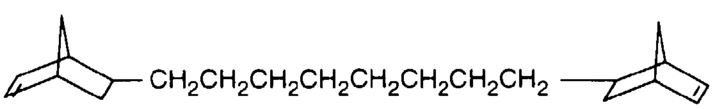

(4),

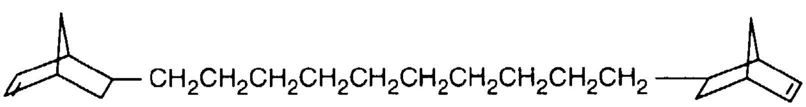

(5),

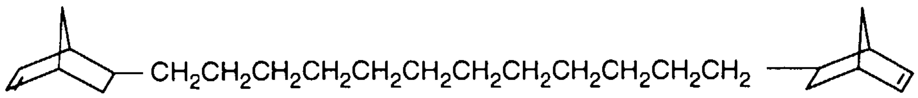

(6)

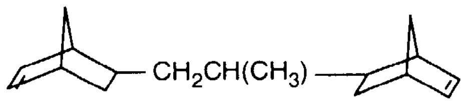

(7),

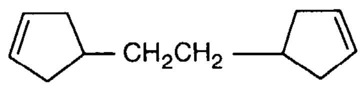

(8),

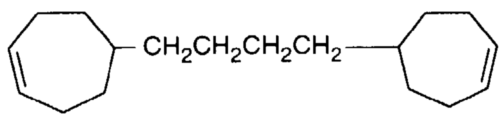

(9),

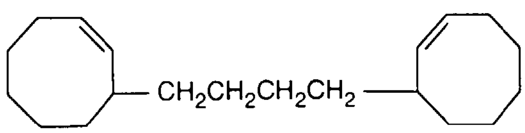

(10),

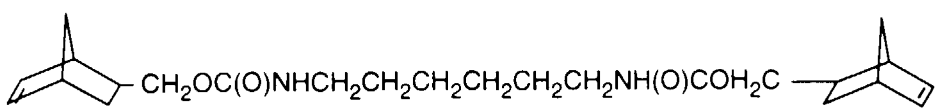

(11),

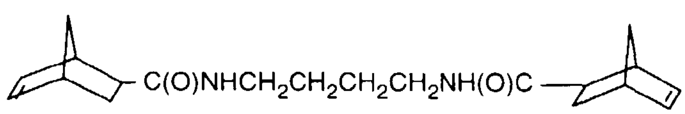

(12),

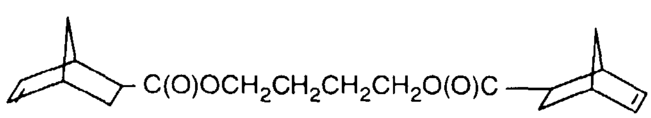

(13),

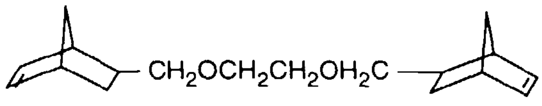

(14),

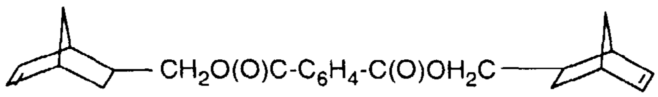

(15),

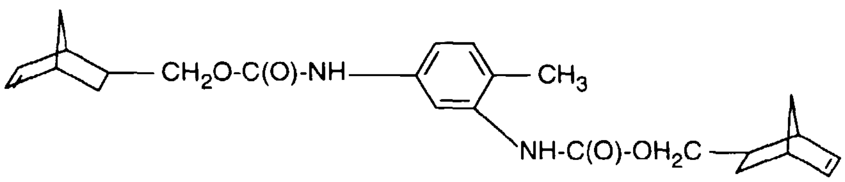

(16)

und

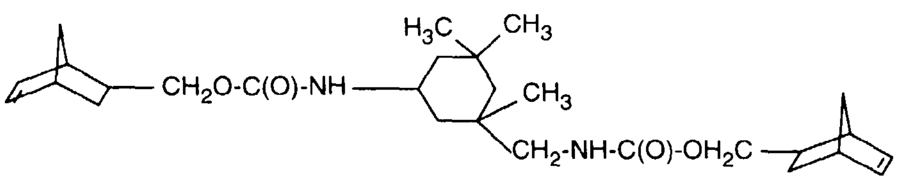

(17).

**4.** Verbindungen gemäss Anspruch 1, dadurch gekennzeichnet, dass die Brückengruppe der Formel VI entspricht,

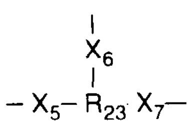

(VI),

worin

$X_5$, $X_6$ und $X_7$ -O-, -$CH_2$-O-, -C(O)O-, -O(O)C-, -$CH_2$-O(O)C-, -C(O)-$NR_{21}$-, -$R_{21}$N-(O)C-, -NH-C(O)-$NR_{21}$-, -O-C(O)-NH-, -$CH_2$-O-C(O)-NH- oder -NH-C(O)-O- bedeuten, und

$R_{23}$ einen dreiwertigen aliphatischen Kohlenwasserstoffrest mit 3 bis 20 C-Atomen, einen dreiwertigen unsubstituierten oder mit $C_1$-$C_4$-Alkyl oder $C_1$-$C_4$-Alkoxy substituierten cycloaliphatischen Rest mit 3 bis 8 Ring-C-Atomen, oder einen dreiwertigen unsubstituierten oder mit $C_1$-$C_4$-Alkyl oder $C_1$-$C_4$-Alkoxy substituierten aromatischen Rest mit 6 bis 18 C-Atomen, einen unsubstituierten oder mit $C_1$-$C_4$-Alkyl oder $C_1$-$C_4$-Alkoxy substituierten dreiwertigen araliphatischen Rest mit 7 bis 19 C-Atomen, oder einen unsubstituierten oder mit $C_1$-$C_4$-Alkyl oder $C_1$-$C_4$-Alkoxy substituierten dreiwertigen heteroaromatischen Rest mit 3 bis 13 C-Atomen und 1 bis 3 Heteroatomen aus der Gruppe -O-, -N- und -S- darstellt, und $R_{21}$ H oder $C_1$-$C_6$-Alkyl ist.

**5.** Verbindungen gemäss Anspruch 4, dadurch gekennzeichnet, dass $X_5$, $X_6$ und $X_7$ -O-, -$CH_2$-O-, -C(O)O-, -O(O)C-, -$CH_2$-O(O)C-, -C(O)-$NR_{21}$-, -$CH_2$-O-C(O)-NH- oder -O-C(O)-NH- darstellen.

6. Verbindungen gemäss Anspruch 4, dadurch gekennzeichnet, dass sich die Reste $R_{23}$ von Triolen; Cyanursäure; Triaminen; Tricarbonsäuren oder Triisocyanaten ableiten.

7. Verbindungen gemäss Anspruch 4, dadurch gekennzeichnet, dass sie aus der folgenden Gruppe von Verbindungen ausgewählt sind

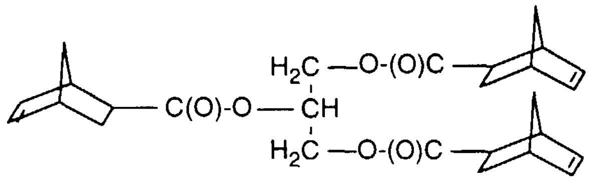

(18),

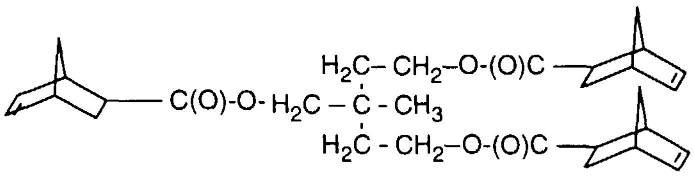

(19),

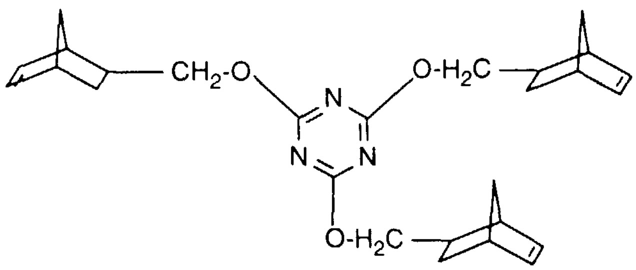

(20),

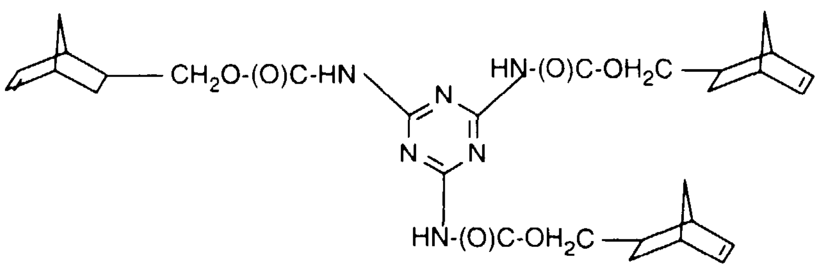

(21)

und

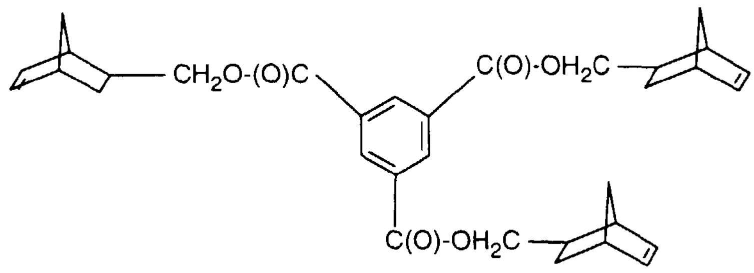

(22).

**8.** Verbindungen gemäss Anspruch 1, dadurch gekennzeichnet, dass die Brückengruppe der Formel VII entspricht,

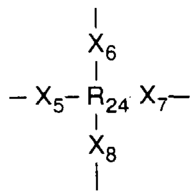

(VII),

worin

$X_5$, $X_6$, $X_7$ und $X_8$ -C(O)O-, $-CH_2$-O(O)C- oder -C(O)-$NR_{21}$- bedeuten, und
$R_{24}$ einen vierwertigen aliphatischen Kohlenwasserstoffrest mit 4 bis 20 C-Atomen, einen vierwertigen unsubstituierten oder mit $C_1$-$C_4$-Alkyl oder $C_1$-$C_4$-Alkoxy substituierten cycloaliphatischen Rest mit 4 bis 8 Ring-C-Atomen, oder einen vierwertigen unsubstituierten oder mit $C_1$-$C_4$-Alkyl oder $C_1$-$C_4$-Alkoxy substituierten aromatischen Rest mit 6 bis 18 C-Atomen, einen vierwertigen unsubstituierten oder mit $C_1$-$C_4$-Alkyl oder $C_1$-$C_4$-Alkoxy substituierten araliphatischen Rest mit 7 bis 19 C-Atomen, oder einen vierwertigen unsubstituierten oder mit $C_1$-$C_4$-Alkyl oder $C_1$-$C_4$-Alkoxy substituierten heteroaromatischen Rest mit 3 bis 13 C-Atomen und 1 bis drei Heteroatomen aus der Gruppe -O-, -N- und -S- darstellt, und $R_{21}$ H oder $C_1$-$C_6$-Alkyl ist.

**9.** Verbindungen gemäss Anspruch 8, dadurch gekennzeichnet, dass sich die Reste $R_{24}$ von Pentaerythrit, Pyromellithsäure oder 3,4,3',4'-Biphenyltetracarbonsäure ableiten.

**10.** Verbindungen gemäss Anspruch 8, dadurch gekennzeichnet, dass sie

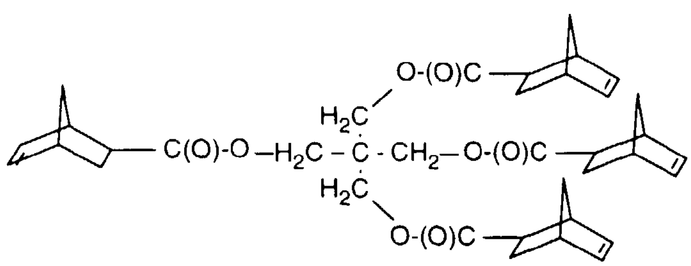

(23)

oder

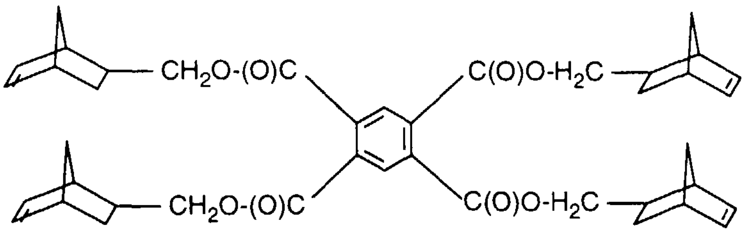

(24)

sind.

**11.** Verbindungen gemäss Anspruch 1, dadurch gekennzeichnet, dass die Verbindungen der Formel I nur Kohlenstoff- und Wasserstoffatome enthalten.

**12.** Zusammensetzung aus (a) mindestens einer Verbindung der Formel I

$$(A)_n\text{-B} \quad \text{(I),}$$

worin A den Rest eines gespannten Cycloolefins bedeutet, B für eine direkte Bindung oder eine n-wertige Brükkengruppe steht, und n eine ganze Zahl von 2 bis 8 darstellt, und (b) einer katalytischen Menge mindestens eines thermo- oder strahlungsaktivierbaren Einkomponenten-Katalysators für eine Metathesepolymerisation, mit Ausnahme von Norbornencarbonsäure(norbornenmethyl)ester der Formel

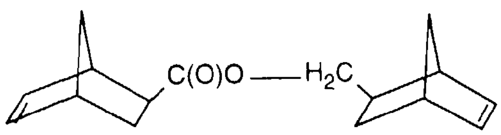

in Kombination mit einer katalytischen Menge mindestens einer thermostabilen Molybdän(VI)- oder Wolfram(VI) verbindung, die mindestens zwei Methylgruppen oder zwei monosubstituierte Methylgruppen am Metall gebunden enthält, wobei der Substituent kein Wasserstoffatom in $\alpha$-Stellung enthält.

**13.** Zusammensetzungen gemäss Anspruch 12, dadurch gekennzeichnet, dass es sich bei den gespannten Cycloolefinen um monocyclische oder polycyclische kondensierte und/oder überbrückte Ringsysteme handelt, die unsubstituiert oder substituiert sind und Heteroatome O, S, N oder Si in einem oder mehreren Ringen und/oder kondensierte alicyclische, aromatische oder heteroaromatische Ringe enthalten können.

**14.** Zusammensetzungen gemäss Anspruch 13, dadurch gekennzeichnet, dass die einzelnen Ringe 3 bis 16 Ringglieder enthalten.

**15.** Zusammensetzungen gemäss Anspruch 13, dadurch gekennzeichnet, dass die cyclischen Ringe 3 bis 12 Ringglieder enthalten.

**16.** Zusammensetzungen gemäss Anspruch 13, dadurch gekennzeichnet, dass die cyclischen Ringe 3 bis 8 Ringglieder enthalten.

**17.** Zusammensetzungen gemäss Anspruch 12, dadurch gekennzeichnet, dass der Rest eines gespannten Cycloolefins der Formel II entspricht

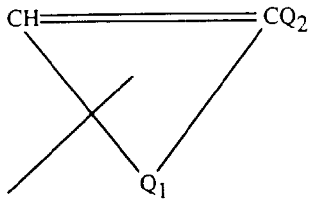

(II),

worin

| | |
|---|---|
| $Q_1$ | ein Rest mit mindestens einem Kohlenstoffatom ist, der zusammen mit der $-CH=CQ_2$-Gruppe einen mindestens 3-gliedrigen alicyclischen Ring bildet, welcher gegebenenfalls ein oder mehrere Heteroatome ausgewählt aus der Gruppe bestehend aus Silicium, Phosphor, Sauerstoff, Stickstoff und Schwefel enthält; und der unsubstituiert oder mit Halogen, =O, -CN, $-NO_2$, $R_1R_2R_3Si-(O)_u-$, -COOM, $-SO_3M$, $-PO_3M$, $-COO(M_1)_{1/2}$, $-SO_3(M_1)_{1/2}$, $-PO_3(M_1)_{1/2}$, $C_1$-$C_{20}$-Alkyl, $C_1$-$C_{20}$-Hydroxyalkyl $C_1$-$C_{20}$-Halogenalkyl, $C_1$-$C_6$-Cyanoalkyl, $C_3$-$C_8$-Cycloalkyl, $C_6$-$C_{16}$-Aryl, $C_7$-$C_{16}$-Aralkyl, $C_3$-$C_6$-Heterocycloalkyl, $C_3$-$C_{16}$-Heteroaryl, $C_4$-$C_{16}$-Heteroaralkyl oder $R_4$-X- substituiert ist; oder bei dem zwei benachbarte C-Atome mit -CO-O-CO- oder $-CO-NR_5-CO-$ substituiert sind; oder bei dem gegebenenfalls an benachbarten Kohlenstoffatomen des alicyclischen Rings ein aromatischer oder heteroaromatischer Ring und/oder weitere alicyclische Ringe ankondensiert sind, welche unsubstituiert oder mit Halogen, -CN, $-NO_2$, $R_6R_7R_8Si-(O)_u-$, -COOM, $-SO_3M$, $-PO_3M$, $-COO(M_1)_{1/2}$, $-SO_3(M_1)_{1/2}$, $-PO_3(M_1)_{1/2}$, $C_1$-$C_{20}$-Alkyl, $C_1$-$C_{20}$-Halogenalkyl, $C_1$-$C_{20}$-Hydroxyalkyl, $C_1$-$C_6$-Cyanoalkyl, $C_3$-$C_8$-Cycloalkyl, $C_6$-$C_{16}$-Aryl, $C_7$-$C_{16}$-Aralkyl, $C_3$-$C_6$-Heterocycloalkyl, $C_3$-$C_6$-Heteroaryl, $C_4$-$C_{16}$-Heteroaralkyl oder $R_{13}$-$X_1$- substituiert ist; |
| X und $X_1$ | unabhängig voneinander für -O-, -S-, -CO-, -SO-, $-SO_2-$, -O-C(O)-, -C(O)-O-, $-C(O)-NR_5-$, $-NR_{10}-C(O)-$, $-SO_2-O-$ oder $-O-SO_2-$ stehen; |
| $R_1$, $R_2$ und $R_3$ | unabhängig voneinander $C_1$-$C_{12}$-Alkyl, $C_1$-$C_{12}$-Perfluoralkyl, Phenyl oder Benzyl bedeuten; |
| $R_4$ und $R_{13}$ | unabhängig $C_1$-$C_{20}$-Alkyl, $C_1$-$C_{20}$-Halogenalkyl, $C_1$-$C_{20}$-Hydroxyalkyl, $C_3$-$C_8$-Cycloalkyl, $C_6$-$C_{16}$-Aryl, $C_7$-$C_{16}$-Aralkyl bedeuten; |
| $R_5$ und $R_{10}$ | unabhängig voneinander Wasserstoff, $C_1$-$C_{12}$-Alkyl, Phenyl oder Benzyl bedeuten, wobei die Alkylgruppen ihrerseits unsubstituiert oder mit $C_1$-$C_{12}$-Alkoxy oder $C_3$-$C_8$-Cycloalkyl substituiert sind; |
| $R_6$, $R_7$ und $R_8$ | unabhängig voneinander $C_1$-$C_{12}$-Alkyl, $C_1$-$C_{12}$-Perfluoralkyl, Phenyl oder Benzyl bedeuten; |
| M | für ein Alkalimetall und $M_1$ für ein Erdalkalimetall stehen; und |
| u | für 0 oder 1 steht; |

wobei der mit $Q_1$ gebildete alicyclische Ring gegebenenfalls weitere nicht-aromatische Doppelbindungen enthält;

| | |
|---|---|
| $Q_2$ | Wasserstoff, $C_1$-$C_{20}$-Alkyl, $C_1$-$C_{20}$-Halogenalkyl, $C_1$-$C_{12}$-Alkoxy, Halogen, -CN oder $R_{11}$-$X_2$- darstellt; |
| $R_{11}$ | $C_1$-$C_{20}$-Alkyl, $C_1$-$C_{20}$-Halogenalkyl, $C_1$-$C_{20}$-Hydroxyalkyl, $C_3$-$C_8$-Cycloalkyl, $C_6$-$C_{16}$-Aryl oder $C_7$-$C_{16}$-Aralkyl bedeutet; |
| $X_2$ | -C(O)-O- oder $-C(O)-NR_{12}-$ ist; |
| $R_{12}$ | Wasserstoff, $C_1$-$C_{12}$-Alkyl, Phenyl oder Benzyl darstellt; |

wobei die vorgenannten Cycloalkyl-, Heterocycloalkyl-, Aryl-, Heteroaryl-, Aralkyl- und Heteroaralkylgruppen unsubstituiert oder mit $C_1$-$C_{12}$-Alkyl, $C_1$-$C_{12}$-Alkoxy, $-NO_2$, -CN oder Halogen substituiert sind, und wobei die Heteroatome der vorgenannten Heterocycloalkyl-, Heteroaryl- und Heteroaralkylgruppen aus der Gruppe -O-, -S-, $-NR_9-$ und -N= ausgewählt sind; und

$R_9$ Wasserstoff, $C_1$-$C_{12}$-Alkyl, Phenyl oder Benzyl darstellt.

**18.** Zusammensetzungen gemäss Anspruch 17, dadurch gekennzeichnet, dass in Formel II $Q_2$ Wasserstoff bedeutet.

**19.** Zusammensetzungen gemäss Anspruch 17, dadurch gekennzeichnet, dass in Formel II der alicyclische Ring, den $Q_1$ zusammen mit der $-CH=CQ_2-$ Gruppe bildet, 3 bis 8 Ringatome aufweist, und wobei es sich um ein monocyclisches, bicyclisches, tricyclisches oder tetracyclisches Ringsystem handelt.

**20.** Zusammensetzungen gemäss Anspruch 17, dadurch gekennzeichnet, dass der Rest eines gespannten Cycloolefins der Formel II entspricht, worin

| | |
|---|---|
| $Q_1$ | ein Rest mit mindestens einem Kohlenstoffatom ist, das zusammen mit der -CH=C$Q_2$-Gruppe einen 3- bis 20-gliedrigen alicyclischen Ring bildet, welcher gegebenenfalls ein oder mehrere Heteroatome ausgewählt aus der Gruppe Silicium, Sauerstoff, Stickstoff und Schwefel enthält; und der unsubstituiert oder mit Halogen, =O, -CN, -$NO_2$, $R_1R_2R_3Si$-$(O)_u$-, -COOM, -$SO_3M$, -$PO_3M$, -COO$(M_1)_{1/2}$, -$SO_3(M_1)_{1/2}$, -$PO_3(M_1)_{1/2}$, $C_1$-$C_{12}$-Alkyl, $C_1$-$C_{12}$-Halogenalkyl, $C_1$-$C_{12}$-Hydroxyalkyl, $C_1$-$C_4$-Cyanoalkyl, $C_3$-$C_6$-Cycloalkyl, $C_6$-$C_{12}$-Aryl, $C_7$-$C_{12}$-Aralkyl, $C_3$-$C_6$-Heterocycloalkyl, $C_3$-$C_{12}$-Heteroaryl, $C_4$-$C_{12}$-Heteroaralkyl oder $R_4$-X- substituiert ist; oder bei dem zwei benachbarte C-Atome in diesem Rest $Q_1$ mit -CO-O-CO- oder -CO-N$R_5$-CO- substituiert sind; oder bei dem gegebenenfalls an benachbarten Kohlenstoffatomen ein aromatischer oder heteroaromatischer Ring und/oder weitere alicyclische Ringe ankondensiert sind, welche unsubstituiert oder mit Halogen, -CN, -$NO_2$, $R_6R_7R_8Si$-, -COOM, -$SO_3M$, -$PO_3M$, -COO$(M_1)_{1/2}$, -$SO_3(M_1)_{1/2}$, -$PO_3(M_1)_{1/2}$, $C_1$-$C_{12}$-Alkyl, $C_1$-$C_{12}$-Halogenalkyl, $C_1$-$C_{12}$-Hydroxyalkyl, $C_1$-$C_4$-Cyanoalkyl, $C_3$-$C_6$-Cycloalkyl, $C_6$-$C_{12}$-Aryl, $C_7$-$C_{12}$-Aralkyl, $C_3$-$C_6$-Heterocycloalkyl, $C_3$-$C_{12}$-Heteroaryl, $C_4$-$C_{12}$-Heteroaralkyl oder $R_{13}$-$X_1$- substituiert sind; |
| X und $X_1$ | unabhängig voneinander für -O-, -S-, -CO-, -SO-, -$SO_2$-, -O-C(O)-, -C(O)-O-, -C(O)-N$R_5$-, -N$R_{10}$-C(O)-, -$SO_2$-O- oder -O-$SO_2$- stehen; und |
| $R_1$, $R_2$ und $R_3$ | unabhängig voneinander $C_1$-$C_6$-Alkyl, $C_1$-$C_6$-Perfiuoralkyl, Phenyl oder Benzyl bedeuten; |
| M | für ein Alkalimetall und $M_1$ für ein Erdalkalimetall stehen; |
| $R_4$ und $R_{13}$ | unabhängig voneinander $C_1$-$C_{12}$-Alkyl, $C_1$-$C_{12}$-Halogenalkyl, $C_1$-$C_{12}$-Hydroxyalkyl, $C_3$-$C_8$-Cycloalkyl, $C_6$-$C_{12}$-Aryl, $C_7$-$C_{12}$-Aralkyl bedeuten; |
| $R_5$ und $R_{10}$ | unabhängig voneinander Wasserstoff, $C_1$-$C_6$-Alkyl, Phenyl oder Benzyl bedeuten, |

wobei die Alkylgruppen ihrerseits unsubstituiert oder mit $C_1$-$C_6$-Alkoxy oder $C_3$-$C_6$-Cycloalkyl substituiert sind;

| | |
|---|---|
| $R_6$, $R_7$ und $R_8$ | unabhängig voneinander $C_1$-$C_6$-Alkyl, $C_1$-$C_6$-Perfluoralkyl, Phenyl oder Benzyl darstellen; |
| u | für 0 oder 1 steht; |

wobei der mit $Q_1$ gebildete alicyclische Ring gegebenenfalls weitere nichtaromatische Doppelbindungen enthält;

| | |
|---|---|
| $Q_2$ | Wasserstoff, $C_1$-$C_{12}$-Alkyl, $C_1$-$C_{12}$-Halogenalkyl, $C_1$-$C_6$-Alkoxy, Halogen, -CN oder $R_{11}$-$X_2$- bedeutet; |
| $R_{11}$ | $C_1$-$C_{12}$-Alkyl, $C_1$-$C_{12}$-Halogenalkyl, $C_1$-$C_{12}$-Hydroxyalkyl, $C_3$-$C_6$-Cycloalkyl, $C_6$-$C_{12}$-Aryl oder $C_7$-$C_{12}$-Aralkyl darstellt; |
| $X_2$ | -C(O)-O- oder -C(O)-N$R_{12}$- ist; und |
| $R_{12}$ | Wasserstoff, $C_1$-$C_6$-Alkyl, Phenyl oder Benzyl bedeutet; |

und wobei die Cycloalkyl-, Heterocycloalkyl-, Aryl-, Heteroaryl-, Aralkyl- und Heteroaralkylgruppen unsubstituiert oder mit $C_1$-$C_6$-Alkyl, $C_1$-$C_6$-Alkoxy, -$NO_2$, -CN oder Halogen substituiert sind, und wobei die Heteroatome der Heterocycloalkyl-, Heteroaryl- und Heteroaralkylgruppen aus der Gruppe -O-, -S-, -N$R_9$- und -N= ausgewählt sind; und $R_9$ Wasserstoff, $C_1$-$C_6$-Alkyl, Phenyl oder Benzyl bedeutet.

**21.** Verbindungen gemäss Anspruch 17, dadurch gekennzeichnet, dass der Rest eines gespannten Cycloolefins der Formel II entspricht, worin

| | |
|---|---|
| $Q_1$ | ein Rest mit mindestens einem Kohlenstoffatom ist, der zusammen mit der -CH=C$Q_2$-Gruppe einen 3- bis 10-gliedrigen alicyclischen Ring bildet, der gegebenenfalls ein Heteroatom ausgewählt aus der Gruppe Silicium, Sauerstoff, Stickstoff und Schwefel enthält, und der unsubstituiert oder mit Halogen, -CN, -$NO_2$, $R_1R_2R_3Si$-, -COOM, -$SO_3M$, -$PO_3M$, -COO$(M_1)_{1/2}$, -$SO_3$ $(M_1)_{1/2}$, -$PO_3(M_1)_{1/2}$, $C_1$-$C_6$-Alkyl, $C_1$-$C_6$-Halogenalkyl, $C_1$-$C_6$-Hydroxyalkyl, $C_1$-$C_4$-Cyanoalkyl, $C_3$-$C_6$-Cycloalkyl, Phenyl, Benzyl oder $R_4$-X- substituiert ist; oder bei dem an benachbarten Kohlenstoffatomen gegebenenfalls ein aromatischer oder heteroaromatischer Ring ankondensiert ist, welcher unsubstituiert oder durch Halogen, -CN, -$NO_2$, $R_6R_7R_8Si$-, -COOM, -$SO_3M$, -$PO_3M$, -COO$(M_1)_{1/2}$, -$SO_3(M_1)_{1/2}$, -$PO_3(M_1)_{1/2}$, $C_1$-$C_6$-Alkyl, $C_1$-$C_6$-Halogenalkyl, $C_1$-$C_6$-Hydroxyalkyl, $C_1$-$C_4$-Cyanoalkyl, $C_3$-$C_6$-Cycloalkyl, Phenyl, Benzyl oder $R_{13}$-$X_1$- substituiert ist; |

| | |
|---|---|
| $R_1$, $R_2$ und $R_3$ | unabhängig voneinander $C_1$-$C_4$-Alkyl, $C_1$-$C_4$-Perfluoralkyl, Phenyl oder Benzyl bedeuten; |
| M | für ein Alkalimetall und $M_1$ für ein Erdalkalimetall stehen; |
| $R_4$ und $R_{13}$ | unabhängig voneinander $C_1$-$C_6$-Alkyl, $C_1$-$C_6$-Halogenalkyl, $C_1$-$C_6$-Hydroxyalkyl oder $C_3$-$C_6$-Cycloalkyl bedeuten; |
| X und $X_1$ | unabhängig voneinander für -O-, -S-, -CO-, -SO- oder -$SO_2$- stehen; |
| $R_6$, $R_7$ und $R_8$ | unabhängig voneinander $C_1$-$C_4$-Alkyl, $C_1$-$C_4$-Perfluoralkyl, Phenyl oder Benzyl darstellen; und |
| $Q_2$ | Wasserstoff bedeutet. |

**22.** Zusammensetzungen gemäss Anspruch 17, dadurch gekennzeichnet, dass es sich bei dem Cycloolefinrest der Formel II um unsubstituiertes oder substituiertes Cyclopropenyl, Cyclobutenyl, Cyclopentenyl, Cycloheptenyl, Cyclooctenyl, Cyclopentadienyl, Cyclohexadienyl, Cycloheptadienyl, Cyclooctadienyl und Norbornenyl oder Norbornenylderivate handelt.

**23.** Zusammensetzungen gemäss Anspruch 17, dadurch gekennzeichnet, dass es sich bei dem Cycloolefinrest der Formel II um einen Rest der Formel III

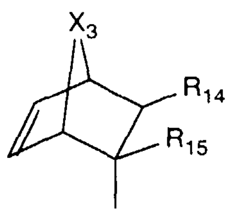

(III),

worin

$X_3$ -$CHR_{16}$-, Sauerstoff oder Schwefel;
$R_{14}$ und $R_{15}$ unabhängig voneinander Wasserstoff, -CN, Trifluormethyl, $(CH_3)_3Si$-O-, $(CH_3)_3Si$- oder -$COOR_{17}$; und
$R_{16}$ und $R_{17}$ unabhängig voneinander Wasserstoff, $C_1$-$C_{12}$-Alkyl, Phenyl oder Benzyl bedeuten;

oder der Formel IV handelt

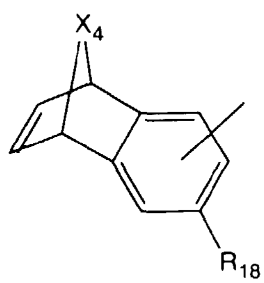

(IV),

worin

$X_4$ -$CHR_{19}$-, Sauerstoff oder Schwefel;
$R_{19}$ Wasserstoff, $C_1$-$C_{12}$-Alkyl, Phenyl oder Benzyl; und
$R_{18}$ Wasserstoff, $C_1$-$C_6$-Alkyl oder Halogen bedeuten.

**24.** Zusammensetzungen gemäss Anspruch 17, dadurch gekennzeichnet, dass es sich bei dem Cycloolefinrest der Formel II um Norbornenyl der Formel

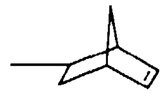

handelt.

**25.** Zusammensetzung gemäss Anspruch 17, dadurch gekennzeichnet, dass sie als Einkomponenten-Katalysator eine thermostabile Ruthenium- oder Osmiumverbindung enthält, die mindestens einen photolabilen an das Ruthenium- oder Osmiumatom gebundenen Liganden besitzt und deren restliche Koordinationsstellen mit nicht-photolabilen Liganden abgesättigt sind.

**26.** Zusammensetzung gemäss Anspruch 25, dadurch gekennzeichnet, dass die Ruthenium- und Osmiumverbindungen der Formel X entsprechen,

$$[L_1Me(L_8)_5]^{2\oplus}[Y_1^{x\ominus}]_{2/x} \qquad (X),$$

worin $L_1$ ein photolabiler Ligand und $L_8$ ein nicht-photolabiler Ligand sind, Me Ru oder Os bedeutet, $Y_1$ ein nicht-koordinierendes Anion bedeutet und x für die Zahlen 1, 2 oder 3 steht.

**27.** Zusammensetzung gemäss Anspruch 17, dadurch gekennzeichnet, dass es sich bei dem Einkomponenten-Katalysator um eine Molybdän(VI)- oder Wolfram(VI)verbindung handelt, die mindestens zwei Methylgruppen oder zwei monosubstituierte Methylgruppen am Metall gebunden enthält, wobei der Substituent kein Wasserstoffatom in $\alpha$-Stellung enthält.

**28.** Zusammensetzung gemäss Anspruch 27, dadurch gekennzeichnet, dass die am Metall gebundenen gegebenenfalls monosubstituierten Methylgruppen der Formel XI entsprechen

$$-CH_2-R \qquad (XI),$$

worin R H, $-CF_3$, $-SiR_{38}R_{39}R_{40}$, $-CR_{41}R_{42}R_{43}$, unsubstituiertes oder mit $C_1$-$C_6$-Alkyl oder $C_1$-$C_6$-Alkoxy substituiertes $C_6$-$C_{16}$-Aryl oder $C_4$-$C_{15}$-Heteroaryl mit 1 bis 3 Heteroatomen aus der Gruppe O, S und N darstellt;

$R_{38}$, $R_{39}$ und $R_{40}$ unabhängig voneinander $C_1$-$C_6$-Alkyl, $C_5$- oder $C_6$-Cycloalkyl, oder unsubstituiertes oder mit $C_1$-$C_6$-Alkyl oder $C_1$-$C_6$-Alkoxy substituiertes Phenyl oder Benzyl bedeuten; und
$R_{41}$, $R_{42}$ und $R_{43}$ unabhängig voneinander $C_1$-$C_{10}$- Alkyl bedeuten, das unsubstituiert oder mit $C_1$-$C_{10}$-Alkoxy substituiert ist, oder $R_{41}$ und $R_{42}$ diese Bedeutung haben und $R_{43}$ $C_6$-$C_{10}$-Aryl oder $C_4$-$C_9$-Heteroaryl ist, das unsubstituiert oder mit $C_1$-$C_6$-Alkyl oder $C_1$-$C_6$-Alkoxy substituiert ist.

**29.** Zusammensetzung gemäss Anspruch 17, dadurch gekennzeichnet, dass es sich bei dem Einkomponenten-Katalysator um eine thermostabile Titan(IV), Niob(V)-, Tantal(V)-, Molybdän(VI)- oder Wolfram(VI)verbindung handelt, in der eine Silylmethylgruppe und mindestens ein Halogen am Metall gebunden sind.

**30.** Zusammensetzung gemäss Anspruch 29, dadurch gekennzeichnet, dass die Silylmethylgruppe der Formel XIV entspricht,

$$-CH_2-SiR_{38}R_{39}R_{40} \qquad (XIV),$$

worin

$R_{38}$, $R_{39}$ und $R_{40}$ unabhängig voneinander $C_1$-$C_{18}$-Alkyl, $C_5$- oder $C_6$-Cycloalkyl, oder unsubstituiertes oder mit $C_1$-$C_6$-Alkyl oder $C_1$-$C_6$-Alkoxy substituiertes Phenyl oder Benzyl bedeuten.

**31.** Zusammensetzung gemäss Anspruch 17, dadurch gekennzeichnet, dass es sich bei dem Einkomponenten-Katalysator um eine Niob(V)- oder Tantal(V)verbindung handelt, die mindestens zwei Methylgruppen oder zwei monosubstituierte Methylgruppen am Metall gebunden enthält, wobei der Substituent kein Wasserstoffatom in $\alpha$-Stellung enthält.

**32.** Zusammensetzung gemäss Anspruch 31, dadurch gekennzeichnet, dass die gegebenenfalls monosubstituierten Methylgruppen der Formel XI entsprechen,

$$-CH_2-R \qquad (XI),$$

worin worin R H, $-CF_3$, $-SiR_{38}R_{39}R_{40}$, $-CR_{41}R_{42}R_{43}$, unsubstituiertes oder mit $C_1$-$C_6$-Alkyl oder $C_1$-$C_6$-Alkoxy substituiertes $C_6$-$C_{16}$-Aryl oder $C_4$-$C_{15}$-Heteroaryl mit 1 bis 3 Heteroatomen aus der Gruppe O, S und N darstellt;

$R_{38}$, $R_{39}$ und $R_{40}$ unabhängig voneinander $C_1$-$C_{12}$-Alkyl, $C_5$- oder $C_6$-Cycloalkyl, oder unsubstituiertes oder mit $C_1$-$C_6$-Alkyl oder $C_1$-$C_6$-Alkoxy substituiertes Phenyl oder Benzyl bedeuten, und
$R_{41}$, $R_{42}$ und $R_{43}$ unabhängig voneinander $C_1$-$C_{10}$- Alkyl bedeuten, das unsubstituiert oder mit $C_1$-$C_{10}$-Alkoxy substituiert ist, oder $R_{41}$ und $R_{42}$ diese Bedeutung haben und $R_{43}$ $C_6$-$C_{10}$-Aryl oder $C_4$-$C_9$-Heteroaryl ist, das unsubstituiert oder mit $C_1$-$C_6$-Alkyl oder $C_1$-$C_6$-Alkoxy substituiert ist.

**33.** Zusammensetzung gemäss Anspruch 17, dadurch gekennzeichnet, dass es sich bei dem Einkomponenten-Katalysator um eine Titan(IV)verbindung handelt, die mindestens zwei Methylgruppen oder zwei monosubstituierte Methylgruppen am Metall gebunden enthält, wobei der Substituent kein Wasserstoffatom in $\alpha$-Stellung enthält.

**34.** Zusammensetzung gemäss Anspruch 33, dadurch gekennzeichnet, dass die gegebenenfalls monosubstituierten Methylgruppen der Formel XI entsprechen,

$$-CH_2-R \qquad (XI),$$

worin worin R H, $-CF_3$, $-SiR_{38}R_{39}R_{40}$, $-CR_{41}R_{42}R_{43}$, unsubstituiertes oder mit $C_1$-$C_6$-Alkyl oder $C_1$-$C_6$-Alkoxy substituiertes $C_6$-$C_{16}$-Aryl oder $C_4$-$C_{15}$-Heteroaryl mit 1 bis 3 Heteroatomen aus der Gruppe O, S und N darstellt;

$R_{38}$, $R_{39}$ und $R_{40}$ unabhängig voneinander $C_1$-$C_{12}$-Alkyl, $C_5$- oder $C_6$-Cycloalkyl, oder unsubstituiertes oder mit $C_1$-$C_6$-Alkyl oder $C_1$-$C_6$-Alkoxy substituiertes Phenyl oder Benzyl bedeuten, und
$R_{41}$, $R_{47}$ und $R_{43}$ unabhängig voneinander $C_1$-$C_{10}$- Alkyl bedeuten, das unsubstituiert oder mit $C_1$-$C_{10}$-Alkoxy substituiert ist, oder $R_{41}$ und $R_{42}$ diese Bedeutung haben und $R_{43}$ $C_6$-$C_{10}$-Aryl oder $C_4$-$C_9$-Heteroaryl ist, das unsubstituiert oder mit $C_1$-$C_6$-Alkyl oder $C_1$-$C_6$-Alkoxy substituiert ist.

**35.** Zusammensetzung gemäss Anspruch 17, dadurch gekennzeichnet, dass der Einkomponenten-Katalysator eine Ruthenium- oder Osmiumverbindung ist, die mindestens eine Phosphingruppe, mindestens einen photolabilen Liganden, und gegebenenfalls Neutralliganden an das Metallatom gebunden enthält, wobei insgesamt 2 bis 5 Liganden gebunden sind, und die Säureanionen zum Ladungsausgleich enthält.

**36.** Zusammensetzung gemäss Anspruch 35, dadurch gekennzeichnet, dass die Phosphinliganden den Formeln XXIII oder XXIIIa entsprechen

$$PR_{91}R_{92}R_{93} \qquad (XXIII),$$

$$R_{91}R_{92}P-Z_1-PR_{91}R_{92} \qquad (XXIIIa),$$

worin $R_{91}$, $R_{92}$ und $R_{93}$ unabhängig voneinander H, $C_1$-$C_{20}$-Alkyl, unsubstituiertes oder mit $C_1$-$C_6$-Alkyl, $C_1$-$C_6$-Halogenalkyl oder $C_1$-$C_6$-Alkoxy substituiertes $C_4$-$C_{12}$-Cycloalkyl, oder unsubstituiertes oder mit $C_1$-$C_6$-Alkyl, $C_1$-$C_6$-Halogenalkyl oder $C_1$-$C_6$-Alkoxy substituiertes $C_6$-$C_{16}$-Aryl, oder unsubstituiertes oder mit $C_1$-$C_6$-Alkyl, $C_1$-$C_6$-Halogenalkyl oder $C_1$-$C_6$-Alkoxy substituiertes $C_7$-$C_{16}$-Aralkyl darstellt; die Reste $R_{91}$ und $R_{92}$ gemeinsam unsub-

stituiertes oder mit $C_1$-$C_6$-Alkyl, $C_1$-$C_6$-Halogenalkyl oder $C_1$-$C_6$-Alkoxy substituiertes Tetra- oder Pentamethylen bedeuten, oder unsubstituiertes oder mit $C_1$-$C_6$-Alkyl, $C_1$-$C_6$-Halogenalkyl oder $C_1$-$C_6$-Alkoxy substituiertes und mit 1 oder 2 1,2-Phenylen kondensiertem Tetra- oder Pentamethylen darstellen, und $R_{93}$ die zuvor angegebene Bedeutung hat; und

$Z_1$ lineares oder verzweigtes, unsubstituiertes oder mit $C_1$-$C_4$-Alkoxy substituiertes $C_2$-$C_{12}$-Alkylen, unsubstituiertes oder mit $C_1$-$C_4$-Alkyl oder $C_1$-$C_4$-Alkoxy substituiertes 1.2- oder 1,3-Cycloalkylen mit 4 bis 8 C-Atomen, oder unsubstituiertes oder mit $C_1$-$C_4$-Alkyl oder $C_1$-$C_4$-Alkoxy substituiertes 1,2 oder 1,3-Heterocycloalkylen mit 5 oder 6 Ringgliedern und einem Heteroatom aus der Gruppe O oder N bedeutet.

**37.** Zusammensetzung gemäss Anspruch 17, dadurch gekennzeichnet, dass es sich bei dem Einkomponenten-Katalysator um eine zweiwertig-kationische Ruthenium- oder Osmiumverbindung mit einem Metallatom, woran 1 bis 3 tertiäre Phosphinliganden mit im Fall der Rutheniumverbindungen sterisch anspruchsvollen Substituenten, gegebenenfalls nicht-photolabile Neutralliganden und Anionen zum Ladungsausgleich gebunden sind. handelt, mit der Massgabe, dass in Ruthenium(trisphenylphosphin)dihalogeniden oder -hydrid-halogeniden die Phenylgruppen mit $C_1$-$C_{18}$-Alkyl, $C_1$-$C_{18}$-Halogenalkyl oder $C_1$-$C_{18}$-Alkoxy substituiert sind.

**38.** Zusammensetzung gemäss Anspruch 37, dadurch gekennzeichnet, dass die Phosphinliganden den Formeln XXIII oder XXIIIa entsprechen

$$PR_{91}R_{92}R_{93} \qquad \text{(XXIII)},$$

$$R_{91}R_{92}P\text{-}Z_1\text{-}PR_{91}R_{92} \qquad \text{(XXIIIa)},$$

worin $R_{91}$, $R_{92}$ und $R_{93}$ unabhängig voneinander H, $C_1$-$C_{20}$-Alkyl, unsubstituiertes oder mit $C_1$-$C_6$-Alkyl, $C_1$-$C_6$-Halogenalkyl oder $C_1$-$C_6$-Alkoxy substituiertes $C_4$-$C_{12}$-Cycloalkyl, oder unsubstituiertes oder mit $C_1$-$C_6$-Alkyl, $C_1$-$C_6$-Halogenalkyl oder $C_1$-$C_6$-Alkoxy substituiertes $C_6$-$C_{16}$-Aryl, oder unsubstituiertes oder mit $C_1$-$C_6$-Alkyl, $C_1$-$C_6$-Halogenalkyl oder $C_1$-$C_6$-Alkoxy substituiertes $C_7$-$C_{16}$-Aralkyl darstellt; die Reste $R_{91}$ und $R_{92}$ gemeinsam unsubstituiertes oder mit $C_1$-$C_6$-Alkyl, $C_1$-$C_6$-Halogenalkyl oder $C_1$-$C_6$-Alkoxy substituiertes Tetra- oder Pentamethylen bedeuten, oder unsubstituiertes oder mit $C_1$-$C_6$-Alkyl, $C_1$-$C_6$-Halogenalkyl oder $C_1$-$C_6$-Alkoxy substituiertes und mit 1 oder 2 1,2-Phenylen kondensiertem Tetra- oder Pentamethylen darstellen, und $R_{93}$ die zuvor angegebene Bedeutung hat; und

$Z_1$ lineares oder verzweigtes, unsubstituiertes oder mit $C_1$-$C_4$-Alkoxy substituiertes $C_2$-$C_{12}$-Alkylen, unsubstituiertes oder mit $C_1$-$C_4$-Alkyl oder $C_1$-$C_4$-Alkoxy substituiertes 1,2- oder 1,3-Cycloalkylen mit 4 bis 8 C-Atomen, oder unsubstituiertes oder mit $C_1$-$C_4$-Alkyl oder $C_1$-$C_4$-Alkoxy substituiertes 1,2 oder 1,3-Heterocycloalkylen mit 5 oder 6 Ringgliedern und einem Heteroatom aus der Gruppe O oder N bedeutet.

**39.** Zusammensetzung gemäss Anspruch 17, dadurch gekennzeichnet, dass der Einkomponenten-Katalysator in einer Menge von 0,001 bis 20 Mol-%, bezogen auf die Menge des Monomers enthalten ist.

**40.** Verfahren zur Herstellung vernetzter Polymerisate durch Metathesepolymerisation gemäss Anspruch 1, dadurch gekennzeichnet, dass man eine Zusammensetzung aus

(a) mindestens einer Verbindung der Formel I und
(b) einer katalytischen Menge mindestens eines thermo- oder strahlungsaktivierbaren Einkomponenten-Katalysators für eine Metathesepolymerisation,
(c) durch Erwärmen polymerisiert,
(d) durch Bestrahlung polymerisiert,
(e) durch Erwärmung und Bestrahlung polymerisiert,
(f) durch kurzzeitige Erwärmung den Einkomponenten-Katalysator aktiviert und die Polymerisation durch Bestrahlung beendet, oder
(g) durch kurzzeitige Bestrahlung den Einkomponenten-Katalysator aktiviert und die Polymerisation durch Erwärmen beendet.

**41.** Verfahren gemäss Anspruch 40, dadurch gekennzeichnet, dass es bei einer Temperatur von 20 bis 300°C durchgeführt wird.

**42.** Vernetzte Metathesepolymerisate aus mindestens einer Verbindung (I) gemäss Anspruch 1.

**43.** Beschichtetes Trägermaterial, dadurch gekennzeichnet, daß auf einem Substrat eine Schicht aus einer Zusammensetzung gemäss Anspruch 17 aufgebracht ist.

**44.** Beschichtetes Substrat mit einer gehärteten Schicht aus einer Zusammensetzung gemäss Anspruch 17.

## Claims

**1.** Compounds of the formula I

$$(A)_n\text{-}B \qquad (I),$$

in which A is the radical of a strained cycloolefin, n is the number two and B is the direct bond or a bridging group of the formula V

$$\text{-}X_5\text{-}R_{20}\text{-}X_6\text{-} \qquad (V)$$

in which

| | |
|---|---|
| $X_5$ and $X_6$ | independently of one another are a direct bond, -O-, -$CH_2$-O-, -C(O)O-, -O(O)C-, -$CH_2$-O(O)C-, -C(O)-$NR_{21}$-, -$R_{21}$N-(O)C-, -NH-C(O)-$NR_{21}$-, -O-C(O)-NH-, -$CH_2$-O-C(O)-NH- or -NH-C(O)-O- and |
| $R_{20}$ | is $C_2$-$C_{18}$alkylene, $C_5$-$C_8$cycloalkylene which is unsubstituted or substituted by $C_1$-$C_4$alkyl or $C_1$-$C_4$alkoxy, $C_6$-$C_{18}$arylene or $C_7$-$C_{19}$aralkylene which are unsubstituted or substituted by $C_1$-$C_4$alkyl or $C_1$-$C_4$alkoxy, or polyoxaalkylene having 2 to 12 oxaalkylene units and 2 to 6 C atoms in the alkylene, and |
| $R_{21}$ | is H or $C_1$-$C_6$alkyl, with the exception of 1,2-bisnorbornenyl-ethane, norbornenecarboxylic acid norbornenemethyl ester and compounds of the formula. |

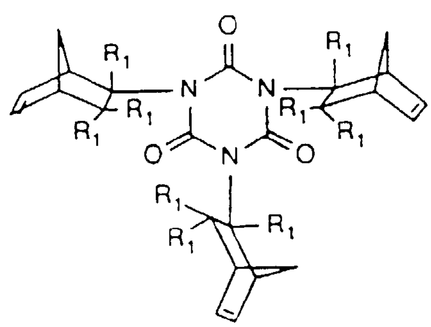

in which $R_1$ is hydrogen or alkyl.

**2.** Compounds according to claim 1, characterized in that, in formula V,

a) $X_5$ and $X_6$ are a direct bond and $R_{20}$ is $C_2$-$C_{18}$alkylene, or
b) $X_5$ and $X_6$ are -O-, -$CH_2$-O-, -C(O)O-, -O(O)C-, -$CH_2$-O(O)C-, -C(O)-$NR_{21}$-, -O- C(O)-NH- or -$CH_2$-O-C(O)-NH-, and $R_{20}$ is $C_2$-$C_{12}$alkylene, phenylene, naphthylene or benzylene which are unsubstituted or substituted

by $C_1$-$C_4$alkyl or $C_1$-$C_4$alkoxy, or $-R_{22}$-(O-$R_{22}$-)$_x$-$OR_{22}$-, in which x is a number from 2 to 4, and $R_{22}$ is -$C_2$-$C_4$alkylene.

**3.** Compounds according to claim 1, characterized in that they are chosen from the following group of compounds

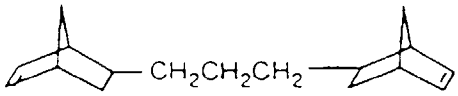

(1),

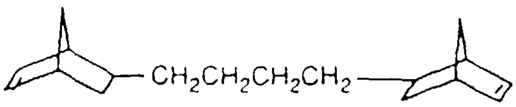

(2),

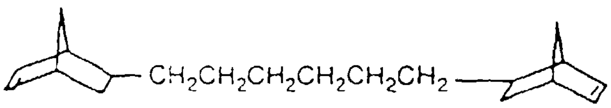

(3),

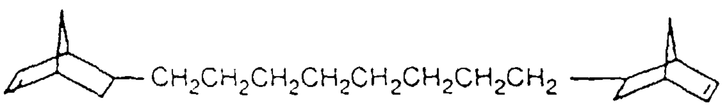

(4),

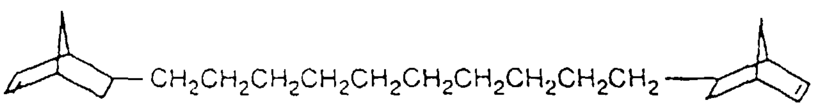

(5),

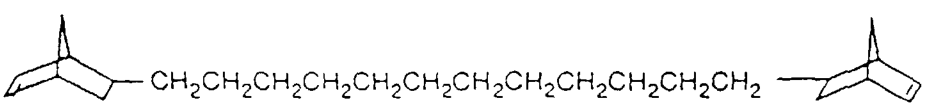

(6)

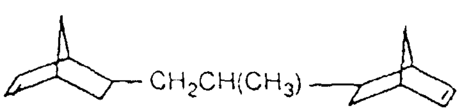

(7),

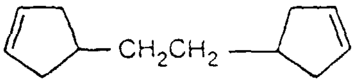

(8),

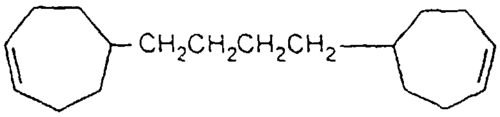

(9),

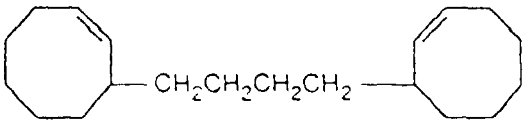

(10),

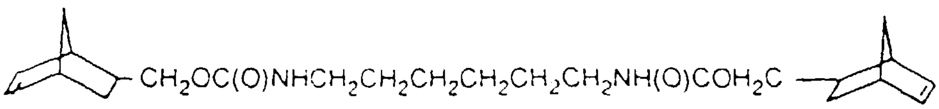

(11),

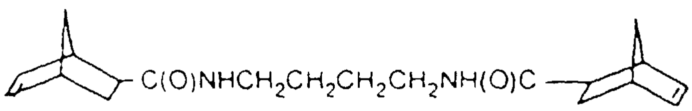

(12),

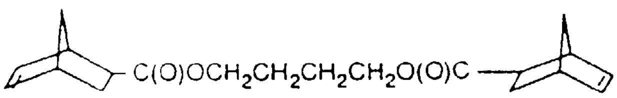

(13),

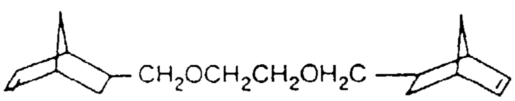

(14),

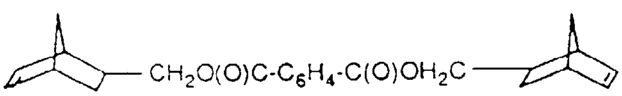

(15),

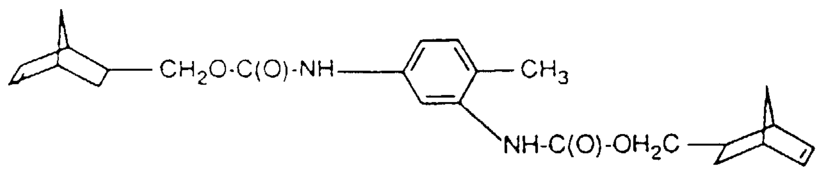

(16)

and

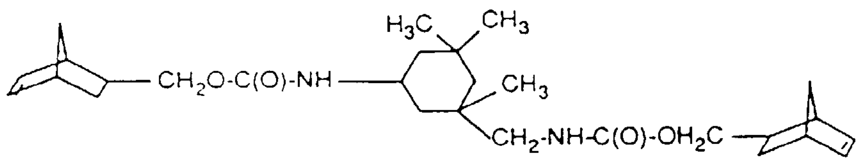

(17).

**4.** Compounds according to claim 1, characterized in that the bridging group corresponds to the formula VI

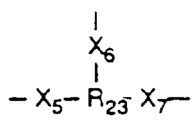

(VI),

in which

$X_5$, $X_6$ and $X_7$ are -O-, $-CH_2-O-$, -C(O)O-, -O(O)C-, $-CH_2-O(O)C-$, $-C(O)-NR_{21}-$, $-R_{21}N-(O)C-$, $-NH-C(O)-NR_{21}-$, -O-C(O)-NH-, $-CH_2-O-C(O)-NH-$ or -NH-C(O)-O-, and
$R_{23}$ is a trivalent aliphatic hydrocarbon radical having 3 to 20 C atoms, a trivalent cycloaliphatic radical which has 3 to 8 ring C atoms and is unsubstituted or substituted by $C_1$-$C_4$alkyl or $C_1$-$C_4$alkoxy, or a trivalent aromatic radical which has 6 to 18 C atoms and is unsubstituted or substituted by $C_1$-$C_4$alkyl or $C_1$-$C_4$alkoxy, a trivalent araliphatic radical which has 7 to 19 C atoms and is unsubstituted or substituted by $C_1$-$C_4$alkyl or $C_1$-$C_4$alkoxy, or a trivalent heteroaromatic radical which has 3 to 13 C atoms and 1 to 3 heteroatoms from the group consisting of-O-, -N- and -S- and is unsubstituted or substituted by $C_1$-$C_4$alkyl or $C_1$-$C_4$alkoxy, and $R_{21}$ is H or $C_1$-$C_6$alkyl.

**5.** Compounds according to claim 4, characterized in that $X_5$, $X_6$ and $X_7$ are -O-,$-CH_2-O-$, -C(O)O-, -O(O)C-, $-CH_2-O(O)C-$, $-C(O)-NR_{21}-$, $-CH_2-O-C(O)-NH-$ or -O-C(O)-NH-.

**6.** Compounds according to claim 4, characterized in that the radicals $R_{23}$ are derived from triols; cyanuric acid; triamines; tricarboxylic acids or triisocyanates.

**7.** Compounds according to claim 4, characterized in that they are chosen from the following group of compounds

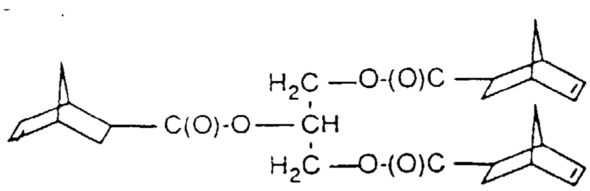

(18),

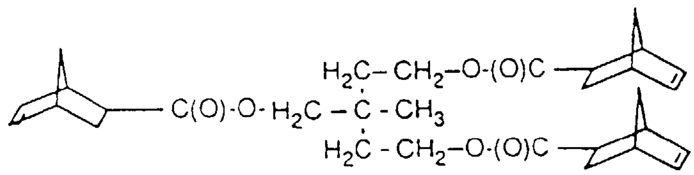

(19),

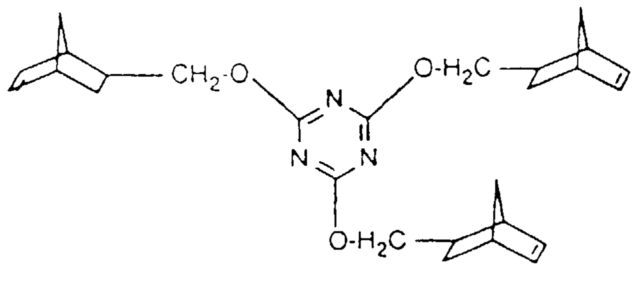

(20),

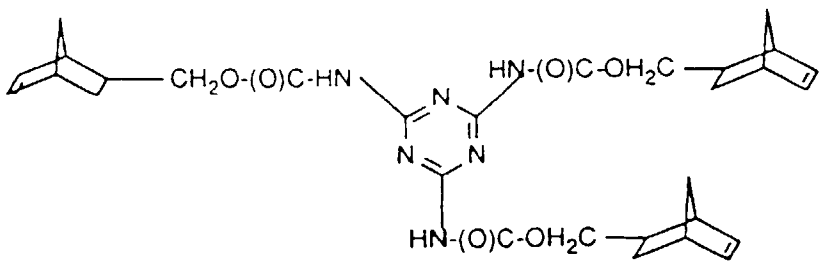

(21)

and

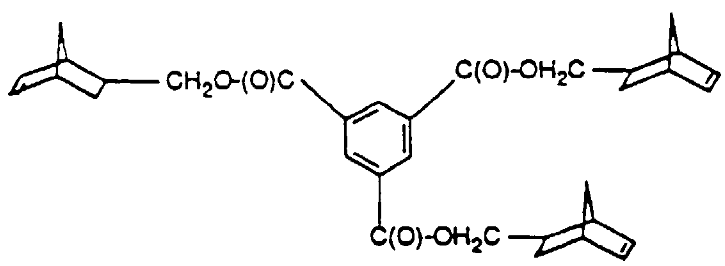

(22).

**8.** Compounds according to claim 1, characterized in that the bridging group corresponds to the formula VII

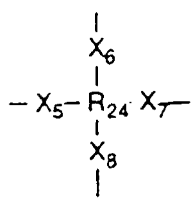

(VII),

in which

$X_5$, $X_6$, $X_7$ and $X_5$ are -C(O)O-, $-CH_2-O(O)C-$ or $-C(O)-NR_{21}-$ and $R_{24}$ is a tetravalent aliphatic hydrocarbon radical having 4 to 20 C atoms, a tetravalent cycloaliphatic radical which has 4 to 8 ring C atoms and is unsubstituted or substituted by $C_1$-$C_4$alkyl or $C_1$-$C_4$alkoxy, or a tetravalent aromatic radical which has 6 to 18 C atoms and is unsubstituted or substituted by $C_1$-$C_4$alkyl or $C_1$-$C_4$alkoxy, a tetravalent araliphatic radical which has 7 to 19 C atoms and is unsubstituted or substituted by $C_1$-$C_4$alkyl or $C_1$-$C_4$alkoxy, or a tetravalent heteroaromatic radical which has 3 to 13 C atoms and 1 to three heteroatoms, from the group consisting of -O-, -N- and -S- and is unsubstituted or substituted by $C_1$-$C_4$alkyl or $C_1$-$C_4$alkoxy, and $R_{21}$ is H or $C_1$-$C_6$alkyl.

**9.** Compounds according to claim 8, characterized in that the radicals $R_{24}$ are derived from pentaerythritol, pyromellitic acid and 3,4,3',4'-biphenyltetracarboxylic acid.

**10.** Compounds according to claim 8, characterized in that they are

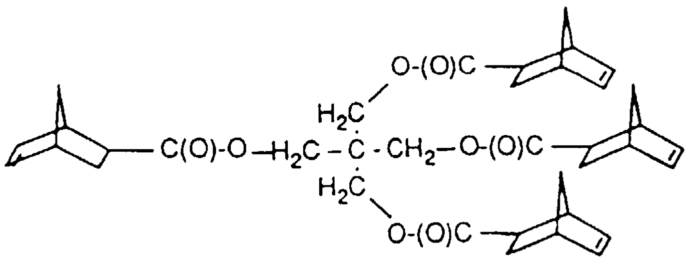

(23)

or

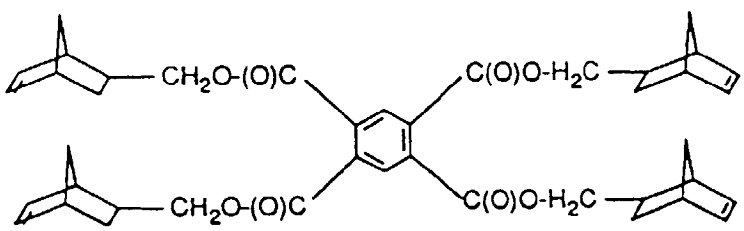

(24)

**11.** Compounds according to claim 1, characterized in that the compounds of the formula I contain only carbon and hydrogen atoms.

**12.** Composition of (a) at least one compound of the formula I

$$(A)_n\text{-}B \qquad (I)$$

in which A is the radical of a strained cycloolefin, B is a direct bond or an n-valent bridging group and n is an integer from 2 to 8, and
(b) a catalytic amount of at least one one-component catalyst for metathesis polymerization which can be activated by heat or radiation,
with the exception of norbornenecarboxylic acid norbenenemethyl ester of the formula

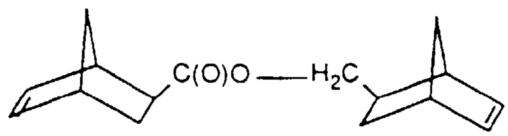

in combination with a catalytic amount of at least one heat-stable molybdenum(VI) or tungsten(VI) compound which contains, bonded to the metal, at least two methyl groups or two monosubstituted methyl groups, the substituent containing no hydrogen atom in the a position.

**13.** Compositions according to claim 12, characterized in that the strained cycloolefins are monocyclic or polycyclic fused and/or bridged ring systems which are unsubstituted or substituted and can contain heteroatoms O, S, N or Si in one or more rings and/or fused alicyclic, aromatic or heteroaromatic rings.

**14.** Compositions according to claim 13, characterized in that the individual rings contain 3 to 16 ring members.

**15.** Compositions according to claim 13, characterized in that the cyclic rings contain 3 to 12 ring members.

**16.** Compositions according to claim 13, characterized in that the cyclic rings contain 3 to 8 ring members.

**17.** Compositions according to claim 12, characterized in that the radical of a strained cycloolefin corresponds to the formula II

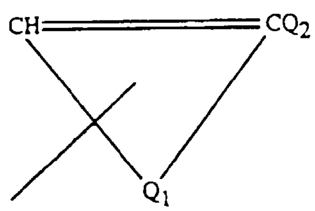

(II),

| | |
|---|---|
| $Q_1$ | is a radical having at least one carbon atom which, together with the $-CH=CQ_2$ group, forms an at least 3-membered alicyclic ring which optionally contains one or more heteroatoms chosen from the group consisting of silicon, phosphorus, oxygen, nitrogen and sulfur; and which is unsubstituted or substituted by halogen, =O, -CN, $-NO_2$, $R_1R_2R_3Si-(O)_u-$, -COOM, $-SO_3M$,-$PO_3M$, $-COO(M_1)_{1/2}$, $-SO_3(M_1)_{1/2}$, $-PO_3(M_1)_{1/2}$,$C_1$-$C_{20}$alkyl, $C_1$-$C_{20}$hydroxyalkyl, $C_1$-$C_{20}$haloalkyl, $C_1$-$C_6$cyanoalkyl, $C_3$-$C_8$cycloalkyl, $C_6$-$C_{16}$aryl, $C_7$-$C_{16}$aralkyl, $C_3$-$C_6$heterocycloalkyl, $C_3$-$C_{16}$heteroaryl, $C_4$-$C_{16}$heteroaralkyl or $R_4$-X-; or in which two adjacent C atoms are substituted by -CO-O-CO- or $-CO-NR_5-CO-$; or in which an aromatic or heteroaromatic ring and/or further alicyclic rings which are unsubstituted or substituted by halogen, -CN, $-NO_2$, $R_6R_7R_8Si-(O)_u-$, -COOM, $-SO_3M$, $-PO_3M$, $-COO(M_1)_{1/2}$, $-SO_3(M_1)_{1/2}$, $-PO_3(M_1)_{1/2}$, $C_1$-$C_{20}$alkyl, $C_1$-$C_{20}$haloalkyl, $C_1$-$C_{20}$hydroxyalkyl, $C_1$-$C_6$cyanoalkyl, $C_3$-$C_8$cycloalkyl, $C_6$-$C_{16}$aryl, $C_7$-$C_{16}$aralkyl, $C_3$-$C_6$heterocycloalkyl, $C_3$-$C_{16}$heteroaryl, $C_4$-$C_{16}$heteroaralkyl or $R_{13}$-$X_1$- are optionally fused onto adjacent carbon atoms of the alicyclic ring; |
| X and $X_1$ | independently of one another are -O-,-S-, -CO-, -SO-, $-SO_2-$, -O-C(O)-, -C(O)-O-, $-C(O)-NR_5-$, $-NR_{10}-C(O)-$, $-SO_2-O-$ or $-O-SO_2-$; |
| $R_1$, $R_2$ and $R_3$ | independently of one another are $C_1$-$C_{12}$alkyl, $C_1$-$C_{12}$Perfluoroalkyl, phenyl or benzyl; |
| $R_4$ and $R_{13}$ | independently are $C_1$-$C_{20}$alkyl, $C_1$-$C_{20}$haloalkyl, $C_1$-$C_{20}$hydroxyalkyl, $C_2$-$C_8$cycloalkyl, $C_6$-$C_{16}$aryl or $C_7$-$C_{16}$aralkyl; |
| $R_5$ and $R_{10}$ | independently of one another are hydrogen, $C_1$-$C_{12}$alkyl, phenyl or benzyl, where the alkyl groups in turn are unsubstituted or substituted by $C_1$-$C_{12}$alkoxy or $C_3$-$C_8$cycloalkyl; |
| $R_6$, $R_7$ and $R_8$ | independently of one another are $C_1$-$C_{12}$alkyl, $C_1$-$C_{12}$perfluoroalkyl, phenyl or benzyl; |
| M | is an alkali metal and $M_1$ is an alkaline earth metal; and |
| u | is 0 or 1; |

where the alicyclic ring formed with $Q_1$ optionally contains further non-aromatic double bonds;

$Q_2$ is hydrogen, $C_1$-$C_{20}$alkyl, $C_1$-$C_{20}$haloalkyl, $C_1$-$C_{12}$alkoxy, halogen, -CN or $R_{11}$-$X_2$;
$R_{11}$ is $C_1$-$C_{20}$alkyl, $C_1$-$C_{20}$haloalkyl, $C_1$-$C_{20}$hydroxyalkyl, $C_3$-$C_8$cycloalkyl, $C_6$-$C_{16}$aryl or $C_7$-$C_{16}$aralkyl;
$X_2$ is -C(O)-O- or -C(O)-$NR_{12}$-;
$R_{12}$ is hydrogen, $C_1$-$C_{12}$alkyl, phenyl or benzyl;

where the abovementioned cycloalkyl, heterocycloalkyl, aryl, heteroaryl, aralkyl and heteroaralkyl groups are unsubstituted or substituted by $C_1$-$C_{12}$alkyl, $C_1$-$C_{12}$alkoxy, -$NO_2$, -CN or halogen, and where the heteroatoms of the abovementioned heterocycloalkyl, heteroaryl and heteroaralkyl groups are chosen from the group consisting of -O-, -S-, -$NR_9$- and -N=; and

$R_9$ is hydrogen, $C_1$-$C_{12}$alkyl, phenyl or benzyl.

**18.** Compositions according to claim 17, characterized in that, in formula II, $Q_2$ is hydrogen.

**19.** Compositions according to claim 17, characterized in that, in formula II, the alicyclic ring which $Q_1$ forms together with the -CH=$CQ_2$- group contains 3 to 8 ring atoms, the ring being a monocyclic, bicyclic, tricyclic or tetracyclic ring system.

**20.** Compositions according to claim 17, characterized in that the radical of a strained cycloolefin corresponds to the formula II, in which

| | |
|---|---|
| $Q_1$ | is a radical with at least one carbon atom which, together with the -CH=$CQ_2$-group, forms a 3- to 20-membered alicyclic ring which optionally contains one or more heteroatoms chosen from the group consisting of silicon, oxygen, nitrogen and sulfur; and which is unsubstituted or substituted by halogen, =0, -CN, -$NO_2$, $R_1R_2R_3Si$-$(O)_u$-, -COOM, -$SO_3M$, -$PO_3M$, -COO$(M_1)_{1/2}$, -$SO_3(M_1)_{1/2}$, -$PO_3(M_1)_{1/2}$, $C_1$-$C_{12}$alkyl, $C_1$-$C_{12}$haloalkyl, $C_1$-$C_{12}$hydroxyalkyl, $C_1$-$C_4$cyanoalkyl, $C_3$-$C_6$cycloalkyl, $C_6$-$C_{12}$aryl, $C_7$-$C_{12}$aralkyl, $C_3$-$C_6$heterocycloalkyl, $C_1$-$C_{12}$heteroaryl, $C_4$-$C_{12}$heteroaralkyl or $R_4$-X-; or in which two adjacent C atoms in this radical $Q_1$ are substituted by -CO-O-CO- or -CO-$NR_5$-CO-; or in which an aromatic or heteroaromatic ring and/or further alicyclic rings which are unsubstituted or substituted by halogen, -CN, -$NO_2$, $R_6R_7R_8Si$-, -COOM, -$SO_3M$, -$PO_3M$, -COO$(M_1)_{1/2}$, -$SO_3(M_1)_{1/2}$, -$PO_3(M_1)_{1/2}$, $C_1$-$C_{12}$alkyl, $C_1$-$C_{12}$haloalkyl, $C_1$-$C_{12}$hydroxyalkyl, $C_1$-$C_4$cyanoalkyl, $C_3$-$C_6$cycloalkyl, $C_6$-$C_{12}$aryl, $C_7$-$C_{12}$aralkyl, $C_3$-$C_6$heterocycloalkyl, $C_3$-$C_{12}$heteroaryl, $C_4$-$C_{12}$heteroaralkyl or $R_{13}$-$X_1$- are optionally fused onto adjacent carbon atoms; |
| X and $X_1$ | independently of one another are -O-, -S-, -CO-, -SO-, -$SO_2$-, -O-C(O)-, -C(O)-O-, -C(O)-$NR_5$-, -$NR_{10}$-C(O)-, -$SO_2$-O- or -O-$SO_2$-; and |
| $R_1$, $R_2$ and $R_3$ | independently of one another are $C_1$-$C_5$alkyl, $C_1$-$C_6$perfluoroalkyl, phenyl or benzyl; |
| M | is an alkali metal and $M_1$ is an alkaline earth metal; |
| $R_4$ and $R_{13}$ | independently of one another are $C_1$-$C_{12}$alkyl, $C_1$-$C_{12}$haloalkyl, $C_1$-$C_{12}$hydroxyalkyl, $C_3$-$C_8$cycloalkyl, $C_6$-$C_{12}$aryl or $C_7$-$C_{12}$aralkyl; |
| $R_5$ and $R_{10}$ | independently of one another are hydrogen, $C_1$-$C_6$alkyl, phenyl or benzyl, |

where the alkyl groups in turn are unsubstituted or substituted by $C_1$-$C_6$alkoxy or $C_3$-$C_6$cycloalkyl;

| | |
|---|---|
| $R_6$, $R_7$ and $R_8$ | independently of one another are $C_1$-$C_6$alkyl, $C_1$-$C_6$perfluoroalkyl, phenyl or benzyl; |
| u | is 0 or 1; |

where the alicyclic ring formed with $Q_1$ optionally contains further non-aromatic double bonds;

$Q_2$ is hydrogen, $C_1$-$C_{12}$alkyl, $C_1$-$C_{12}$haloalkyl, $C_1$-$C_6$alkoxy, halogen, -CN or $R_{11}$-$X_2$-,
$R_{11}$ is $C_1$-$C_{12}$alkyl, $C_1$-$C_{12}$haloalkyl, $C_1$-$C_{12}$hydroxyalkyl, $C_3$-$C_6$cycloalkyl, $C_6$-$C_{12}$aryl or $C_7$-$C_{12}$aralkyl;
$X_2$ is -C(O)-O- or -C(O)-$NR_{12}$; and
$R_{12}$ is hydrogen, $C_1$-$C_6$alkyl, phenyl or benzyl;

and where the cycloalkyl, heterocycloalkyl, aryl, heteroaryl, aralkyl and heteroaralkyl groups are unsubstituted or substituted by $C_1$-$C_6$alkyl, $C_1$-$C_6$alkoxy,-$NO_2$, -CN or halogen, and where the heteroatoms of the heterocycloalkyl, heteroaryl and heteroaralkyl groups are chosen from the group consisting of -O-,-S-, -$NR_9$- and -N=; and $R_9$ is hydrogen, $C_1$-$C_6$alkyl, phenyl or benzyl.

**21.** Compositions according to claim 17, characterized in that the radical of a strained cycloolefin corresponds to the formula II, in which

| | |
|---|---|
| $Q_1$ | is a radical with at least one carbon atom which, together with the -CH=C$Q_2$-group, forms a 3- to 10-membered alicyclic ring which optionally contains a heteroatom chosen from the group consisting of silicon, oxygen, nitrogen and sulfur and is unsubstituted or substituted by halogen, -CN, -$NO_2$, $R_1R_2R_3$Si-, -COOM, -$SO_3$M, -$PO_3$M, -COO$(M_1)_{1/2}$, -$SO_3(M_1)_{1/2}$, -$PO_3(M_1)_{1/2}$, $C_1$-$C_6$alkyl, $C_1$-$C_6$haloalkyl, $C_1$-$C_6$hydroxyalkyl, $C_1$-$C_4$cyanoalkyl, $C_3$-$C_6$cycloalkyl, phenyl, benzyl or $R_4$-X-; or in which an aromatic or heteroaromatic ring which is unsubstituted or substituted by halogen, -CN, -$NO_2$, $R_6R_7R_8$Si-, -COOM, -$SO_3$M, -$PO_3$M, -COO$(M_1)_{1/2}$, -$SO_3(M_1)_{1/2}$, -$PO_3(M_1)_{1/2}$, $C_1$-$C_6$alkyl, $C_1$-$C_6$haloalkyl, $C_1$-$C_6$hydroxyalkyl, $C_1$-$C_4$cyanoalkyl, $C_3$-$C_6$cycloalkyl, phenyl, benzyl or $R_{13}$-$X_1$- is optionally fused onto adjacent carbon atoms; |
| $R_1$, $R_2$ and $R_3$ | independently of one another are $C_1$-$C_4$alkyl, $C_1$-$C_4$perfluoroalkyl, phenyl or benzyl; |
| M | is an alkali metal and $M_1$ is an alkaline earth metal; |
| $R_4$ and $R_{13}$ | independently of one another are $C_1$-$C_6$alkyl, $C_1$-$C_6$haloalkyl, $C_1$-$C_6$hydroxyalkyl or $C_3$-$C_6$cycloalkyl; |
| X and $X_1$ | independently of one another are -O-, -S-, -CO-, -SO- or -$SO_2$-; |
| $R_6$, $R_7$ and $R_8$ | independently of one another are $C_1$-$C_4$alkyl, $C_1$-$C_4$perfluoroalkyl, phenyl or benzyl; and |
| $Q_2$ | is hydrogen. |

**22.** Compositions according to claim 17, characterized in that the cycloolefin radical of the formula II is unsubstituted or substituted cyclopropenyl, cyclobutenyl, cyclopentenyl, cycloheptenyl, cyclooctenyl, cyclopentadienyl, cyclohexadienyl, cycloheptadienyl, cyclooctadienyl and norbornenyl or norbornenyl derivatives.

**23.** Compositions according to claim 17, characterized in that the cycloolefin radical of the formula II is a radical of the formula III

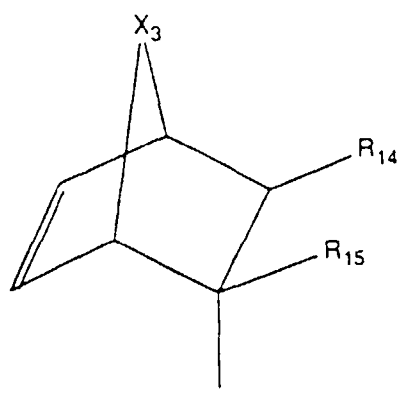

(III),

in which

$X_3$ is -CH$R_{16}$-, oxygen or sulfur;
$R_{14}$ and $R_{15}$ independently of one another are hydrogen, -CN, trifluoromethyl, $(CH_3)_3$Si-O-, $(CH_3)_3$Si- or -COO$R_{17}$; and
$R_{16}$ and $R_{17}$ independently of one another are hydrogen, $C_1$-$C_{12}$-alkyl, phenyl or benzyl;

or of the formula IV

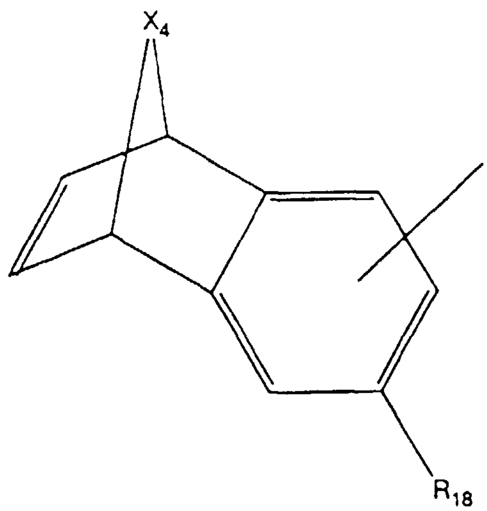

(IV),

in which

$X_4$ is $-CHR_{19}-$, oxygen or sulfur;
$R_{19}$ is hydrogen, $C_1$-$C_{12}$alkyl, phenyl or benzyl; and
$R_{18}$ is hydrogen, $C_1$-$C_6$alkyl or halogen.

**24.** Compositions according to claim 17, characterized in that the cycloolefin radical of the formula II is norbornenyl of the formula

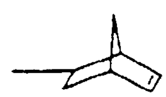

**25.** Composition according to claim 17, characterized in that it comprises, as the one-component catalyst, a heat-stable ruthenium or osmium compound which contains at least one photolabile ligand bonded to the ruthenium or osmium atom, and whose remaining coordination sites are satisfied with non-photolabile ligands.

**26.** Composition according to claim 25, characterized in that the ruthenium and osmium compounds correspond to the formula X

$$[L_1Me)L_8)_5]^{2\oplus}[Y_1^{x\ominus}]_{2/x} \quad \text{(X)},$$

in which $L_1$ is a photolabile ligand and $L_8$ is a non-photolabile ligand, Me is Ru or Os, $Y_1$ is a non-coordinating anion and x is the numbers 1, 2 or 3.

**27.** Composition according to claim 17, characterized in that the one-component catalyst is a molybdenum(VI) or tungsten(VI) compound which contains at least two methyl groups or two monosubstituted methyl groups bonded to the metal, the substituent containing no hydrogen atom in the α position.

**28.** Composition according to claim 27, characterized in that the optionally monosubstituted methyl groups, bonded to the metal, correspond to the formula XI

$$-CH_2-R \quad \text{(XI)},$$

in which R is H, $-CF_3$, $-SiR_{38}R_{39}R_{40}$, $-CR_{41}R_{42}R_{43}$, $C_6$-$C_{16}$aryl which is unsubstituted or substituted by $C_1$-$C_6$alkyl or $C_1$-$C_6$alkoxy or $C_4$-$C_{15}$heteroaryl having 1 to 3 heteroatoms from the group consisting of O, S and N;

$R_{38}$, $R_{39}$ and $R_{40}$ independently of one another are $C_1$-$C_6$alkyl, $C_5$- or $C_6$cycloalkyl or phenyl or benzyl which are unsubstituted or substituted by $C_1$-$C_6$alkyl or $C_1$-$C_6$alkoxy; and
$R_{41}$, $R_{42}$ and $R_{43}$ independently of one another are $C_1$-$C_{10}$alkyl, which is unsubstituted or substituted by $C_1$-$C_{10}$alkoxy, or $R_{41}$ and $R_{42}$ have this meaning and $R_{43}$ is $C_6$-$C_{10}$aryl or $C_4$-$C_9$heteroaryl, which is unsubstituted or substituted by $C_1$-$C_6$alkyl or $C_1$-$C_6$alkoxy.

**29.** Composition according to claim 17, characterized in that the one-component catalyst is a heat-stable titanium(IV), niobium(V), tantalum(V), molybdenum(VI) or tungsten(VI) compound in which a silylmethyl group and at least one halogen are bonded to the metal.

**30.** Composition according to claim 29, characterized in that the silylmethyl group corresponds to the formula XIV

$$-CH_2-SiR_{38}R_{39}R_{40} \qquad (XIV),$$

in which

$R_{38}$, $R_{39}$ and $R_{40}$ independently of one another are $C_1$-$C_{18}$-alkyl, $C_5$- or $C_6$cycloalkyl, or phenyl or benzyl which are unsubstituted or substituted by $C_1$-$C_6$alkyl or $C_1$-$C_6$alkoxy.

**31.** Composition according to claim 17, characterized in that the one-component catalyst is a niobium(V) or tantalum (V) compound which contains at least two methyl groups or two monosubstituted methyl groups bonded to the metal, the substituent containing no hydrogen atom in the $\alpha$ position.

**32.** Composition according to claim 31, characterized in that the optionally monosubstituted methyl groups correspond to the formula XI

$$-CH_2-R \qquad (XI)$$

in which R is H, $-CF_3$, $-SiR_{38}R_{39}R_{40}$, $-CR_{41}R_{42}R_{43}$, $C_6$-$C_{16}$aryl which is unsubstituted or substituted by $C_1$-$C_6$alkyl or $C_1$-$C_6$alkoxy or $C_4$-$C_{15}$heteroaryl which has 1 to 3 heteroatoms from the group consisting of O, S and N;

$R_{38}$, $R_{39}$ and $R_{40}$ independently of one another are $C_1$-$C_{12}$alkyl, $C_5$- or $C_6$cycloalkyl or phenyl or benzyl which are unsubstituted or substituted by $C_1$-$C_6$alkyl or $C_1$-$C_6$alkoxy, and
$R_{41}$, $R_{42}$ and $R_{43}$ independently of one another are $C_1$-$C_{10}$alkyl, which is unsubstituted or substituted by $C_1$-$C_{10}$alkoxy, or $R_{41}$ and $R_{42}$ have this meaning and $R_{43}$ is $C_6$-$C_{10}$aryl or $C_4$-$C_9$heteroaryl, which is unsubstituted or substituted by $C_1$-$C_6$alkyl or $C_1$-$C_6$alkoxy.

**33.** Composition according to claim 17, characterized in that the one-component catalyst is a titanium(IV) compound which contains at least two methyl groups or two monosubstituted methyl groups bonded to the metal, the substituent containing no hydrogen atom in the $\alpha$ position.

**34.** Composition according to claim 33, characterized in that the optionally monosubstituted methyl groups correspond to the formula XI

$$-CH_2-R \qquad (XI)$$

in which R is H, $-CF_3$, $-SiR_{38}R_{39}R_{40}$, $-CR_{41}R_{42}R_{43}$, $C_6$-$C_{16}$aryl which is unsubstituted or substituted by $C_1$-$C_6$alkyl or $C_1$-$C_6$alkoxy or $C_4$-$C_{15}$heteroaryl which has 1 to 3 heteroatoms from the group consisting of O, S and N;

$R_{38}$, $R_{39}$ and $R_{40}$ independently of one another are $C_1$-$C_{12}$alkyl, $C_5$- or $C_6$cycloalkyl or phenyl or benzyl which are unsubstituted or substituted by $C_1$-$C_6$alkyl or $C_1$-$C_6$alkoxy, and

$R_{41}$, $R_{42}$ and $R_{43}$ independently of one another are $C_1$-$C_{10}$alkyl, which is unsubstituted or substituted by $C_1$-$C_{10}$alkoxy, or $R_{41}$ and $R_{42}$ have this meaning and $R_{43}$ is $C_6$-$C_{10}$aryl or $C_4$-$C_9$heteroaryl, which is unsubstituted or substituted by $C_1$-$C_6$alkyl or $C_1$-$C_6$alkoxy.

**35.** Composition according to claim 17, characterized in that the one-component catalyst is a ruthenium or osmium compound which contains at least one phosphine group, at least one photolabile ligand, and optionally neutral ligands bonded to the metal atom, a total of 2 to 5 ligands being bonded, and which contains acid anions for charge balancing.

**36.** Composition according to claim 35, characterized in that the phosphine ligands correspond to the formulae XXIII or XXIIIa.

$$PR_{91}R_{92}R_{93} \quad \text{(XXIII)},$$

$$R_{91}R_{92}P\text{-}Z_1\text{-}PR_{91}R_{92} \quad \text{(XXIIIa)},$$

in which $R_{91}$, $R_{92}$ and $R_{93}$ independently of one another are H, $C_1$-$C_{20}$alkyl, $C_4$-$C_{12}$cycloalkyl which is unsubstituted or substituted by $C_1$-$C_6$alkyl, $C_1$-$C_6$haloalkyl or $C_1$-$C_6$alkoxy, or $C_6$-$C_{16}$aryl which is unsubstituted or substituted by $C_1$-$C_6$alkyl, $C_1$-$C_6$haloalkyl or $C_1$-$C_6$alkoxy, or $C_7$-$C_{16}$aralkyl which is unsubstituted or substituted by $C_1$-$C_6$alkyl, $C_1$-$C_6$haloalkyl or $C_1$-$C_6$alkoxy; the radicals $R_{91}$ and $R_{92}$ together are tetra- or pentamethylene, which is unsubstituted or substituted by $C_1$-$C_6$alkyl, $C_1$-$C_6$haloalkyl or $C_1$-$C_6$alkoxy, or tetra- or pentamethylene, which is unsubstituted or substituted by $C_1$-$C_6$alkyl, $C_1$-$C_6$haloalkyl or $C_1$-$C_6$alkoxy and fused with 1 or 2 1,2-phenylene, and $R_{93}$ has the meaning given above; and

$Z_1$ is linear or branched $C_2$-$C_{12}$alkylene which is unsubstituted or substituted by $C_1$-$C_4$alkoxy, 1,2- or 1,3-cycloalkylene which has 4 to 8 C atoms and is unsubstituted or substituted by $C_1$-$C_4$alkyl or $C_1$-$C_4$alkoxy, or 1,2 or 1,3-heterocycloalkylene which has 5 or 6 ring members and one heteroatom from the group consisting of O or N and is unsubstituted or substituted by $C_1$-$C_4$alkyl or $C_1$-$C_4$alkoxy.

**37.** Composition according to claim 17, characterized in that the one-component catalyst is a divalent-cationic ruthenium or osmium compound with a metal atom to which are bonded, 1 to 3 tertiary phosphine ligands with, in the case of the ruthenium compounds, sterically exacting substituents, optionally non-photolabile neutral ligands and anions for charge balancing, with the proviso that, in ruthenium (trisphenylphosphine) dihalides or hydride-halides, the phenyl groups are substituted by $C_1$-$C_{18}$alkyl, $C_1$-$C_{18}$haloalkyl or $C_1$-$C_{18}$alkoxy.

**38.** Composition according to claim 37, characterized in that the phosphine ligands correspond to the formulae XXIII or XXIIIa

$$PR_{91}R_{92}R_{93} \quad \text{(XXIII)},$$

$$R_{91}R_{92}P\text{-}Z_1\text{-}PR_{91}R_{92} \quad \text{(XXIIIa)},$$

in which $R_{91}$, $R_{92}$ and $R_{93}$ independently of one another are H, $C_1$-$C_{20}$alkyl, $C_4$-$C_{12}$cycloalkyl which is unsubstituted or substituted by $C_1$-$C_6$alkyl, $C_1$-$C_6$haloalkyl or $C_1$-$C_6$alkoxy, or $C_6$-$C_{16}$aryl which is unsubstituted or substituted by $C_1$-$C_6$alkyl, $C_1$-$C_6$haloalkyl or $C_1$-$C_6$alkoxy, or $C_7$-$C_{16}$aralkyl which is unsubstituted or substituted by $C_1$-$C_6$alkyl, $C_1$-$C_6$haloalkyl or $C_1$-$C_6$alkoxy; the radicals $R_{91}$ and $R_{92}$ together are tetra- or pentamethylene, which is unsubstituted or substituted by $C_1$-$C_6$alkyl, $C_1$-$C_6$haloalkyl or $C_1$-$C_6$alkoxy, or tetra- or pentamethylene, which is unsubstituted or substituted by $C_1$-$C_6$alkyl, $C_1$-$C_6$haloalkyl or $C_1$-$C_6$alkoxy and fused with 1 or 2 1,2-phenylene, and $R_{93}$ has the meaning given above; and

$Z_1$ is linear or branched $C_2$-$C_{12}$alkylene which is unsubstituted or substituted by $C_1$-$C_4$alkoxy, 1,2- or 1,3-cycloalkylene which has 4 to 8 C atoms and is unsubstituted or substituted by $C_1$-$C_4$alkyl or $C_1$-$C_4$alkoxy, or 1,2 or 1,3-heterocycloalkylene which has 5 or 6 ring members and one heteroatom from the group consisting of

O or N and is unsubstituted or substituted by $C_1$-$C_4$alkyl or $C_1$-$C_4$alkoxy.

**39.** Composition according to claim 17, characterized in that the one-component catalyst is present in an amount of from 0.001 to 20 mol%, based on the amount of the monomer.

**40.** Process for the preparation of crosslinked polymers by metathesis polymerization according to claim 1, characterized in that a composition of

(a) at least one compound of the formula I and
(b) a catalytic amount of at least one one-component catalyst for a metathesis polymerization which can be activated by heat or radiation,
(c) is subjected to polymerization by heating,
(d) is subjected to polymerization by irradiation,
(e) is subjected to polymerization by heating and irradiation,
(f) the one-component catalyst is activated by brief heating and the polymerization is ended by irradiation, or
(g) the one-component catalyst is activated by brief irradiation and the polymerization is ended by heating.

**41.** Process according to claim 40, characterized in that it is carried out at a temperature of 20 to 300°C.

**42.** Crosslinked metathesis polymers of at least one compound (I) according to claim 1.

**43.** Coated carrier material, characterized in that a layer of a composition according to claim 17 is applied to a substrate.

**44.** Coated substrate with a cured layer of a composition according to claim 17.

**Revendications**

**1.** Composés de formule I

$$(A)_n\text{-}B \qquad (I)$$

où A représente le reste d'une cyclooléfine tendue, n vaut 2 et B représente une liaison directe ou un groupe pontal de formule V

$$-X_5\text{-}R_{20}\text{-}X_6- \qquad (V)$$

dans laquelle

$X_5$ et $X_6$ représentent, indépendamment l'un de l'autre, une liaison directe, -O-, $-CH_2$-O-, -C(O)O-, -O((O)C-, $-CH_2$-O(O)C-, -C(O)-$NR_{21}$-, $R_{21}$N-(O)C-, -NH-C(O)-$NR_{21}$-, -O-C(O)-NH-, $-CH_2$-O-C(O)-NH- ou -NH-C(O)-O-, et
$R_{20}$ représente des groupes alkylène en $C_2$-$C_{18}$, cycloalkylène en $C_5$-$C_8$ non substitué ou substitué par des substituants alkyle en $C_1$-$C_4$ ou alkoxy en $C_1$-$C_4$, arylène en $C_6$-$C_{18}$ ou aralkylène en $C_7$-$C_{19}$ non substitué ou substitué par des groupes alkyle en $C_1$-$C_4$ ou alkoxy en $C_1$-$C_4$, ou polyoxaalkylène comportant de 2 à 12 unités oxaalkylène et 2 à 6 atomes de carbone dans le fragment alkylène, et
$R_{21}$ représente un atome d'hydrogène ou un groupe alkyle en $C_1$-$C_6$,

à l'exception du 1,2-bis-norbornényle-éthane, l'ester norbornène méthylique de l'acide norbornène carboxylique et des composés de formule

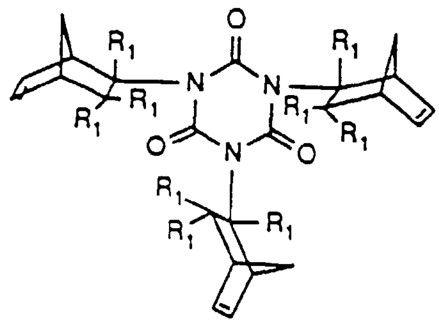

dans laquelle $R_1$ représente des atomes d'hydrogène ou des groupes alkyle.

**2.** Composés selon la revendication 1, caractérisés en ce que dans la formule V

a) $X_5$ et $X_6$ représentent une liaison directe et $R_{20}$ représente un groupe alkylène en $C_2$-$C_{18}$, ou
b) $X_5$ et $X_6$ représentent -O-, -$CH_2$-O-, -C(O)O-, -O((O)C-, -$CH_2$-O(O)C-, -C(O)-$NR_{21}$-, -O-C(O)-NH- ou -$CH_2$-O-C(O)-NH- et $R_{20}$ représente des groupes alkylène en $C_2$-$C_{12}$, phénylène, naphtylène ou benzylène non substitué ou substitué par des substituants alkyle en $C_1$-$C_4$ ou alkoxy en $C_1$-$C_4$, ou -$R_{22}$-(O-$R_{22}$-)$_x$-$OR_{22}$-, où x va de 2 à 4 et $R_{22}$ représente alkylène en $C_2$-$C_4$.

**3.** Composés selon la revendication 1, caractérisés en ce qu'ils sont pris dans le groupe des composés suivants

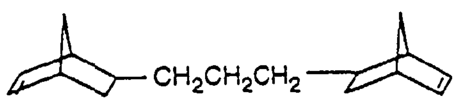

(1),

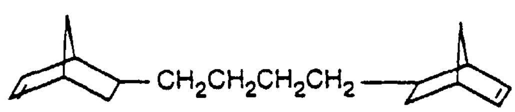

(2),

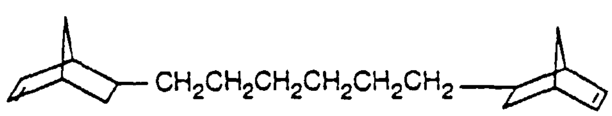

(3),

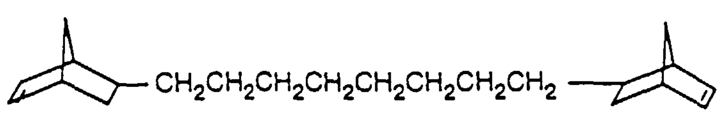

(4),

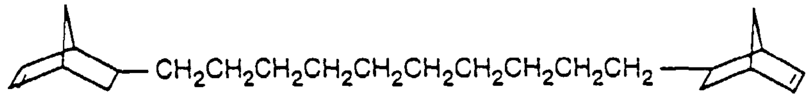
(5),

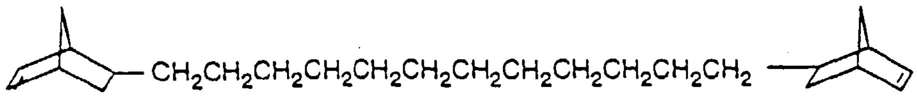
(6)

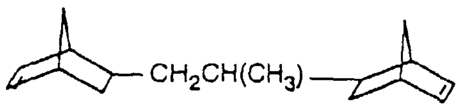
(7),

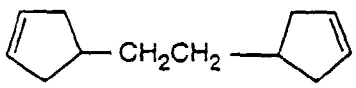
(8),

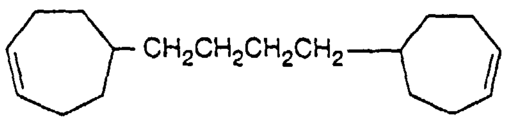
(9),

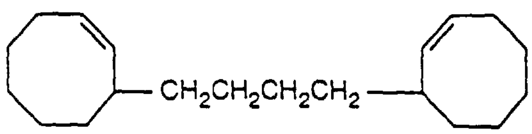
(10),

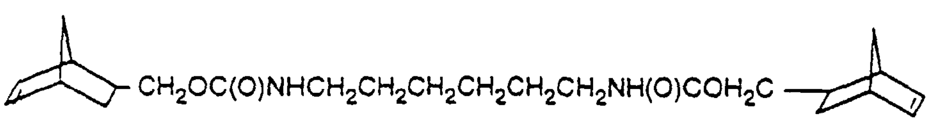
(11),

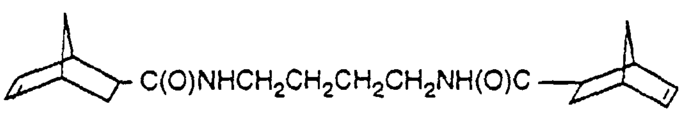
(12),

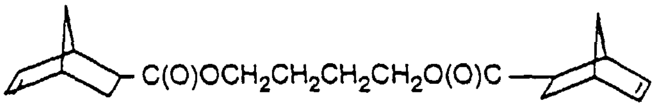

(13),

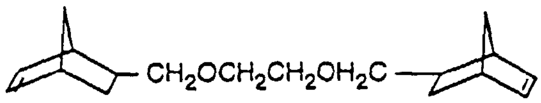

(14),

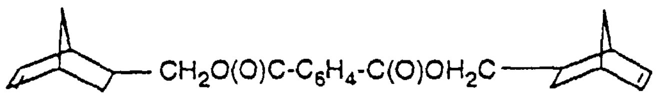

(15),

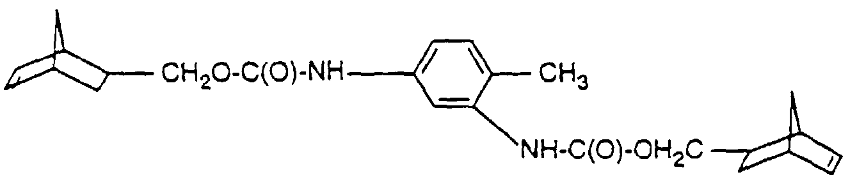

(16)

et

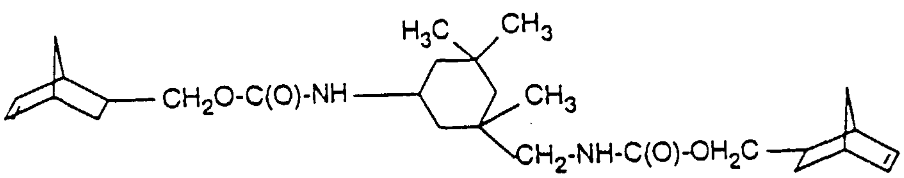

(17).

4. Composés selon la revendication 1, caractérisés en ce que le groupe pontal répond à la formule VI

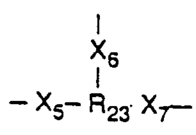

(VI),

dans laquelle

$X_5$, $X_6$ et $X_7$ représentent -O-, $-CH_2-O-$, -C(O)O-, -O(O)C-, $-CH_2-O(O)C-$, $-C(O)-NR_{21}-$, $R_{21}N-(O)C-$, $-NH-C(O)-NR_{21}-$, -O-C(O)-NH-, $-CH_2-O-C(O)-NH-$ ou -NH-C(O)-O-, et

$R_{23}$ représente un reste hydrocarboné aliphatique trifonctionnel comportant 3 à 20 atomes de carbone, un reste cycloaliphatique trifonctionnel comportant 3 à 8 atomes de carbone de cycle non substitué ou substitué par des substituants alkyle en $C_1$-$C_4$ ou alkoxy en $C_1$-$C_4$, ou un reste aromatique trifonctionnel comportant 6 à 18 atomes de carbone non substitué ou substitué par

des substituants alkyle en $C_1$-$C_4$ ou alkoxy en $C_1$-$C_4$, ou un reste araliphatique trifonctionnel comportant 7 à 19 de carbone non substitué ou substitué par des substituants alkyle en $C_1$-$C_4$ ou alkoxy en $C_1$-$C_4$, ou un reste hétéroaromatique trifonctionnel comportant de 3 à 13 atomes de carbone et 1 à 3 hétéroatomes du groupe des atomes -O-, -N- et -S-, non substitué ou substitué par des substituants alkyle en $C_1$-$C_4$ ou alkoxy en $C_1$-$C_4$, et

$R_{21}$ représente un atome d'hydrogène ou un groupe alkyle en $C_1$-$C_6$.

**5.** Composés selon la revendication 4, caractérisés en ce que $X_5$, $X_6$ et $X_7$ représentent -O-, -$CH_2$-O-, -C(O)O-, -O(O)C-, -$CH_2$-O(O)C-, -C(O)-$NR_{21}$-, -$CH_2$-O-C(O)-NH- ou -NH-C(O)-O-.

**6.** Composés selon la revendication 4, caractérisés en ce que les restes $R_{23}$ dérivent des triols ; de l'acide cyanurique ; des triamines ; des acides tricarboxyliques ou des triisocyanates.

**7.** Composés selon la revendication 4, caractérisés en ce qu'ils sont pris dans le groupe des composés suivants

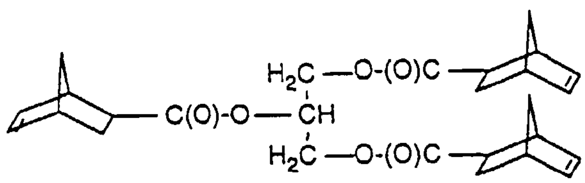

(18),

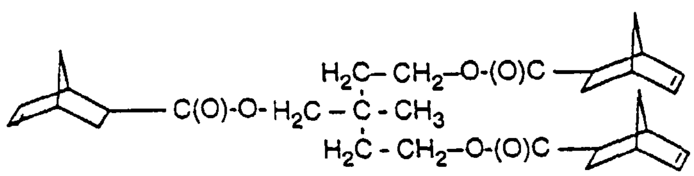

(19),

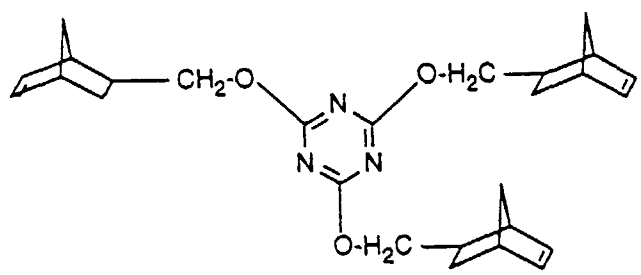

(20),

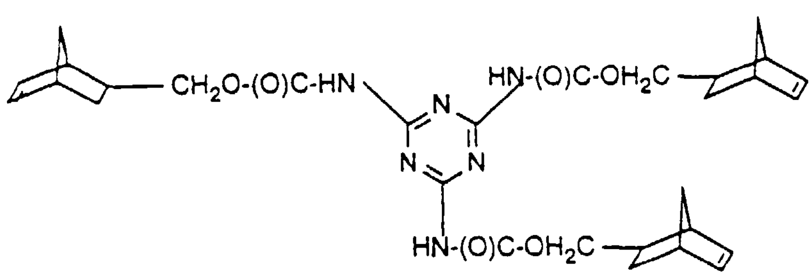

(21)

et

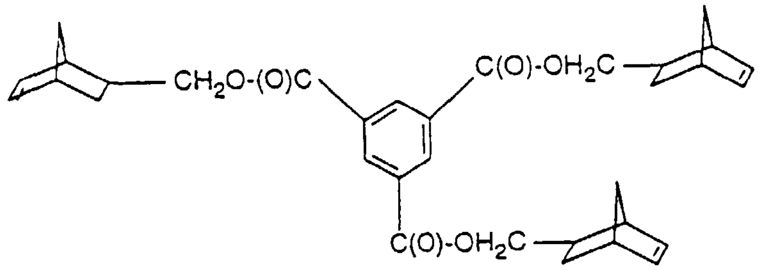

(22).

8. Composés selon la revendication 1, caractérisés en ce que le groupe pontal répond à la formule VII

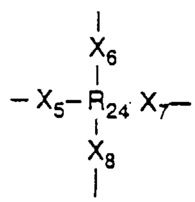

(VII),

dans laquelle

$X_5$, $X_6$, $X_7$ et $X_8$ représentent -C(O)O-, $-CH_2$-O(O)C- ou -C(O)-$NR_{21}$-, et

$R_{24}$ représente un reste hydrocarboné aliphatique tétrafonctionnel comportant 4 à 20 atomes de carbone, un reste cycloaliphatique comportant 4 à 8 atomes de carbone de cycle non substitué ou substitué par des substituants alkyle en $C_1$-$C_4$ ou alkoxy en $C_1$-$C_4$, ou un reste aromatique tétrafonctionnel comportant 6 à 18 atomes de carbone non substitué ou substitué par des substituants alkyle en $C_1$-$C_4$ ou alkoxy en $C_1$-$C_4$, un reste araliphatique tétrafonctionnel comportant 7 à 19 atomes de carbone non substitué ou substitué par des substituants alkyle en $C_1$-$C_4$ ou alkoxy en $C_1$-$C_4$, ou un reste hétéroaromatique tétra-fonctionnel comportant 3 à 13 atomes de carbone et 1 à 3 hétéroatomes pris parmi les atomes de -O-, -N- et -S-, non substitué ou substitué par des substituants alkyle en $C_1$-$C_4$ ou alkoxy en $C_1$-$C_4$, et

$R_{21}$ représente un atome d'hydrogène ou un groupe alkyle en $C_1$-$C_6$.

9. Composés selon la revendication 8, caractérisés en ce que les restes $R_{24}$ dérivent du pentaérythritol, de l'acide pyromellithique ou de l'acide 3,4,3',4'-biphényltétracarboxylique.

10. Composés selon la revendication 8, caractérisés en ce qu'ils sont

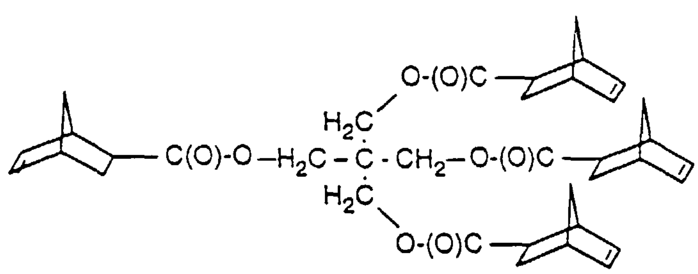

(23)

ou

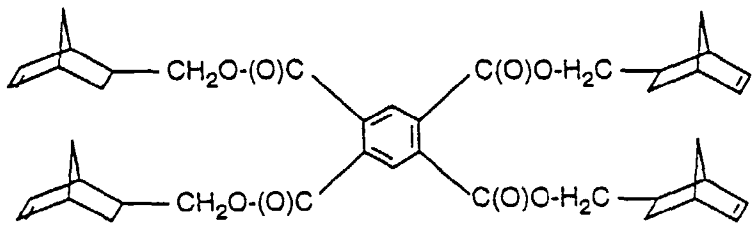

(24)

**11.** Composés selon la revendication 1, caractérisés en ce que les composés de formule I contiennent uniquement des atomes de carbone et des atomes d'hydrogène.

**12.** Composition à partir de

(a) au moins un composé de formule I

$$(A)_n\text{-}B \qquad (I)$$

dans laquelle A représente le reste d'une cyclooléfine tendue, B représente une liaison directe ou un groupe pontal à fonctionnalité n, et n est un nombre entier de 2 à 8,
b) une quantité catalytique d'un catalyseur à un composant activable par la chaleur ou par un rayonnement pour une polymérisation par métathèse, à l'exception de l'ester norbornène méthylique de l'acide norbornène carboxylique de formule

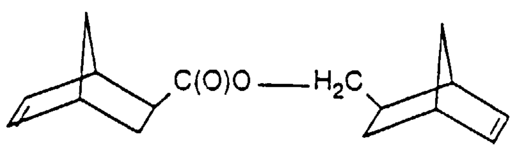

en combinaison avec une quantité catalytique d'au moins un composé de molybdène(VI) ou de tungstène(VI) stable à chaleur, lequel contient au moins deux groupes méthyle ou deux groupes méthyle monosubstitué liés au métal, le substituant ne présentant pas d'atome d'hydrogène en position α.

**13.** Compositions selon la revendication 12, caractérisées en ce que pour les cyclooléfines tendues, il s'agit de systèmes cycliques monocycliques ou polycycliques condensés et/ou pontés, lesquels sont non substitués ou substitués et peuvent contenir des hétéroatomes pris parmi les atomes de O, S, N ou Si dans un ou plusieurs cycles et/ou des cycles condensés alicycliques, aromatiques ou hétéroaromatiques.

**14.** Compositions selon la revendication 13, caractérisées en ce que les différents cycles contiennent 3 à 16 chaînons.

**15.** Compositions selon la revendication 13, caractérisées en ce que les cycles cycliques contiennent 3 à 12 chaînons.

**16.** Compositions selon la revendication 13, caractérisées en ce que les cycles cycliques contiennent 3 à 8 chaînons.

**17.** Compositions selon la revendication 12, caractérisées en ce que le reste d'une cyclooléfine tendue répond à la formule II

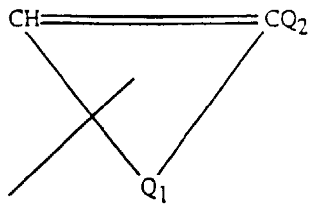

(II),

dans laquelle

Q$_1$ représente un reste avec au moins un atome de carbone, lequel forme, conjointement avec le groupe -CH=CQ$_2$-, un cycle alicyclique comportant au moins 3 chaînons, lequel comporte éventuellement un ou plusieurs hétéroatomes pris dans le groupe constitué par des atomes de silicium, de phosphore, d'oxygène, d'azote et de soufre ; et lequel est non substitué ou substitué par des atomes d'halogènes, des substituants =O, -CN, -NO$_2$, R$_1$R$_2$R$_3$Si-(O)$_u$-, -COOM, -SO$_3$M, -PO$_3$M, -COO(M$_1$)$_{1/2}$, -SO$_3$(M$_1$)$_{1/2}$, -PO$_3$(M$_1$)$_{1/2}$, alkyle en C$_1$-C$_{20}$, hydroxyalkyle en C$_1$-C$_{20}$, haloalkyle en C$_1$-C$_{20}$, cyanoalkyle en C$_1$-C$_6$, cycloalkyle en C$_3$-C$_8$, aryle en C$_6$-C$_{16}$, aralkyle en C$_7$-C$_{16}$, hétérocycloalkyle en C$_3$-C$_6$, hétéroaryle en C$_3$-C$_{16}$, hétéroaralkyle en C$_4$-C$_{16}$ ou R$_4$-X ; ou dans lequel deux atomes de carbone adjacents sont substitués par -CO-O-CO- ou -CO-NR$_5$-CO- ; ou dans lequel éventuellement, sur des atomes de carbone adjacents du cycle alicyclique, sont condensés un cycle aromatique ou hétéroaromatique et/ou autres cycles alicycliques, lesquels sont non substitués ou substitués par des atomes d'halogènes, des substituants -CN, -NO$_2$, R$_6$R$_7$R$_8$Si-(O)$_u$-, -COOM, -SO$_3$M, -PO$_3$M, -COO(M$_1$)$_{1/2}$, -SO$_3$(M$_1$)$_{1/2}$, -PO$_3$(M$_1$)$_{1/2}$, alkyle en C$_1$-C$_{20}$, haloalkyle en C$_1$-C$_{20}$, hydroxyalkyle en C$_1$-C$_{20}$, cyanoalkyle en C$_1$-C$_6$, cycloalkyle en C$_3$-C$_8$, aryle en C$_6$-C$_{16}$, aralkyle en C$_7$-C$_{16}$, hétérocycloalkyle en C$_3$-C$_6$, hétéroaryle en C$_3$-C$_{16}$, hétéroaralkyle en C$_4$-C$_{16}$ ou R$_{13}$-X$_1$ ;

X et X$_1$ représentent, indépendamment l'un de l'autre, -O-, -S-, -CO-, -SO-, -SO$_2$-, -O-C(O)-, -C(O)-O-, -C(O)-NR$_5$-, -NR$_{10}$-C(O)-, -SO$_2$-O- ou -O-SO$_2$- ;

R$_1$, R$_2$ et R$_3$ représentent indépendamment les uns des autres des groupes alkyle en C$_1$-C$_{12}$, perfluoroalkyle en C$_1$-C$_{12}$, phényle ou benzyle ;

R$_4$ et R$_{13}$ représentent indépendamment l'un de l'autre, des groupes alkyle en C$_1$-C$_{20}$, haloalkyle en C$_1$-C$_{20}$, hydroxyalkyle en C$_1$-C$_{20}$, cycloalkyle en C$_3$-C$_8$, aryle en C$_6$-C$_{16}$, aralkyle en C$_7$-C$_{16}$ ;

R$_5$ et R$_{10}$ représentent indépendamment l'un de l'autre des atomes d'hydrogène, des groupes alkyle en C$_1$-C$_{12}$, phényle ou benzyle, les groupes alkyle, de leur côté, pouvant être non substitués ou substitués par des groupes alkoxy en C$_1$-C$_{12}$ ou cycloalkyle en C$_3$-C$_8$ ;

R$_6$, R$_7$ et R$_8$ représentent indépendamment les uns des autres des groupes alkyle en C$_1$-C$_{12}$, perfluoroalkyle en C$_1$-C$_{12}$, phényle ou benzyle ;

M représente un métal alcalin et M$_1$ un métal alcalino-terreux ; et

u vaut 0 ou 1 ;

le cycle alicyclique formé par Q$_1$ contenant éventuellement d'autres doubles liaisons non aromatiques ;

Q$_2$ représente des atomes d'hydrogène, des groupes alkyle en C$_1$-C$_{20}$, haloalkyle en C$_1$-C$_{20}$, alkoxy en C$_1$-C$_{12}$, halogène, -CN ou R$_{11}$-X$_2$- ;

R$_{11}$ représente des groupes alkyle en C$_1$-C$_{20}$, haloalkyle en C$_1$-C$_{20}$, hydroxyalkyle en C$_1$-C$_{20}$, cycloalkyle en C$_3$-C$_8$, aryle en C$_6$-C$_{16}$ ou aralkyle en C$_7$-C$_{16}$ ;

X$_2$ représente -C(O)-O- ou -C(O)-NR$_{12}$- ;

R$_{12}$ représente des atomes d'hydrogène, groupes alkyle en C$_1$-C$_{12}$, perfluoroalkyle en C$_1$-C$_{12}$, phényle ou benzyle ;

les groupes cycloalkyle, hétérocycloalkyle, aryle, hétéroaryle, aralkyle et hétéroaralkyle précités pouvant être non substitués ou substitués par des substituants alkyle en C$_1$-C$_{12}$, alkoxy en C$_1$-C$_{12}$, -NO$_2$, -CN ou halogène, et les hétéroatomes des groupes hétérocycloalkyle, hétéroaryle et hétéroaralkyle étant pris dans le groupe comportant -O-, -S-, -NR$_9$- et -N= ; et

$R_9$ représente des atomes d'hydrogène, des groupes alkyle en $C_1$-$C_{12}$, phényle ou benzyle.

**18.** Compositions selon la revendication 17, caractérisées en ce que dans le formule II, $Q_2$ représente un atome d'hydrogène.

**19.** Compositions selon la revendication 17, caractérisées en ce que dans la formule II, le cycle alicyclique, lequel est formé par $Q_1$ conjointement avec le groupe -CH=$CQ_2$, présente 3 à 8 atomes de carbone, s'agissant d'un système cyclique monocyclique, bicyclique, tricyclique ou tétracyclique.

**20.** Compositions selon la revendication 17, caractérisées en ce que le reste d'une cyclooléfine tendue répond à la formule II, où

$Q_1$ représente un reste avec au moins un atome de carbone, lequel reste forme conjointement avec le groupe -CH=$CQ_2$- un cycle alicyclique de 3 à 20 chaînons, lequel peut éventuellement comporter un ou plusieurs hétéroatomes pris dans le groupe constitué par les atomes de silicium, d'oxygène, d'azote et de soufre ; et lequel est non substitué ou substitué par des atomes d'halogènes, des substituants =O, -CN, -$NO_2$, $R_1R_2R_3Si$-$(O)_u$-, -COOM, -$SO_3M$, -$PO_3M$, -$COO(M_1)_{1/2}$, -$SO_3(M_1)_{1/2}$, -$PO_3(M_1)_{1/2}$, alkyle en $C_1$-$C_{12}$, haloalkyle en $C_1$-$C_{12}$, hydroxyalkyle en $C_1$-$C_{12}$, cyanoalkyle en $C_1$-$C_4$, cycloalkyle en $C_3$-$C_6$, aryle en $C_6$-$C_{12}$, aralkyle en $C_7$-$C_{12}$, hétérocycloalkyle en $C_3$-$C_6$, hétéroaryle en $C_3$-$C_{12}$, hétéroaralkyle en $C_4$-$C_{12}$ ou $R_4$-X ; ou dans lequel deux atomes de carbone adjacents dans ce reste $Q_1$ sont substitués par -CO-O-CO- ou -CO-$NR_5$-CO- ; ou dans lequel éventuellement sur des atomes de carbone adjacents sont condensés un cycle aromatique ou hétéroaromatique et/ou d'autres cycles alicycliques, lesquels sont non substitués ou substitués par des atomes d'halogènes, des substituants -CN, -$NO_2$, $R_6R_7R_8Si$-, -COOM, -$SO_3M$, -$PO_3M$, -$COO(M_1)_{1/2}$, -$SO_3(M_1)_{1/2}$, -$PO_3(M_1)_{1/2}$, alkyle en $C_1$-$C_{12}$, haloalkyle en $C_1$-$C_{12}$, hydroxyalkyle en $C_1$-$C_{12}$, cyanoalkyle en $C_1$-$C_4$, cycloalkyle en $C_3$-$C_6$, aryle en $C_6$-$C_{12}$, aralkyle en $C_7$-$C_{12}$, hétérocycloalkyle en $C_3$-$C_6$, hétéroaryle en $C_3$-$C_{12}$, hétéroaralkyle en $C_4$-$C_{12}$ ou $R_{13}$-$X_1$ ;

X et $X_1$ représentent indépendamment l'un de l'autre -O-, -S-, -CO-, -SO-, -$SO_2$-, -O-C(O)-, -C(O)-O-, -C(O)-$NR_5$-, -$NR_{10}$-C(O)-, -$SO_2$-O- ou -O-$SO_2$- ; et

$R_1$, $R_2$ et $R_3$ représentent indépendamment les uns des autres des groupes alkyle en $C_1$-$C_6$, perfluoroalkyle en $C_1$-$C_6$, phényle ou benzyle ;

M représente un métal alcalin et $M_1$ un métal alcalino-terreux ; et

$R_4$ et $R_{13}$ représentent indépendamment l'un de l'autre, des groupes alkyle en $C_1$-$C_{12}$, haloalkyle en $C_1$-$C_{12}$, hydroxyalkyle en $C_1$-$C_{12}$, cycloalkyle en $C_3$-$C_8$, aryle en $C_6$-$C_{12}$, aralkyle en $C_7$-$C_{12}$ ;

$R_5$ et $R_{10}$ représentent indépendamment l'un de l'autre des atomes d'hydrogène, des groupes alkyle en $C_1$-$C_6$, phényle ou benzyle,

les groupes alkyle, de leur côté, pouvant être non substitués ou substitués par des groupes alkoxy en $C_1$-$C_6$ ou cycloalkyle en $C_3$-$C_6$ ;

$R_6$, $R_7$ et $R_8$ représentent indépendamment les uns des autres des groupes alkyle en $C_1$-$C_6$, perfluoroalkyle en $C_1$-$C_6$, phényle ou benzyle ;

u vaut 0 ou 1 ;

le cycle alicyclique formé par $Q_1$ contenant éventuellement d'autres doubles liaisons non aromatiques ;

$Q_2$ représente un atome d'hydrogène, des groupes alkyle en $C_1$-$C_{12}$, haloalkyle en $C_1$-$C_{12}$, alkoxy en $C_1$-$C_6$, des atomes d'halogène, des groupes -CN ou $R_{11}$-$X_2$- ;

$R_{11}$ représente des groupes alkyle en $C_1$-$C_{12}$, haloalkyle en $C_1$-$C_{12}$, hydroxyalkyle en $C_1$-$C_{12}$, cycloalkyle en $C_3$-$C_6$, aryle en $C_6$-$C_{12}$ ou aralkyle en $C_7$-$C_{12}$ ;

$X_2$ représente -C(O)-O- ou -C(O)-$NR_{12}$- ; et

$R_{12}$ représente des atomes d'hydrogène, groupes alkyle en $C_1$-$C_6$, perfluoroalkyle en $C_1$-$C_{12}$, phényle ou benzyle ;

les groupes cycloalkyle, hétérocycloalkyle, aryle, hétéroaryle, aralkyle et hétéroaralkyle précités pouvant être non substitués ou substitués par des substituants alkyle en $C_1$-$C_6$, alkoxy en $C_1$-$C_6$, -$NO_2$, -CN ou des atomes d'halogènes, et les hétéroatomes des groupes hétérocycloalkyle, hétéroaryle et hétéroaralkyle étant pris dans le groupe comportant -O-, -S-, -$NR_9$- et -N= ; et

$R_9$ représente des atomes d'hydrogène, des groupes alkyle en $C_1$-$C_6$, phényle ou benzyle.

**21.** Compositions selon la revendication 17, caractérisées en ce que le reste d'une cyclooléfine tendue répond à la formule II, où

| | |
|---|---|
| $Q_1$ | représente un reste avec au moins un atome de carbone, lequel forme, conjointement avec le groupe $-CH{=}CQ_2-$, un cycle alicyclique de 3 à 10 chaînons, lequel peut éventuellement comporter un ou hétéroatome pris dans le groupe constitué par des atomes de silicium, d'oxygène, d'azote et de soufre, et lequel est non substitué ou substitué par des atomes d'halogènes, des substituants -CN, $-NO_2$, $R_1R_2R_3Si-$, -COOM, $-SO_3M$, $-PO_3M$, $-COO(M_1)_{1/2}$, $-SO_3(M_1)_{1/2}$, $-PO_3(M_1)_{1/2}$, alkyle en $C_1$-$C_6$, haloalkyle en $C_1$-$C_6$, hydroxyalkyle en $C_1$-$C_6$, cyanoalkyle en $C_1$-$C_4$, cycloalkyle en $C_3$-$C_6$, phényle, benzyle ou $R_4$-X ; ou dans lequel sur deux atomes de carbone adjacents est éventuellement condensé un cycle aromatique ou hétéroaromatique lequel est non substitué ou substitué par des atomes d'halogènes, des substituants -CN, $-NO_2$, $R_6R_7R_8Si-$, -COOM, $-SO_3M$, $-PO_3M$, $-COO(M_1)_{1/2}$, $-SO_3(M_1)_{1/2}$, $-PO_3(M_1)_{1/2}$, alkyle en $C_1$-$C_6$, hydroxyalkyle en $C_1$-$C_6$, haloalkyle en $C_1$-$C_6$, cyanoalkyle en $C_1$-$C_4$, cycloalkyle en $C_3$-$C_6$, phényle, benzyle ou $R_{13}$-$X_1$ ; |
| $R_1$, $R_2$ et $R_3$ | représentent indépendamment les uns des autres des groupes alkyle en $C_1$-$C_4$, perfluoroalkyle en $C_1$-$C_4$, phényle ou benzyle ; |
| M | représente un métal alcalin et $M_1$ un métal alcalino-terreux ; et |
| $R_4$ et $R_{13}$ | représentent indépendamment l'un de l'autre, des groupes alkyle en $C_1$-$C_6$, haloalkyle en $C_1$-$C_6$, hydroxyalkyle en $C_1$-$C_6$ ou cycloalkyle en $C_3$-$C_6$ ; |
| X et $X_1$ | représentent indépendamment l'un de l'autre -O-, -S-, -CO-, -SO- ou $-SO_2-$ ; |
| $R_6$, $R_7$ et $R_8$ | représentent indépendamment les uns des autres des groupes alkyle en $C_1$-$C_4$, perfluoroalkyle en $C_1$-$C_4$, phényle ou benzyle ; |
| $Q_2$ | représente un atome d'hydrogène. |

**22.** Compositions selon la revendication 17, caractérisées en ce que, pour le reste cyclooléfine de formule II, il s'agit de cyclopropényle, cyclobutényle, cyclopentényle, cycloheptényle, cyclooctényle, cyclopentadiényle, cyclohexadiényle, cycloheptadiényle, cyclooctadiényle ou norbornényle ou dérivés de norbornényle.

**23.** Compositions selon la revendication 17, caractérisées en ce que, pour le reste de cyclooléfine de formule II, il s'agit d'un reste de formule III

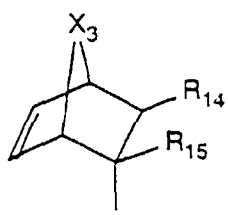

(III)

dans laquelle

| | |
|---|---|
| $X_3$ | représente un groupe $-CHR_{16}-$, des atomes d'oxygène ou de soufre ; |
| $R_{14}$ et $R_{15}$ | représentent indépendammment l'un de l'autre des atomes d'hydrogène, des groupes -CN, trifluorométhyle, $(CH_3)_3Si-O-$, $(CH_3)_3Si-$ ou $-COOR_{17}$ ; et |
| $R_{16}$ et $R_{17}$ | représentent indépendamment l'un de l'autre des atomes d'hydrogène, des groupes alkyle en $C_1$-$C_{12}$, phényle ou benzyle ; |

ou de formule IV

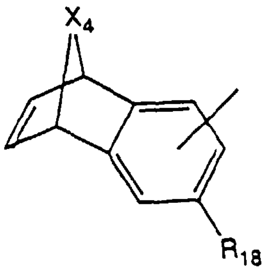

(IV),

dans laquelle

$X_4$ représente représente un groupe $-CHR_{19}-$, des atomes d'oxygène ou de soufre ;
$R_{19}$ représente des atomes d'hydrogène, des groupes alkyle en $C_1$-$C_{12}$, phényle ou benzyle ; et
$R_{18}$ représente des atomes d'hydrogène, des groupes alkyle en $C_1$-$C_6$ ou des atomes d'halogènes.

**24.** Compositions selon la revendication 17, caractérisées en ce que pour le reste cyclooléfine de formule II, il s'agit de norbornényle de formule

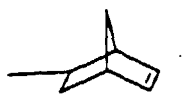

**25.** Composition selon la revendication 17, caractérisée en qu'elle contient, en tant que catalyseur à un composant, un composé thermostable de ruthénium ou d'osmium, lequel possède au moins un ligand lié à l'atome de ruthénium ou d'osmium et dont les autres sites de coordination sont saturés par des ligands non photolabiles.

**26.** Composition selon la revendication 25, caractérisée en ce que les composés de ruthénium et d'osmium répondent à la formule X

$$[L_1Me(L_8)_5]^{2\oplus}[Y_1]_{2/x} \qquad (X),$$

dans laquelle $L_1$ représente un ligand photolabile et $L_8$ un ligand non photolabile, Me représente Ru ou Os, $Y_1$ représente un anion non coordonné et x vaut 1, 2 ou 3.

**27.** Composition selon la revendication 17, caractérisée en ce qu'il s'agit, pour le catalyseur à un composant, d'un composé de molybdène(VI) ou de tungstène(VI), lequel contient au moins deux groupes méthyle ou deux groupes méthyle substitué liés au métal, le substituants ne contenant pas d'atome d'hydrogène en position $\alpha$.

**28.** Composition selon la revendication 27, caractérisée en ce que les groupes méthyle éventuellement monosubstitué liés au métal répondent à la formule XI

$$-CH_2-R \qquad (XI)$$

dans laquelle

R représente un atome de H, $-CF_3$, $-SiR_{38}R_{39}R_{40}$, $-CR_{41}R_{42}R_{43}$, un groupe aryle en $C_6$-$C_{16}$ ou hétéroaryle en $C_4$-$C_{15}$ comportant 1 à 3 hétéroatomes pris dans le groupe des atomes de O, S ou N, non substitué ou substitué par des substituants alkyle en $C_1$-$C_6$ ou alkoxy en $C_1$-$C_4$ ;

$R_{38}$, $R_{39}$ et $R_{40}$ représentent indépendamment les uns des autres des groupes alkyle en $C_1$-$C_6$, cycloalkyle en $C_5$ ou $C_6$ ou phényle ou benzyle non substitué ou substitué par des substituants alkyle en $C_1$-$C_6$ ou alkoxy en $C_1$-$C_6$ ; et

$R_{41}$, $R_{42}$ et $R_{43}$ représentent indépendamment l'un de l'autre des groupes alkyle en $C_1$-$C_{10}$ non substitué ou substitué par des groupes alkoxy en $C_1$-$C_{10}$, ou $R_{41}$ et $R_{42}$ possèdent cette signification et $R_{43}$ représente des groupes aryle en $C_6$-$C_{10}$ ou hétéroaryle en $C_4$-$C_9$ non substitué ou substitué par des groupes alkyle en $C_1$-$C_6$ ou alkoxy en $C_1$-$C_6$.

**29.** Composition selon la revendication 17, caractérisée en ce que, pour le catalyseur à un composant, il s'agit d'un composé de titane(IV), de niobium(V), de tantale(V), de molybdène(VI) ou de tungstène(VI) dans lequel un groupe silyle et au moins un atome d'halogène sont liés au métal.

**30.** Composition selon la revendication 29, caractérisée en ce que le groupe silylméthyle répond à la formule XIV

$$-CH_2-SiR_{38}R_{39}R_{40} \quad (XIV)$$

dans laquelle

$R_{38}$, $R_{39}$ et $R_{40}$ représentent indépendamment les uns des autres des groupes alkyle en $C_1$-$C_{18}$, cycloalkyle en $C_5$ ou $C_6$, ou phényle ou benzyle non substitué ou substitué par des substituants alkyle en $C_1$-$C_6$ ou alkoxy en $C_1$-$C_6$.

**31.** Composition selon la revendication 17, caractérisée en ce que, pour le catalyseur à un composant, il s'agit d'un composé de niobium(V) et ou de tantale(V) lequel contient au moins deux groupes méthyle ou deux groupes méthyle monosubstitué liés au métal, le substituants ne contenant pas d'atome d'hydrogène en position $\alpha$.

**32.** Composition selon la revendication 31, caractérisée en ce que les groupes méthyle éventuellement monosubstitué répondent à la formule XI

$$-CH_2-R \quad (XI),$$

où

R représente un atome d'hydrogène, des groupes $-CF_3$, $-SiR_{38}R_{39}R_{40}$, $-CR_{41}R_{42}R_{43}$, aryle en $C_6$-$C_{16}$ ou hétéroaryle en $C_4$-$C_{15}$ comportant 1 à 3 hétéroatomes pris dans le groupe des atomes de O, S ou N, non substitué ou substitué par des groupes alkyle en $C_1$-$C_6$ ou alkoxy en $C_1$-$C_6$ ;

$R_{38}$, $R_{39}$ et $R_{40}$ représentent indépendamment les uns des autres des groupes alkyle en $C_1$-$C_{12}$, cycloalkyle en $C_5$ ou $C_6$, ou phényle ou benzyle non substitué ou substitué par des substituants alkyle en $C_1$-$C_6$ ou alkoxy en $C_1$-$C_6$ ; et

$R_{41}$, $R_{42}$ et $R_{43}$ représentent indépendamment l'un de l'autre des groupes alkyle en $C_1$-$C_{10}$ non substitué ou substitué par des groupes alkoxy en $C_1$-$C_{10}$, ou $R_{41}$ et $R_{42}$ possèdent cette signification et $R_{43}$ représente des groupes aryle en $C_6$-$C_{10}$ ou hétéroaryle en $C_4$-$C_9$ non substitué ou substitué par des groupes alkyle en $C_1$-$C_6$ ou alkoxy en $C_1$-$C_6$.

**33.** Composition selon la revendication 17, caractérisée en ce que, pour le catalyseur à un composant, il s'agit d'un composé de titane(IV) lequel contient au moins deux groupes méthyle ou deux groupes méthyle monosubstitué liés au métal, le substituants ne contenant pas d'atome d'hydrogène en position $\alpha$.

**34.** Composition selon la revendication 33, caractérisée en ce que les groupes méthyle éventuellement monosubstitué répondent à la formule XI

$$-CH_2-R \quad (XI)$$

où

R représente un atome d'hydrogène, des groupes $-CF_3$, $-SiR_{38}R_{39}R_{40}$, $-CR_{41}R_{42}R_{43}$, aryle en $C_6$-$C_{16}$ ou hétéroaryle en $C_4$-$C_{15}$ comportant 1 à 3 hétéroatomes pris dans le groupe des atomes de O, S ou N, non substitué ou substitué par des groupes alkyle en $C_1$-$C_6$ ou alkoxy en $C_1$-$C_6$ ;

$R_{38}$, $R_{39}$ et $R_{40}$ représentent indépendamment les uns des autres des groupes alkyle en $C_1$-$C_{12}$, cycloalkyle en $C_5$ ou $C_6$, ou phényle ou benzyle non substitué ou substitué par des substituants alkyle en $C_1$-$C_6$ ou alkoxy en $C_1$-$C_6$, et

$R_{41}$, $R_{42}$ et $R_{43}$ représentent indépendamment l'un de l'autre des groupes alkyle en $C_1$-$C_{10}$ non substitué ou substitué par des groupes alkoxy en $C_1$-$C_{10}$, ou $R_{41}$ et $R_{42}$ possèdent cette signification et $R_{43}$ représente des groupes aryle en $C_6$-$C_{10}$ ou hétéroaryle en $C_4$-$C_9$ non substitué ou substitué par des groupes alkyle en $C_1$-$C_6$ ou alkoxy en $C_1$-$C_6$.

**35.** Composition selon la revendication 17, caractérisée en ce que, pour le catalyseur à un composant, il s'agit d'un composé de ruthénium ou d'osmium lequel contient au moins un groupe phosphine, au moins un ligand photolabile, et éventuentuellement des ligands neutres liés à l'atome de métal, au total 2 à 5 ligands étant liés, et les anions acides afin d'équilibrer la charge.

**36.** Composition selon la revendication 35, caractérisée en ce que les ligands du type phosphine répondent aux formules XXIII ou XXIIIa

$$PR_{91}R_{92}R_{93} \quad \text{(XXIII)},$$

$$R_{91}R_{92}P\text{-}Z_1\text{-}PR_{91}R_{92} \quad \text{(XXIIIa)},$$

où

$R_{91}$, $R_{92}$ et $R_{93}$ représentent indépendammment les uns des autres des atomes d'hydrogène, des groupes alkyle en $C_1$-$C_{20}$, cycloalkyle en $C_4$-$C_{12}$ non substitué ou substitué par des substituants alkyle en $C_1$-$C_6$, haloalkyle en $C_1$-$C_6$ ou alkoxy en $C_1$-$C_6$, ou aryle en $C_6$-$C_{16}$ non substitué ou substitué par des substituants alkyle en $C_1$-$C_6$, haloalkyle en $C_1$-$C_6$ ou alkoxy en $C_1$-$C_6$, ou aralkyle en $C_7$-$C_{16}$ non substitué ou substitué par des substituants alkyle en $C_1$-$C_6$, haloalkyle en $C_1$-$C_6$ ou alkoxy en $C_1$-$C_6$ ; les restes $R_{91}$ et $R_{92}$ ensemble représentent un groupe tétra- ou pentaméthylène non substitué ou substitué par des substituants alkyle en $C_1$-$C_6$, haloalkyle en $C_1$-$C_6$ ou alkoxy en $C_1$-$C_6$, ou un groupe tétra- ou pentaméthylène non substitué ou substitué par des substituants alkyle en $C_1$-$C_6$, haloalkyle en $C_1$-$C_6$ ou alkoxy en $C_1$-$C_6$ et condensé sur 1 ou 2 groupes 1,2-phénylène, et $R_{93}$ possède la signification donnée ci-dessus ;

$Z_1$ représente des groupes alkylène en $C_2$-$C_{12}$ à chaîne droite ou ramifiée non substitué ou substitué par un substituant alkoxy en $C_1$-$C_4$, des groupes 1,2- ou 1,3-cycloalkylène comportant 4 à 8 atomes de carbone non substitué ou substitué par des substituants alkyle en $C_1$-$C_4$ ou alkoxy en $C_1$-$C_4$, ou des groupes 1,2- ou 1,3-hétérocycloalkylène comportant un cycle à 5 ou 6 chaînons et un hétéroatome pris parmi les atomes de O ou N, non substitué ou substitué par des substituants alkyle en $C_1$-$C_4$ ou alkoxy en $C_1$-$C_4$.

**37.** Composition selon la revendication 17, caractérisée en ce que, pour le catalyseur à un composant, il s'agit d'un composé de ruthénium ou d'osmium cationique bivalent comportant un atome de métal auquel sont liés 1 à 3 ligands du type phosphine tertiaire avec, dans le cas du composé de ruthénium, un substituant à encombrement stérique, éventuellement des ligands neutres non photolabiles et des anions afin d'équilibrer la charge, à la condition que, dans les (triphénylphosphino)dihalogénures ou hydridohalogénures de ruthénium, les groupes phényle soient substitués par des substituants alkyle en $C_1$-$C_{18}$, haloalkyle en $C_1$-$C_{18}$ ou alkoxy en $C_1$-$C_{18}$.

**38.** Composition selon la revendication 37, caractérisée en ce que les ligands du type phosphine répondent aux formules XXIII ou XXIIIa

$$PR_{91}R_{92}R_{93} \quad (XXIII),$$

$$R_{91}R_{92}P\text{-}Z_1\text{-}PR_{91}R_{92} \quad (XXIIIa),$$

où

$R_{91}$, $R_{92}$ et $R_{93}$ représentent indépendammment les uns des autres des atomes d'hydrogène, des groupes alkyle en $C_1$-$C_{20}$, cycloalkyle en $C_4$-$C_{12}$ non substitué ou substitué par des substituants alkyle en $C_1$-$C_6$, haloalkyle en $C_1$-$C_6$ ou alkoxy en $C_1$-$C_6$, ou aryle en $C_6$-$C_{16}$ non substitué ou substitué par des substituants alkyle en $C_1$-$C_6$, haloalkyle en $C_1$-$C_6$ ou alkoxy en $C_1$-$C_6$, ou aralkyle en $C_7$-$C_{16}$ non substitué ou substitué par des substituants alkyle en $C_1$-$C_6$, haloalkyle en $C_1$-$C_6$ ou alkoxy en $C_1$-$C_6$ ; les restes $R_{91}$ et $R_{92}$ ensemble représentent un groupe tétra- ou pentaméthylène non substitué ou substitué par des substituants alkyle en $C_1$-$C_6$, haloalkyle en $C_1$-$C_6$ ou alkoxy en $C_1$-$C_6$, ou un groupe tétra- ou pentaméthylène non substitué ou substitué par des substituants alkyle en $C_1$-$C_6$, haloalkyle en $C_1$-$C_6$ ou alkoxy en $C_1$-$C_6$ et condensé sur 1 ou 2 groupes 1,2-phénylène, et $R_{93}$ possède la signification donnée cidessus ;

$Z_1$ représente des groupes alkylène en $C_2$-$C_{12}$ à chaîne droite ou ramifiée non substitué ou substitué par un substituant alkoxy en $C_1$-$C_4$, des groupes 1,2- ou 1,3-cycloalkylène comportant 4 à 8 atomes de carbone non substitué ou substitué par des substituants alkyle en $C_1$-$C_4$ ou alkoxy en $C_1$-$C_4$, ou des groupes 1,2- ou 1,3-hétérocycloalkylène comportant un cycle à 5 ou 6 chaînons et un hétéroatome pris parmi les atomes de O ou N, non substitué ou substitué par des substituants alkyle en $C_1$-$C_4$ ou alkoxy en $C_1$-$C_4$.

**39.** Composition selon la revendication 17, caractérisée en ce que le catalyseur à un composant est contenu dans une quantité de 0,001 à 20% en moles, par rapport à la quantité du monomère.

**40.** Procédé de préparation de polymères réticulés par polymérisation par métathèse selon la revendication 1, caractérisé en ce que l'on polymérise

(a) au moins un composé de formule I et
(b) une quantité catalytique d'au moins un catalyseur à un composant activable par la chaleur ou par un rayonnement pour une polymérisation par métathèse (c) par chauffage,
(d) par irradiation,
(e) par chauffage et par irradiation,
(f) on active le catalyseur à un composant par chauffage de courte durée et on termine la polymérisation par irradiation, ou
(g) on active le catalyseur à un composant par irradiation de courte durée et on termine la polyémrisation par chauffage.

**41.** Procédé selon la revendication 40, caractérisé en ce qu'il est mis en oeuvre à une température de 20 à 300°C.

**42.** Polymères par métathèse réticulés d'au moins un composé (I) selon la revendication 1.

**43.** Matière de support revêtue caractérisée en ce que l'on applique sur un substrat une couche d'une composition selon la revendication 17.

**44.** Substrat revêtu d'une couche durcie d'une composition selon la revendication 17.